# EUROPÄISCHE PATENTSCHRIFT

(11) **EP 1 948 627 B1**
(45) Veröffentlichungstag und Bekanntmachung des Hinweises auf die Patenterteilung: **14.03.2012**
(21) Anmeldenummer: 06829004.8
(22) Anmeldetag: 10.11.2006
(51) Int. Cl.: C07D 277/06, C07D 295/18, C07D 417/12, A61K 31/427, A61P 25/00

(54) **NEUE DUALE PEPTIDASE-INHIBITOREN ALS PRODRUGS ZUR THERAPIE VON ENTZÜNDLICHEN UND ANDEREN ERKRANKUNGEN**
NOVEL DUAL PEPTIDASE INHIBITORS AS PRODRUGS FOR THE THERAPY OF INFLAMMATORY AND OTHER DISORDERS
NOUVEAUX INHIBITEURS MIXTES DE PEPTIDASE EN TANT QUE PROMEDICAMENTS POUR LA THERAPIE DE MALADIES INFLAMMATOIRES ET AUTRES

(30) Priorität: 16.11.2005 DE 102005054700
(43) Veröffentlichungstag der Anmeldung: 30.07.2008
(73) Patentinhaber: IMTM GmbH, 39120 Magdeburg (DE)
(72) Erfinder: ANSORGE, Siegfried, 39291 Hohenwarthe (DE); NEUBERT, Klaus, 06128 Halle (DE); BANK, Ute, 39418 Stassfurt (DE); REICHSTEIN, Irene, 06122 Halle (DE); FAUST, Jürgen, 06114 Halle (DE); TÄGER, Michael, 39326 Heinrichsberg (DE); FUCHS, Petra, 06128 Halle (DE); SENNS, Bianca, 06132 Halle (DE)
(74) Vertreter: Bauch-Koepe, Katharina Anna
(86) Internationale Anmeldenummer: PCT/EP2006/010818
(87) Internationale Veröffentlichungsnummer: WO 2007/057128

(56) Entgegenhaltungen:
- WO-A2-2005/034940
- US-A- 5 939 560
- SHIMAZAWA R ET AL: "NOVEL SMALL MOLECULE NONPEPTIDE AMINOPEPTIDASE N INHIBITORS WITH A CYCLIC IMIDE SKELETON" JOURNAL OF ENZYME INHIBITION, NEW YORK, NY, US, Bd. 14, Nr. 4, 1999, Seiten 259-275, XP001018772

## Beschreibung

Die Erfindung betrifft neue Stoffe und Verbindungen, die in der Lage sind, die Enzyme Dipeptidylpeptidase IV (DPIV) sowie Peptidasen mit analoger enzymatischer Wirkung und Alanyl-Aminopeptidase N (APN) sowie Peptidasen mit analoger enzymatischer Wirkung gemeinsam zu inhibieren ("duale Inhibitoren"). Weiter betrifft die Erfindung Verfahren zur Herstellung der neuen dualen Inhibitoren der DPIV und der APN. Die Erfindung betrifft auch die vorstehenden neuen Verbindungen zur Verwendung in der Medizin. Die Erfindung betrifft darüber hinaus auch die Verwendung dieser dualen Inhibitoren zur Prophylaxe und Therapie von Erkrankungen mit überschießender Immunantwort und entzündlicher Genese, von neuronalen Erkrankungen und zerebralen Schädigungen sowie von Tumorerkrankungen, Hauterkrankungen, Diabetes Typ II und SARS.

Die Dipeptidylpeptidase IV (DPIV, CD26, EC 3.4.14.5) ist eine ubiquitär auftretende Serin-Protease, die die Hydrolyse von Peptiden spezifisch hinter Prolin und in geringerem Maße Alanin bzw. - mit Einschränkungen - weiteren Aminosäuren wie Serin, Threonin, Valin und Glycin in der zweiten Position des N-Terminus katalysiert. Zur Gen-Familie der Enzyme mit zu DPIV analoger enzymatischer Aktivität gehören u. a. DP 8, DP 9 und FAP/Seprase (T. Chen et al.: Adv. Exp. Med. Biol. 524, 79, 2003). Eine analoge Substratspezifität wie DPIV weist auch Attractin (mahagony protein) auf (J. S. Duke-Cohan et al.: J. Immunol. 156, 1714, 1996). Das Enzym wird ebenfalls durch DPIV-Inhibitoren gehemmt.

Zur Gruppe der - ebenfalls ubiquitär vorkommenden - Alanyl-Aminopeptidasen gehören die überwiegend als Typ II-Membranprotein auftretende Aminopeptidase N (APN, CD13, EC 3.4.11.2) sowie die zytosolische, lösliche Alanyl-Aminopeptidase (EC 3.4.11.14, Puromycin-sensitive Aminopeptidase, Aminopeptidase PS, Enkephalin-abbauende Aminopeptidase). Alanyl-Aminopeptidasen, auch die beiden vorgenannten Aminopeptidasen, wirken Metall-abhängig, beispielsweise Zink-abhängig, und katalysieren die Hydrolyse der Peptidbindungen hinter N-terminalen Aminosäuren von Oligopeptiden, im Falle der APN mit einer Bevorzugung von Alanin am N-Terminus (A. J. Barrett et al.: Handbook of Proteolytic Enzymes, Academic Press 1998). Alle Hemmstoffe der Aminopeptidase N hemmen auch die zytosolische Alanyl-Aminopeptidase, dagegen existieren spezifische Inhibitoren der zytosolischen Aminopeptidase (M. Komodo et al.: Bioorg. and Med. Chem. 9, 121, 2001).

Für beide Enzymgruppen wurden wichtige biologische Funktionen in unterschiedlichen Zellsystemen nachgewiesen. Dies gilt u. a. für das Immunsystem (U. Lendeckel et al.: Intern. J. Mol. Med. 4, 17, 1999; T. Kähne et al.: Intern. J. Mol. Med. 4, 3, 1999; I. De Meester et al: Advanc. Exp. Med. Biol. 524, 3, 2002; Internationale Patentanmeldung WO 01/89569; Internationale Patentanmeldung WO 02/053170; Internationale Patentanmeldung PCT/EP 03/07199), das neuronale System (Internationale Patentanmeldung WO 02/053169 und Deutsche Patentanmeldung 103 37 074.9), die Fibroblasten (Deutsche Patentanmeldung 103 30 842.3), die Keratinozyten (Internationale Patentanmeldung WO 02/053170), die Talgdrüsenzellen/Sebozyten (Internationale Patentanmeldung PCT/EP 03/02356), Tumore sowie für Infektionen durch Viren, z.B. Coronaviren (D. P. Kontoyiannis et al.: Lancet 361, 1558, 2003).

Die Fähigkeit der DPIV, die inkretorischen Hormone GIP und GLP spezifisch zu inaktivieren, hat zur Entwicklung eines neuen therapeutischen Konzepts zur Behandlung von Glukose-Stoffwechselstörungen geführt (D. M. Evans: Drugs 5, 577, 2002).

Für beide Enzymgruppen sind unterschiedliche Inhibitoren bekannt (Reviews in D. M. Evans: Drugs 5, 577, 2002; und M.-C. Fournie-Zaluski und B. P. Roques: in J. Langner and S. Ansorge, Ectopeptidases, Kluwer Academic/Plenum Publishers, P. 51, 2002).

Die isolierte Hemmung der Alanyl-Aminopeptidasen und der Dipeptidylpeptidase IV sowie die Hemmung von Enzymen mit analoger Substratspezifität, insbesondere aber die kombinierte Hemmung von Enzymen beider Enzymgruppen, führt an Immunzellen zur starken Hemmung der DNA-Synthese und damit zur starken Hemmung der Zellvermehrung sowie zur Veränderung der Zytokinproduktion, insbesondere zur Induktion des immunregulatorisch wirkenden TGF-β1 (Internationale Patentanmeldung WO 01/89569, Internationale Patentanmeldung WO 02/053170) sowie zur Hemmung der Bildung und Freisetzung inflammatorischer Zytokine des TH1- und TH2-Typs, z. B. Interleukin-4 (IL-4) (Internationale Patentanmeldung WO 02/053 170 und Deutsche Patentanmeldung Nr. 101 02 392.8). An regulatorischen T-Zellen bewirken Alanyl-Aminopeptidase-Inhibitoren eine starke Induktion von TGF-β1 (Internationale Patentanmeldung PCT/EP 03/07199). Im neuronalen System wurde durch Hemmung der beiden Enzymsysteme eine Verminderung bzw. Verzögerung akuter und chronischer zerebraler Schädigungsprozesse nachgewiesen (Internationale Patentanmeldung WO 02/053169 und Deutsche Patentanmeldung 103 37 074.9). Auch an Fibroblasten (Deutsche Patentanmeldung 103 30 842.3), Keratinozyten (Internationale Patentanmeldung WO 02/0531 70) und Sebozyten (Internationale Patentanmeldung PCT/EP 03/02356) wurde gezeigt, dass die kombinierte Hemmung der Alanyl-Aminopeptidase N und DPIV eine Hemmung des Wachstums und eine Veränderung der Zytokinproduktion bewirkt.

Die Druckschrift WO 2005/034940 betrifft Substanzen, die sowohl Alanylaminopeptidasen als auch Dipeptidylpeptidase IV inhibieren. Darüber hinaus betrifft diese Druckschrift die Verwendung mindestens einer derartigen Substanz oder mindestens einer derartigen Substanz enthaltenden pharmazeutischen Zubereitung zur Prophylaxe und Therapie von Erkrankungen, insbesondere zur Prophylaxe und Therapie von Erkrankungen mit überschießender Immunantwort, von anderen chronisch entzündlichen Erkrankungen, neuronalen Erkrankungen, und zerebralen Schädigungen, Hauterkrankungen, Tumorerkrankungen und speziellen Virusinfektionen.

Damit ergibt sich der überraschende Sachverhalt, dass die Alanyl-Aminopeptidasen und die Dipeptidylpeptidase IV sowie analog wirkende Enzyme fundamentale zentrale biologische Funktionen in unterschiedlichen Organen und Zellsystemen erfüllen und eine kombinierte Hemmung beider Enzymgruppen ein neues wirkungsvolles therapeutisches Prinzip für die Behandlung unterschiedlichster, zumeist chronischer Erkrankungen repräsentiert.

An akzeptierten Tiermodellen konnten die Anmelder inzwischen zeigen, dass insbesondere die kombinierte Gabe von Inhibitoren der beiden Peptidase-Gruppen auch *in vivo* eine Hemmung des Wachstums verschiedener Zellsysteme und eine Unterdrückung einer überschießenden Immunantwort, chronisch-entzündlicher Vorgänge sowie zerebraler Schädigungen bewirkt (Internationale Patentanmeldung WO 01/89569). Die isolierte Gabe einzelner bekannter Inhibitoren zeigte eine schwächere Wirkung.

Die bisherigen Ergebnisse wurden überwiegend mit Hilfe bekannter, in der Literatur beschriebener und zum Teil kommerziell zugänglicher Inhibitoren der Alanyl-Aminopeptidase N und der Dipeptidylpeptidase IV allein, besonders aber in Kombination der Inhibitoren beider Enzymgruppen, erhalten.

Es wurden nun überraschend neuartige, überwiegend nicht-peptidische, niedermolekulare Substanzen gefunden, die als Prodrugs eingesetzt werden können und unter physiologischen und pathologischen Bedingungen zu Wirkstoffen oder einem Gemisch von Wirkstoffen reagieren können, die in dualer Weise sowohl die Alanyl-Aminopeptidase N und Enzyme mit analoger Substratspezifität als auch die Dipeptidylpeptidase IV und Enzyme mit analoger Substratspezifität inhibieren. Die Umwandlung der Prodrugs erfolgt durch Reduktion von -S-S- oder -Se-Se-Brücken, vorzugsweise durch zelluläre Thiole (SH-Gruppen tragende Verbindungen).

Prodrugs der hier offenbarten Art wirken damit vorzugsweise an Zellen und Geweben. Darüber hinaus wird durch die Verwendung der Prodrugs vermieden, dass die inhibitorische Kapazität der Hemmstoffe durch Bindung an freie Peptidasen im Blutplasma reduziert wird.

Die Erfindung betrifft neue Substanzen, die sowohl Ala-p-Nitroanilid- als auch Gly-Pro-p-Nitroanilid-spaltende Peptidasen spezifisch inhibieren und damit die Fähigkeit der kombinierten Hemmung beider Peptidase-Gruppen in jeweils einer Substanz vereinen.

Die Erfindung betrifft darüber hinaus neue Substanzen, die als solche, jedoch auch als Ausgangsstoffe für weitere Substanzen, zur Prophylaxe und Therapie von Erkrankungen mit überschießender Immunantwort (Autoimmunerkrankungen, Allergien und Transplantatrejektionen, Sepsis), anderen chronisch-entzündlichen Erkrankungen einschließlich Arteriosklerose, neuronalen Erkrankungen und zerebralen Schädigungen, Hauterkrankungen (u. a. Akne und Schuppenflechte), Tumorerkrankungen und speziellen Virusinfektionen (u. a. SARS) sowie Diabetes Typ II genutzt werden können.

Die Erfindung betrifft Verbindungen der allgemeinen Formeln (1) und (2)

A-B-D-B'-A' (1)

und

A - B - D - E (2),

worin
- A und A' gleich oder verschieden sein können und für einen Rest 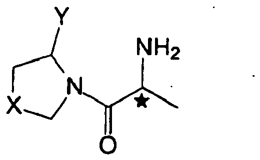 stehen, worin X für S, O, CH₂, CH₂CH₂, CH₂O oder CH₂NH steht und Y für H oder CN steht und * ein chirales Kohlenstoff-Atom bezeichnet;
- B und B' gleich und verschieden sein können und für einen O, N oder S enthaltenden oder nicht enthaltenden, unsubstituierten oder substituierten, unverzweigten oder verzweigten Alkylen-Rest, Cycloalkylen-Rest, Aralkylen-Rest, Heterocycloalkylen-Rest, Heteroarylalkylen-Rest, Arylamidoalkylen-Rest, Heteroarylamidoalkylen-Rest, unsubstituierten oder einfach oder mehrfach substituierten Arylen-Rest oder Heteroarylen-Rest mit einem oder mehreren fünf-, sechs- oder siebengliedrigen Ringen steht;
- D für - S - S - oder - Se - Se - steht; und
- E für die Gruppe - CH₂ - *CH (NH₂) - R⁹ steht, worin R⁹ für einen O, N oder S enthaltenden oder nicht enthaltenden unsubstituierten substituierten unverzweigten oder verzweigten Alkyl-Rest, Cycloalkyl-Rest, Aralkyl-Rest, Heterocycloalkyl-Rest, Heteroarylalkyl-Rest, Arylamidoalkyl-Rest, Heteroarylamidoalkyl-Rest, unsubstituierten oder einfach oder mehrfach substituierten Aryl-Rest oder Heteroaryl-Rest mit einem oder mehreren fünf-, sechs- oder siebengliedrigen Ringen steht und * ein chirales Kohlenstoff-Atom bezeichnet;
- oder deren Säureadditionssalze mit organischen und/oder anorganischen Säuren.

Bevorzugte Ausführungsformen der Verbindungen der allgemeinen Formeln (1) und (2) ergeben sich aus den Unteransprüchen 2 bis 10.

Die Erfindung betrifft auch ein Verfahren zur Herstellung der Verbindungen der allgemeinen Formeln (1) und (2) entsprechend dem auf der nachfolgenden Seite angegebenen allgemeinen Syntheseschema, worin A, A', B, B', D und E die oben im Detail angegebenen Bedeutungen haben, worin man entsprechend dem nachfolgenden Syntheseschema 1 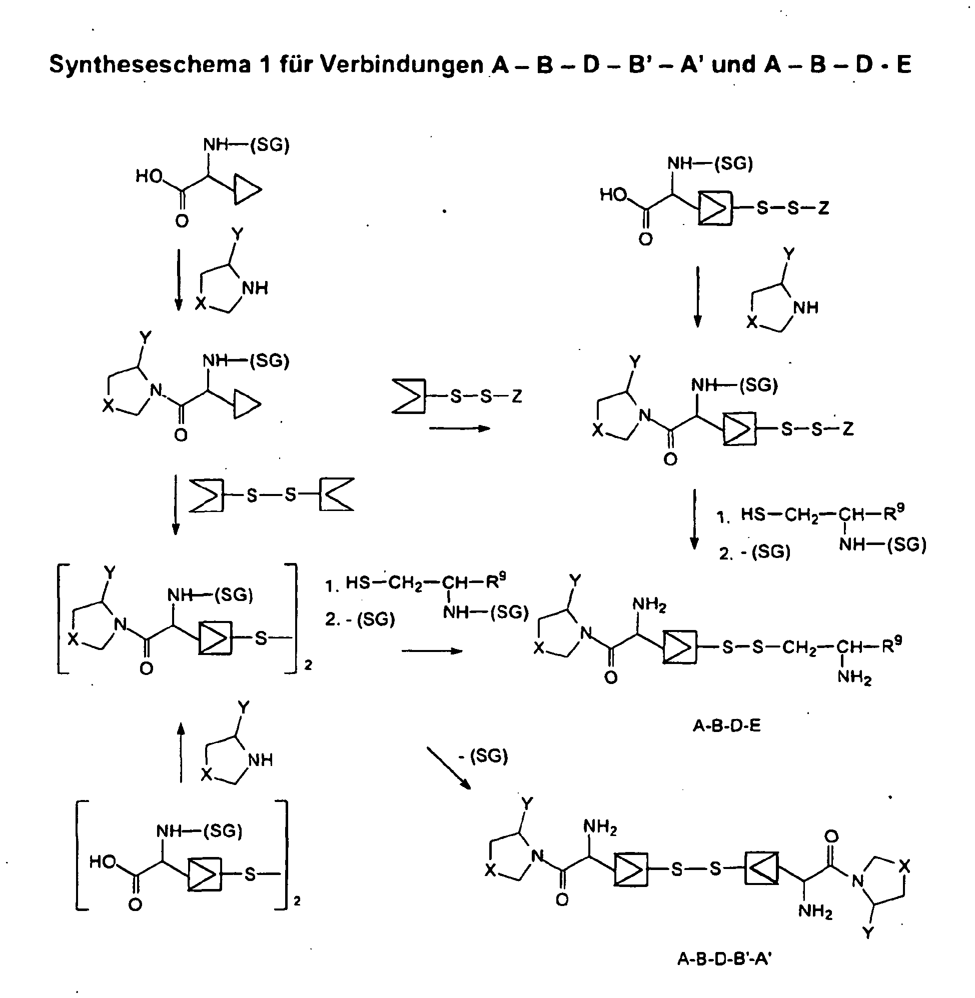
- Verbindungen der allgemeinen Formel 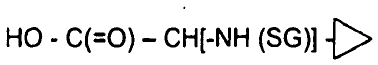 worin (SG) eine Schutzgruppe bedeutet und 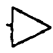 für ein Strukturelement von B steht, mit einer heterocyclischen Verbindung der allgemeinen Formel 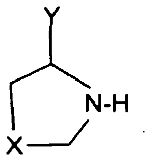 umsetzt, worin X für S, O, CH₂, CH₂CH₂, CH₂O oder CH₂NH steht und Y für H oder CN steht; das erhaltene Kondensationsprodukt der Formel 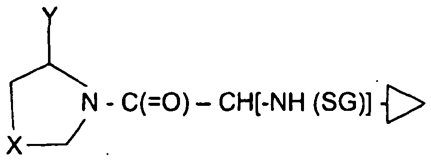 mit einer Verbindung der allgemeinen Formel 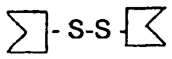 umsetzt, worin 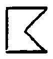 für ein Strukturelement von B steht; und das erhaltene Reaktionsprodukt unter Abspaltung der Schutzgruppen (SG) in eine Verbindung der allgemeinen Formel A - B - D - B' - A' (1) überführt, in der A und A' gleich oder verschieden sein können und B und B' gleich oder verschieden sein können und worin A, A', B, B' und D die oben angegebenen Bedeutungen haben können; oder
- Verbindungen der allgemeinen Formel 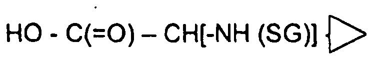 worin (SG) eine Schutzgruppe bedeutet und 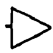 für ein Strukturelement von B steht, mit einer heterocyclischen Verbindung der allgemeinen Formel 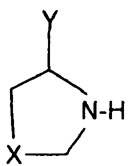 umsetzt, worin X für S, O, CH₂, CH₂CH₂, CH₂O oder CH₂NH steht und Y für H oder CN steht; das erhaltene Kondensationsprodukt der Formel 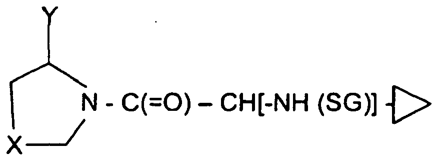 mit einer Verbindung der allgemeinen Formel 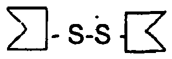 umsetzt, worin 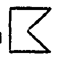 für ein Strukturelement von B steht; und das erhaltene Reaktionsprodukt mit einer Verbindung der allgemeinen Formel

   HS - CH₂ - CH [- NH - (SG)] - (_{R}⁹)

   und nach Abspaltung der Schutzgruppen (SG) zu einer Verbindung der allgemeinen Formel A - B - D - E (2) umsetzt, worin A, B, D und E die oben angegebenen Bedeutungen haben können; oder
- eine Verbindung der allgemeinen Formel 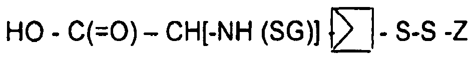 worin (SG) für eine Schutzgruppe steht, 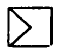 für ein Strukturelement von B steht und Z für einen Rest steht, der eine -S-S-Gruppe für einen Thiolaustausch aktiviert, mit einer heterocyclischen Verbindung der allgemeinen Formel 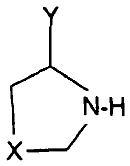 umsetzt, worin X für S, O, CH₂, CH₂CH₂, CH₂O oder CH₂NH steht und Y für H oder CN steht; das erhaltene Kondensationsprodukt der Formel 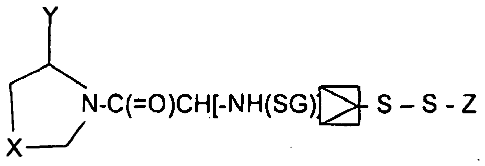 mit einer mit einer Verbindung der allgemeinen Formel

   HS-CH₂-CH[-NH-(SG)]-(R⁹)

   und nach Abspaltung der Schutzgruppen (SG) zu einer Verbindung der allgemeinen Formel A - B - D - E (2) umsetzt, worin A, B, D und E die oben angegebenen Bedeutungen haben können; oder
- eine Verbindung der allgemeinen Formel 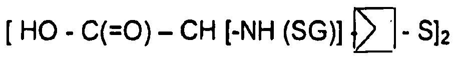 worin SG für eine Schutzgruppe steht und 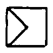 für ein Strukturelement von B steht, mit einer heterocyclischen Verbindung der allgemeinen Formel 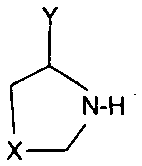 umsetzt, worin X für S, O, CH₂, CH₂CH₂, CH₂O oder CH₂NH steht und Y für H oder CN steht; und das erhaltene Reaktionsprodukt mit einer Verbindung der allgemeinen Formel

   HS-CH₂-CH[-NH-(SG)]-R⁹

   zu einer Verbindung der allgemeinen Formel A - B - D - E (2) umsetzt, worin A, B, D und E die oben angegebenen Bedeutungen haben können; oder
- eine Verbindung der allgemeinen Formel 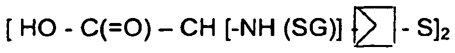 worin SG für eine Schutzgruppe steht und 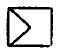 für ein Strukturelement von B steht, mit einer heterocyclischen Verbindung der allgemeinen Formel 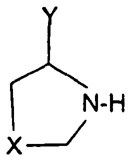 umsetzt, worin X für S, O, CH₂, CH₂CH₂. CH₂O oder CH₂NH steht und Y für H oder CN steht; und das erhaltene Reaktionsprodukt unter Abspaltung der Schutzgruppen SG in eine Verbindung der allgemeinen Formel A - B - D - B' - A' (1) überführt, in der A und A' gleich oder verschieden sein können und B und B' gleich oder verschieden sein können und worin A, A', B, B' und D die oben angegebenen Bedeutungen haben können; oder
- Verbindungen der allgemeinen Formel 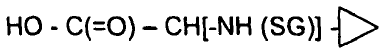 worin (SG) eine Schutzgruppe bedeutet und 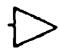 für ein Strukturelement von B steht, mit einer heterocyclischen Verbindung der allgemeinen Formel 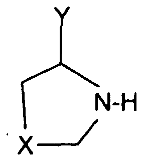 umsetzt, worin X für S, O, CH₂, CH₂CH₂, CH₂O oder CH₂NH steht und Y für H oder CN steht; das erhaltene Kondensationsprodukt der Formel 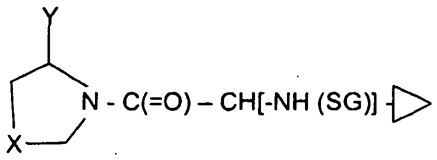 mit einer Verbindung der allgemeinen Formel 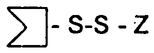 umsetzt, worin 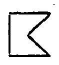 für ein Strukturelement von B steht und Z für einen Rest steht, der eine -S-S-Gruppe für einen Thiolaustausch aktiviert, und (SG) für eine Schutzgruppe steht; und das erhaltene Reaktionsprodukt der Formel 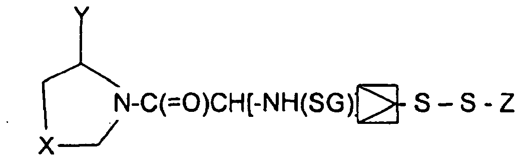 mit einer mit einer Verbindung der allgemeinen Formel

   HS-CH₂-CH[-NH-(SG)]-(R⁹)

   und nach Abspaltung der Schutzgruppen (SG) zu einer Verbindung der allgemeinen Formel A - B - D - E (2) umsetzt, worin A, B, D und E die oben angegebenen Bedeutungen haben können.

Die Erfindung betrifft auch die oben angegebenen und nachfolgend im Detail beschriebenen Verbindungen der allgemeinen Formeln (1) und (2) zur Verwendung in der Medizin.

Die Erfindung betrifft weiter die oben angegebenen und nachfolgend im Detail beschriebenen Verbindungen der allgemeinen Formeln (1) und (2) als Inhibitoren-Vorstufen oder Inhibitoren-Prodrugs.

Bevorzugte Ausführungsformen ergeben sich aus den Unteransprüchen 14 bis 16_{.}

Die Erfindung betrifft weiter die Verwendung mindestens einer Verbindung der obigen und nachfolgend im Detail beschriebenen allgemeinen Formeln (1) und (2) zur Herstellung eines Medikament zur Prophylaxe und Therapie von Erkrankungen mit überschießender Immunantwort und entzündlicher Genese, einschließlich Arteriosklerose, neuronalen Erkrankungen, zerebralen Schädigungen, Hauterkrankungen, Tumorerkrankungen und Virus-bedingten Erkrankungen sowie Diabetes Typ II.

Die Erfindung betrifft auch die Verwendung mindestens einer Verbindung der obigen und nachfolgend im Detail beschriebenen allgemeinen Formeln (1) und (2) zur Herstellung eines Medikament zur Prophylaxe und Therapie von Erkrankungen mit überschießender Immunantwort und entzündlicher Genese, einschließlich Arteriosklerose, neuronalen Erkrankungen, zerebralen Schädigungen, Hauterkrankungen, Tumorerkrankungen und Virus-bedingten Erkrankungen sowie Diabetes Typ II.

Bevorzugte Ausführungsformen der Verwendung sind in den Ansprüchen 19 bis 20 beansprucht.

Die Erfindung betrifft weiter ein Verfahren zur Generierung von mindestens einem Inhibitor der Dipeptidylpeptidase IV (DPIV) und von Peptidasen mit analoger enzymatischer Wirkung sowie der Alanyl-Aminopeptidase N (APN) und von Peptidasen mit analoger enzymatischer Wirkung aus mindestens einer Verbindung der allgemeinen Formeln (1) und (2) entsprechend der nachfolgenden detaillierten Beschreibung, worin man die mindestens eine Verbindung der allgemeinen Formeln (1) und (2) gemäß der nachfolgenden detaillierten Beschreibung reduzierenden Bedingungen aussetzt, wie sie an Zellen und Geweben gegeben sind.

Bevorzugte Ausführungsformen des Verfahrens ergeben sich aus dem Anspruch 22.

Die Erfindung betrifft auch pharmazeutische oder kosmetische Zubereitungen, die wenigstens eine Verbindung wenigstens einer der allgemeinen Formeln (1) und (2) nach einem der Ansprüche 1 bis 10 und gemäß der nachfolgenden detaillierten Beschreibung umfassen, gegebenenfalls zusammen mit einem oder mehreren pharmazeutisch oder kosmetisch annehmbaren Träger(n), Hilfsstoff(en) und/oder Adjuvant(ien).

Erfindungsgemäß haben die neuen Verbindungen entweder die allgemeine Formel (1):

A-B-D-B'-A' (1)

oder die allgemeine Formel (2):

A-B-D-E (2),

wobei überraschend gefunden wurde, dass die Verbindungen der vorgenannten allgemeinen Formeln selbst inhibitorische Wirkung in Bezug auf die nachgenannten Enzyme haben und darüber hinaus unter definierten Bedingungen zu Verbindungen reagieren, die Inhibitoren der Dipeptidylpeptidase IV (DPIV) und von Peptidasen mit analoger enzymatischer Wirkung, der Alanyl-Aminopeptidase N (APN) und von Peptidasen mit analoger enzymatischer Wirkung sind.

In der vorliegenden Beschreibung und in den Patentansprüchen wird unter "Dipeptidylpeptidase IV" (DPIV, CD26, EC 3.4.14.5.) die Serin-Protease verstanden, die die Hydrolyse von Peptidbindungen spezifisch hinter Prolin und in geringerem Maße Alanin bzw. - mit Einschränkung - weiteren Aminosäuren wie Serin, Threonin, Valin und Glycin in der zweiten Position des N-Terminus von Peptiden katalysiert.

Unter dem Begriff "Peptidasen mit Dipeptidylpeptidase IV analoger enzymatischer Wirkung" werden in der vorliegenden Beschreibung und in den Patentansprüchen Peptidasen verstanden, die eine Hydrolyse von Peptiden spezifisch hinter Prolin oder Alanin in der zweiten Position des N-Terminus katalysieren. Beispiele für Peptidasen mit Dipeptidylpeptidase IV analoger enzymatischer Wirkung sind, ohne die Erfindung auf diese zu beschränken, DP 8, DP 9 und FAP/Seprase (T. Chen et al., a. a. O.) und Attractin (mahagony protein) (J. S. Duke-Cohan et al., a. a. O.).

In der vorliegenden Beschreibung und in den Patentansprüchen wird unter "Alanyl-Aminopeptidase N" (APN, CD13, EC 3.4.11.2.) die Protease verstanden, die Metall- (Zink-) abhängig wirkt und die Hydrolyse von Peptidbindungen spezifisch hinter N-terminalen Aminosäuren von Peptiden und vorzugsweise Alanin am N-Terminus katalysiert.

Unter dem Begriff "Peptidasen mit Alanyl-Aminopeptidase N analoger enzymatischer Wirkung" werden in der vorliegenden Beschreibung und in den Patentansprüchen Peptidasen verstanden, die - wie APN - Metall-abhängig wirken und eine Hydrolyse von Peptidbindungen spezifisch hinter N-terminalen Aminosäuren von Peptiden und vorzugsweise Alanin am N-Terminus katalysieren. Ein Beispiel für eine Peptidase mit Alanyl-Aminopeptidase N analoger enzymatischer Wirkung ist, ohne die Erfindung auf diese zu beschränken, die zytosolische, lösliche Alanyl-Aminopeptidase (EC 3.4.11.14, Puromycin-sensitive Aminopeptidase, Aminopeptidase PS, Enkephalin-abbauende Aminopeptidase) (A. J. Barret et al., a. a. O.).

Unter dem Begriff "Inhibitor" werden in der vorliegenden Beschreibung und in den Patentansprüchen solche Verbindungen natürlichen Ursprungs, synthetischen Ursprungs oder natürlichen Ursprungs mit synthetischer Modifikation verstanden, die eine regulierende Wirkung, insbesondere eine hemmende Wirkung, auf ein Enzym oder auf eine Gruppe von Enzymen haben. Die regulierende Wirkung kann auf unterschiedlichsten Effekten beruhen, ohne dass von der vorstehenden breiten Definition des Begriffs "Inhibitor" beschränkende Abstriche gemacht werden müssen. Bevorzugte Inhibitoren gemäß der Erfindung sind Inhibitoren mit hemmender Wirkung auf Enzyme, weiter bevorzugt auf Gruppen bestimmter Enzyme, beispielsweise Inhibitoren mit hemmender Wirkung auf Dipeptidylpeptidase IV (DP IV) und auf Peptidasen mit Dipeptidylpeptidase IV analoger enzymatischer Wirkung bzw. Inhibitoren mit hemmender Wirkung auf Alanyl-Aminopeptidase N (APN) und auf Peptidasen mit Alanyl-Aminopeptidase N analoger enzymatischer Wirkung, wie dies oben definiert wurde.

Unter dem Begriff "Vorstufe" werden in der vorliegenden Beschreibung und in den Patentansprüchen natürlich vorkommende oder synthetische oder natürlich vorkommende, jedoch synthetisch modifizierte Verbindungen verstanden, aus denen sich andere Verbindungen unter bestimmte Bedingungen chemisch ableiten bzw. derivatisieren lassen. So werden unter Inhibitor-Vorstufen Verbindungen natürlicher oder synthetischer Herkunft oder natürliche, jedoch synthetisch modifizierte Verbindungen verstanden, die gezielt zu Inhibitoren reagieren können.

Unter dem Begriff "Prodrug" werden in der vorliegenden Beschreibung und in den Patentansprüchen natürlich vorkommende oder synthetische oder natürlich vorkommende, jedoch synthetisch modifizierte Verbindungen verstanden, aus denen sich andere Verbindungen unter bestimmten Bedingungen, vorzugsweise unter physiologischen oder pathologischen Bedingungen, chemisch ableiten bzw. derivatisieren lassen, wobei diese anderen Verbindungen eine pharmakologische Wirksamkeit entfalten, die sich qualitativ und/oder quantitativ von der der Ausgangssubstanz unterscheidet. So werden unter Inhibitor-Prodrugs Verbindungen natürlicher oder synthetischer Herkunft oder natürliche, jedoch synthetisch modifizierte Verbindungen verstanden, die vorzugsweise unter physiologischen oder pathologischen Bedingungen, noch mehr bevorzugt unter physiologischen oder pathologischen Bedingungen im Säuger, beispielsweise im Menschen, gezielt zu neuen Substanzen mit inhibitorischer Wirksamkeit reagieren können. Dies schließt nicht aus, dass diese Prodrugs als solche auch schon vor der Umwandlung in Drugs mit bestimmter pharmakologischer (beispielsweise inhibitorischer) Wirksamkeit in der Lage sind, pharmakologische Wirksamkeit zu entfalten (beispielsweise eines der beiden oben genannten Enzyme zu hemmen). Bedingungen zur Umwandlung von Prodrugs in Drugs im Säuger bzw. Menschen können solche sein, wie sie regelmäßig in der physiologischen Umgebung eines Säugers, beispielsweise eines Menschen, oder im Körper eines Säugers, beispielsweise eines Menschen, vorliegen. Alternativ können solche physiologischen Bedingungen nur unter bestimmten, beispielsweise einen bestimmten physiologischen Zustand wie beispielsweise ein Krankheitsbild bestimmenden, Bedingungen in einem Säuger wie beispielsweise in einem Menschen vorliegen, oder sie können durch äußere Einwirkung, beispielsweise (ohne Beschränkung) durch medikamentöse Einwirkung, auf den Organismus eines Säugers wie beispielsweise den Organismus eines Menschen induziert oder eingestellt werden.

In den Verbindungen der obigen allgemeinen Formeln (1) und (2), stehen A und A', die gleich oder verschieden sein können, für einen Rest 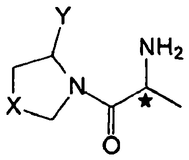 worin X für S, O, CH₂, CH₂CH₂, CH₂O oder CH₂NH steht und Y für H oder CN steht und * für ein chirales Kohlenstoff-Atom steht. Besonders bevorzugt sind erfindungsgemäß Verbindungen der allgemeinen Formeln (1), in denen A und A' gleich sind, sowie Verbindungen der allgemeinen Formeln (1) und (2), in denen in dem obigen für A stehenden Rest X für S, CH₂ oder CH₂CH₂ und/oder Y für H oder CN steht.

In weiter bevorzugten Ausführungsformen der Erfindung stellen solche Verbindungen der allgemeinen Formeln (1) und (2) Prodrugs zu besonders wirksamen Inhibitoren dar, in denen das mit * bezeichnete chirale Kohlenstoff-Atom eine S-bzw. L-Konfiguration aufweist.

In den Verbindungen der obigen allgemeinen Formeln (1) und (2) können B und B' gleich oder verschieden sein und stehen für einen O, N oder S enthaltenden oder nicht enthaltenden, unsubstituierten oder substituierten, unverzweigten oder verzweigten Alkylen-Rest, Cycloalkylen-Rest, Aralkylen-Rest, Heterocycloalkylen-Rest, Heteroarylalkylen-Rest, Arylamidoalkylen-Rest, Heteroarylamidoalkylen-Rest, unsubstituierten oder einfach oder mehrfach substituierten Arylen-Rest oder Heteroarylen-Rest mit einem oder mehreren fünf-, sechs- oder siebengliedrigen Ringen.

In der vorliegenden Beschreibung und in den Patentansprüchen wird unter dem Begriff "Alkyl-Rest" verstanden ein einwertiger geradkettiger ("unverzweigter") oder verzweigter Rest aus aneinander über Einfachbindungen gebundenen Kohlenstoffatomen mit an die Kohlenstoff-Atome gebundenen WasserstoffAtomen. Alkyl-Reste im Sinne der vorliegenden Erfindung sind also gesättigte einwertige Kohlenwassertoff-Reste. Bevorzugterweise umfassen die Alkyl-Reste in den Verbindungen der allgemeinen Formeln (1) und (2) 1 bis 18 Kohlenstoff-Atome und sind damit gewählt aus den Resten Methyl, Ethyl, n-Propyl, i-Propyl und den zahlreichen verschiedenen geradkettigen und verzweigten Isomeren der Reste Butyl, Pentyl, Hexyl, Heptyl, Octyl, Nonyl, Decyl, Undecyl, Dodecyl, Tridecyl, Tetradecyl, Pentadecyl, Hexadecyl, Heptadecyl und Octadecyl. Besonders bevorzugt sind geradkettige und verzweigte Alkyl-Reste mit 1 bis 12 Kohlenstoff-Atomen, und geradkettige und verzweigte Alkyl-Reste mit 1 bis 6 Kohlenstoffatomen sind noch mehr bevorzugt. Am meisten bevorzugt sind die Reste Methyl, Ethyl, n-Propyl, i-Propyl, n-Butyl, i-Butyl, sec-Butyl und tert-Butyl.

Entsprechend werden in der vorliegenden Beschreibung und in den Patentansprüchen unter den Begriffen "Alkenyl-Rest" und "Alkinyl-Rest" einwertige geradkettige ("unverzweigte") oder verzweigte Reste aus aneinander über Einfachbindungen und mindestens eine Doppelbindung bzw. Dreifachbindung an beliebiger, aber definierter Stelle im Molekül gebundenen Kohlenstoff-Atomen mit an die restlichen Bindungen der Kohlenstoff-Atome gebundenen Wasserstoff-Atomen verstanden, die mindestens 2 Kohlenstoff-Atome und bis zu 18 Kohlenstoff-Atome aufweisen. Als solche Reste kommen beispielsweise bevorzugt Vinyl-Reste oder Allyl-Reste infrage; Kohlenstoff-Kohlenstoff-Mehrfachbindungen aufweisende Reste sind jedoch nicht auf die beiden vorgenannten Reste beschränkt.

In der vorliegenden Beschreibung und in den Patentansprüchen wird unter dem Begriff "Atkylen-Rest" verstanden ein zweiwertiger geradkettiger ("unverzweigter") oder verzweigter Rest aus aneinander über Einfachbindungen gebundenen Kohlenstoffatomen mit an die Kohlenstoff-Atome gebundenen WasserstoffAtomen. Alkylen-Reste im Sinne der vorliegenden Erfindung sind also gesättigte zweiwertige Kohlenwassertoff-Reste. Bevorzugterweise umfassen die Alkylen-Reste in den Verbindungen der allgemeinen Formeln (1) und (2) 1 bis 18 Kohlenstoff-Atome und sind damit gewählt aus den Resten Methylen, Ethylen, n-Propylen, 2,2-Propylen, 1,2-Propylen und den zahlreichen verschiedenen geradkettigen und verzweigten Isomeren der Reste Butylen, Pentylen, Hexylen, Heptylen, Octylen, Nonylen, Decylen, Undecylen, Dodecylen, Tridecylen, Tetradecylen, Pentadecylen, Hexadecylen, Heptadecylen und Octadecylen. Besonders bevorzugt sind geradkettige und verzweigte Alkylen-Reste mit 1 bis 12 Kohlenstoff-Atomen, und geradkettige und verzweigte Alkylen-Reste mit 1 bis 6 Kohlenstoffatomen sind noch mehr bevorzugt. Am meisten bevorzugt sind die Reste Methylen, Ethylen, n-Propylen, 2,2-Propylen, 1,2-Propylen und die zahlreichen verschiedenen Butylen-Stellungsisomere.

In den Alkyl-Resten und/oder den Alkylen-Resten, die erfindungsgemäß Teil der Verbindungen der allgemeinen Formeln (1) und (2) sein können, können die Ketten aus Kohlenstoff-Atomen durch -O-Atome, -N-Atome oder -S-Atome unterbrochen sein; es können sich also im Verlauf der Kette statt einer oder mehrerer -CH₂-Gruppe(n) eine oder mehrere Gruppe(n) aus der Gruppe -O-, -NH- und -S- befinden, wobei üblicherweise nicht zwei der Gruppen -O-, -NH- und/oder -S- in der Kette aufeinander folgen. Die eine oder mehreren Gruppe(n) -O-, -NH- oder -S- können dabei an beliebigen Stellen im Molekül eingeführt sein. Vorzugsweise ist dann, wenn eine derartige Hetero-Gruppe vorhanden ist, eine derartige Gruppe im Molekül enthalten.

Sowohl geradkettige als auch verzweigte Alkyl- oder Alkylen-Reste in den Verbindungen der allgemeinen Formeln (1) und (2) können erfindungsgemäß in einer weiteren Ausführungsform mit einem oder mehreren Substituenten, vorzugsweise mit einem Substituenten, substituiert sein. Der/die Substituent(en) kann/können an beliebigen Positionen des aus Kohlenstoffatomen gebildeten Grundgerüsts stehen und kann/können vorzugsweise, ohne die Erfindung hierauf zu beschränken, gewählt sein aus der Gruppe, die besteht aus Halogenatomen wie Fluor, Chlor, Brom und lod, besonders bevorzugt Chlor und Brom, Alkyl-Gruppen mit 1 bis 6 C-Atomen, besonders bevorzugt Alkyl-Gruppen mit 1 bis 4 C-Atomen, Alkoxy-Gruppen mit 1 bis 6 C-Atomen im Alkyl-Rest, vorzugsweise mit 1 bis 3 C-Atomen im Alkyl-Rest, unsubstituierten oder mit einem oder zwei Alkylresten mit jeweils unabhängig voneinander 1 bis 6 C-Atomen, vorzugsweise 1 bis 3 C-Atomen, substituierten Amino-Gruppen, Carbonyl-Gruppen und Carboxyl-Gruppen. Letztere können auch in Form von Salzen oder Estern mit Alkoholen mit 1 bis 6 Kohlenstoff-Atomen im Alkyl-Rest vorliegen; der Begriff "Carboxyl-Gruppen" schließt also Gruppen der Grundstruktur -COO⁻ M⁺ (mit M = einwertiges Metallatom wie z. B. Alkalimetall-Atom oder entsprechendes Äquivalent eines mehrwertigen Metallatoms wie z. B. halbes Äquivalent eines zweiwertigen Metallatoms wie beispielsweise eines Erdalkalimetall-Atoms) oder der Grundstruktur -COORₓ (mit Rₓ = Alkylgruppe mit 1 bis 6 Kohlenstoff-Atomen) ein. Die substituierenden Alkyl-Gruppen sind gewählt aus den oben im einzelnen definierten Alkyl-Gruppen und sind ganz besonders bevorzugt Methyl-Gruppen, Ethyl-Gruppen, n-Propyl-Gruppen, i-Propyl-Gruppen, n-Butyl-Gruppen, i-Butyl-Gruppen, sec-Butyl-Gruppen oder tert-Butyl-Gruppen. Alkoxy-Gruppen sind Alkyl-Gruppen im oben definierten Sinn, die über ein Brücken-O-Atom an das aus Kohlenstoff-Atomen gebildete Grundgerüst gebunden sind. Sie sind bevorzugt gewählt aus der Gruppe, die besteht aus den Resten Methoxy, Ethoxy, n-Propoxy, i-Propoxy; n-Butoxy, i-Butoxy, sec-Butoxy und tert-Butoxy. Aminogruppen sind Gruppen der Grundstruktur -NRₓR_{y}, worin die Reste Rₓ und R_{y} unabhängig voneinander stehen können für Wasserstoff oder Alkyl-Gruppen (entsprechend der obigen Definition) mit 1 bis 6 Kohlenstoff-Atomen, besonders bevorzugt mit 1 bis 3 C-Atomen, wobei die Reste Rₓ und R_{y} gleich oder voneinander verschieden sein können. Besonders bevorzugte Amino-Gruppen als Substituenten sind die Gruppen -NH₂, -NH(CH₃), - N(CH₃)₂, -NH(C₂H₅) und -N(C₂H₅)₂. Unter den Begriff "Amino-Gruppen" fallen auch Gruppen der vorstehend definierten Struktur, die als quaternierte Ammonium-lonen vorliegen, entweder durch Salzbildung mit organischen Säuren oder anorganischen Säuren (d. h. Reste der Struktur RₓR_{y}R_{z}N⁺Q⁻, worin Rₓ, R_{y} und R_{z} gleich oder verschieden sein können, vorzugsweise gleich sind, und die oben für Rₓ und R_{y} definierten Bedeutungen haben können und mindestens einer der Reste Wasserstoff aus der Quaternierung mit der organischen oder anorganischen Säure ist und Q für einen Säurerest der organischen oder anorganischen Säure steht) oder durch Salzbildung mit geeigneten Quaternierungsreagentien, die dem Fachmann auf diesem Gebiet bekannt sind, wie beispielsweise (ohne Beschränkung hierauf) mit Alkylhalogeniden.

In der vorliegenden Beschreibung und in den Patentansprüchen steht der Begriff "Cycloalkyl" für unsubstituierte oder substituierte einwertige Reste aus zu geschlossenen Ringen verbundenen -CH₂-Gruppen. Diese können erfindungsgemäß bevorzugt drei bis acht Atome im Ring enthalten und können entweder ausschließlich aus Kohlenstoff-Atomen bestehen oder ein oder mehrere Heteroatom(e) enthalten, das/die gewählt ist/sind aus -O-, -S- und -NRₓ-, worin Rₓ für Wasserstoff oder einen (wie oben definierten) Alkyl-Rest mit 1 bis 6 Kohlenstoff-Atomen steht. In den Fällen, in denen Heteroatome in die Ringe eingebunden sind, können diese - bei mehreren Heteroatomen - gleich oder verschieden sein. Vorzugsweise ist im Fall der Präsenz von Heteroatomen ein Heteroatom in den Ring eingebunden. Besonders bevorzugt unter den rein carbocyclischen Ringen sind die Reste Cyclopentyl, Cyclopentenyl, Cyclopentadienyl, Cyclohexyl, Cyclohexenyl, Cyclohexadienyl, Cycloheptyl, Cycloheptenyl, Cycloheptadienyl und Cycloheptatrienyl. Beispiele für Heteroatome enthaltende Cycloalkyl-Reste, die auch als Heterocycloalkyl-Reste bezeichnet werden, sind in weiteren Ausführungsformen der Erfindung die Reste Tetrahydrofuranyl, Pyrrolidinyl, Pyrazolidinyl, Imidazolidinyl, Piperidinyl, Piperazinyl und Morpholinyl.

Mögliche Substituenten an diesen carbocyclischen oder heterocyclischen Cycloalkylresten können vorzugsweise, ohne die Erfindung hierauf zu beschränken, gewählt sein aus der obigen Gruppe von Substituenten für lineare Alkyl-Gruppen. Besonders bevorzugte Substituenten für Cycloalkyl-Gruppen sind die Substituenten -Cl, -Br, Methyl, Ethyl, n-Propyl, i-Propyl, n-Butyl, i-Butyl, sec-Butyl oder tert-Butyl, Methoxy, Ethoxy, n-Propoxy, i-Propoxy; n-Butoxy, i-Butoxy, sec-Butoxy und tert-Butoxy, -NH₂, -NH(CH₃), -N(CH₃)₂, -NH(C₂H₅) und -N(C₂H₅)₂, Carbonyl und Carboxyl.

In der vorliegenden Beschreibung und in den Patentansprüchen steht der Begriff "Cycloalkylen" für unsubstituierte oder substituierte zweiwertige Reste aus zu geschlossenen Ringen verbundenen -CH₂-Gruppen. Diese können erfindungsgemäß bevorzugt drei bis acht Atome im Ring enthalten und können entweder ausschließlich aus Kohlenstoff-Atomen bestehen oder ein oder mehrere Heteroatom(e) enthalten, das/die gewählt ist/sind aus -O-, -S- und -NRₓ-, worin Rₓ für Wasserstoff oder einen (wie oben definierten) Alkyl-Rest mit 1 bis 6 Kohlenstoff-Atomen steht. Besonders bevorzugt unter den rein carbocyclischen Ringen sind die Reste Cyclopentylen, Cyclopentenylen, Cyclopentadienylen, Cyclohexylen, Cyclohexenylen, Cyclohexadienylen, Cycloheptylen, Cycloheptenylen, Cycloheptadienylen und Cycloheptatrienylen. Auch die oben bei den Cycloalkyl-Resten definierten heterocyclischen Gruppen können in den Verbindungen der allgemeinen Formeln (1) und (2) als Gruppen "B" als zweiwertige Reste auftreten, und besonders bevorzugt sind solche cyclischen zweiwertigen Reste, in denen eine Gruppe -0- oder -NRₓ- in den Ring eingebunden ist. In diesen Fällen sind beide Valenzen an beliebigen C-Atomen im Ring lokalisiert. Bevorzugterweise ist ein Heteroatom oder sind zwei Heteroatome in den Ring eingebunden, und in besonders bevorzugten Ausführungsformen solcher Gruppen sind die zweiwertigen Reste abgeleitet von Tetrahydrofuran, Pyrrolidin, Pyrazolidin, Imidazolidin, Piperidin, Piperazin und Morpholin.

Mögliche Substituenten an diesen carbocyclischen oder heterocyclischen Cycloalkylen-Resten können vorzugsweise, ohne die Erfindung hierauf zu beschränken, gewählt sein aus der obigen Gruppe von Substituenten für lineare Alkyl-Gruppen. Besonders bevorzugte Substituenten für Cycloalkylen-Gruppen sind die Substituenten -Cl, -Br, Methyl, Ethyl, n-Propyl, i-Propyl, n-Butyl, i-Butyl, sec-Butyl oder tert-Butyl, Methoxy, Ethoxy, n-Propoxy, i-Propoxy; n-Butoxy, i-Butoxy, sec-Butoxy und tert-Butoxy, -NH₂, -NH(CH₃), -N(CH₃)₂, -NH(C₂H₅) und -N(C₂H₅)₂, Carbonyl und Carboxyl.

Im Rahmen der vorliegenden Beschreibung und in den Patentansprüchen wird unter "Aryl-Rest" ein von einem cyclischen Molekül mit aromatischen Charakter (4n + 2 in Ringorbitalen delokalisierte π-Elektronen) abgeleiteter einwertiger Kohlenwasserstoff-Rest verstanden, der unsubstituiert oder substituiert sein kann. Die Ringstruktur eines solchen Aryl-Restes kann eine fünfgliedrige, sechsgliedrige oder siebengliedrige Ringstruktur mit einem Ring oder eine aus zwei oder mehreren aneinander gebundenen ("annellierten") Ringen gebildete Struktur sein, wobei die annellierten Ringe die gleiche oder eine unterschiedliche Zahl von Ringgliedern, insbesondere von C-Atomen, haben können. Bei aus mehreren aneinander kondensierten Ringen bestehenden Systemen sind benzokondensierte Ringe besonders bevorzugt, d. h. Ringsysteme, in denen zumindest einer der Ringe ein aromatischer, nur aus C-Atomen bestehender Sechsring (Phenylring) ist. Typische, nicht jedoch beschränkende Beispiele von Aryl-Resten stellen Cyclopentadienyl-Reste (C₅H₅⁻) (als fünfgliedriger Ring), Phenyl-Reste (als sechsgliedriger Ring), Cycloheptatrienyl-Reste (C₇H₇⁺) (als siebengliedriger Ring), Naphthyl-Reste (als zwei annellierte sechsgliedrige Ringe umfassendes Ringsystem) sowie von Anthracen und Phenanthren abgeleitete einwertige Reste (drei annellierte sechsgliedrige Ringe) dar. Die erfindungsgemäß am meisten bevorzugten Aryl-Reste sind Phenyl- und Naphthyl-Reste.

Mögliche Substituenten an diesen carbocyclischen Aryl-Resten können vorzugsweise gewählt sein aus der obigen Gruppe von. Substituenten für lineare Alkyl-Gruppen, ohne die Erfindung auf diese Substituenten zu beschränken. Besonders bevorzugte Substituenten für Aryl-Gruppen sind die Substituenten -Cl, -Br, Methyl, Ethyl, n-Propyl, i-Propyl, n-Butyl, i-Butyl, sec-Butyl oder tert-Butyl, Methoxy, Ethoxy, n-Propoxy, i-Propoxy; n-Butoxy, i-Butoxy, sec-Butoxy, tert-Butoxy, -NH₂, -NH(CH₃), -N(CH₃)₂, -NH(C₂H₅) und -N(C₂H₅)₂, Carbonyl und Carboxyl. Es kann ein oder es können mehrere derartige Substituent(en), die gleich oder verschieden voneinander sein können, an einen Aryl-Rest gemäß der vorliegenden Erfindung gebunden sein. Die substituierte(n) Position(en) am Aryl-Ring(-System) kann/können beliebig gewählt sein.

Eine vergleichbare Definition wie bei den Aryl-Resten gilt im Rahmen der vorliegenden Beschreibung und der Patentansprüche bezüglich der Definition des Begriffs "Arylen-Rest": Hierunter wird ein zweiwertiger Rest verstanden, dessen grundsätzlicher Aufbau und dessen Auswahl und dessen Substituent(en) mit den vorstehenden Angaben zur Definition der "Aryl-Reste" vergleichbar sind, nur dass es sich um einen zweiwertigen Rest handelt, dessen Einbindung an beliebigen zwei Kohlenstoff-Atomen des Ringes erfolgen kann.

Im Rahmen der vorliegenden Beschreibung und der Patentansprüche wird unter "Heteroaryl-Rest" ein Arylrest (im Sinne der obigen Definition) verstanden, in dessen Ringstruktur ein Heteroatom oder mehrere Heteroatome, vorzugsweise aus der Gruppe O, N oder S, enthalten sind, ohne dass der aromatische Charakter des Moleküls dabei verloren geht. Heteroaryl-Reste gemäß der Erfindung können unsubstituiert oder substituiert sein. Die Ringstruktur eines solchen Heteroaryl-Restes kann eine fünfgliedrige, sechsgliedrige oder siebengliedrige Ringstruktur mit einem Ring oder eine aus zwei oder mehreren aneinander gebundenen ("annellierten") Ringen gebildete Struktur sein, wobei die annellierten Ringe die gleiche oder eine unterschiedliche Zahl von Ringgliedern haben können. Das/die Heteroatom(e) kann/können allein in einem oder auch in mehreren der Ringe des Ringsystems vorkommen. Die Heteroaryl-Reste bestehen vorzugsweise aus einem oder zwei Ringen. Bei aus mehreren aneinander kondensierten Ringen bestehenden Systemen sind benzokondensierte Ringe besonders bevorzugt, d. h. Ringsysteme, in denen zumindest einer der Ringe ein aromatischer carbocyclischer (d. h. nur aus C-Atomen bestehender) Sechsring ist. Besonders bevorzugte Heteroaryl-Reste sind gewählt aus Furanyl, Thiophenyl, Pyridyl, Indolyl, Cumaronyl, Thionaphthenyl, Chinolinyl (Benzopyridyl), Chinazolinyl (Benzopyrimidinyl) und Chinoxylinyl (Benzopyrazinyl).

Mögliche Substituenten an diesen Heteroaryl-Resten können vorzugsweise gewählt sein aus der obigen Gruppe von Substituenten für lineare Alkyl-Gruppen, ohne die Erfindung auf diese Substituenten zu beschränken. Besonders bevorzugte Substituenten für Heteroaryl-Gruppen sind die Substituenten -Cl, -Br, Methyl, Ethyl, n-Propyl, i-Propyl, n-Butyl, i-Butyl, sec-Butyl oder tert-Butyl, Methoxy, Ethoxy, n-Propoxy, i-Propoxy; n-Butoxy, i-Butoxy, sec-Butoxy, tert-Butoxy, -NH₂, -NH(CH₃), -N(CH₃)₂, -NH(C₂H₅) und -N(C₂H₅)₂, Carbonyl und Carboxyl. Es kann ein oder es können mehrere derartige Substituent(en), die gleich oder verschieden voneinander sein können, an einen Heteroaryl-Rest gemäß der vorliegenden Erfindung gebunden sein. Die substituierte(n) Position(en) am Heteroaryl-Ring (-System) kann/können beliebig gewählt sein.

Eine vergleichbare Definition wie bei den Heteroaryl-Resten gilt im Rahmen der vorliegenden Beschreibung und der Patentansprüche bezüglich der Definition des Begriffs "Heteroarylen-Rest": Hierunter wird ein zweiwertiger Rest verstanden, dessen grundsätzlicher Aufbau und dessen Auswahl und dessen Substituent(en) mit den vorstehenden Angaben zur Definition der "Heteroaryl-Reste" vergleichbar sind, nur dass es sich um einen zweiwertigen Rest handelt, dessen Einbindung an beliebigen zwei Kohlenstoff-Atomen des Ringes bzw. Ringsystems oder auch an einem Stickstoff-Atom erfolgen kann.

Im Rahmen der vorliegenden Beschreibung und der Patentansprüche bedeuten die nachfolgend verwendeten Begriffe "Aralkyl-Rest", "Heteroarylalkyl-Rest", "Heterocycloalkyl-Rest", "Arylamidoalkyl-Rest" und "Heteroarylamidoalkyl-Rest" Alkyl-Reste (bzw. - genauer gesagt - Alkylen-Reste) im Sinne der obigen allgemeinen und spezifischen Definition, die an einer ihrer Bindungen mit einem Aryl-Rest (gemäß der obigen allgemeinen und spezifischen Definition), Heteroaryl-Rest (gemäß der obigen allgemeinen und spezifischen Definition), Heterocyclyl-Rest (gemäß der obigen allgemeinen und spezifischen Definition der mit Heteroatomen substituierten Cycloalkyl-Reste), Arylamido-Rest (gemäß der nachfolgenden allgemeinen und spezifischen Definition) oder Heteroarylamido-Rest (gemäß der nachfolgenden allgemeinen und spezifischen Definition) substituiert sind. Diese Reste können unsubstituiert oder substituiert sein.

In bevorzugten Ausführungsformen der Erfindung sind Aralkyl-Reste solche Reste, in denen der Aryl-Rest ein Phenyl-Rest, substituierter Phenyl-Rest, Naphthyl-Rest oder substituierter Naphthyl-Rest ist und die Alkyl(en)-Gruppe geradkettig oder verzweigt ist und 1 bis 6 Kohlenstoff-Atome aufweist. Mit ganz besonderem Vorteil lassen sich als Aralkyl-Reste die Reste Benzyl, Phenethyl, Naphthylmethyl und Naphthylethyl verwenden, von denen Benzyl-Reste ganz besonders bevorzugt sind.

Mögliche Substituenten an den Aryl-Gruppen der Aralkyl-Reste können vorzugsweise gewählt sein aus der obigen Gruppe von Substituenten für lineare Alkyl-Gruppen, ohne die Erfindung auf diese Substituenten zu beschränken. Besonders bevorzugte Substituenten für Aryl-Gruppen der Aralkyl-Reste sind die Substituenten -Cl, -Br, Methyl, Ethyl, n-Propyl, i-Propyl, n-Butyl, i-Butyl, sec-Butyl oder tert-Butyl, Methoxy, Ethoxy, n-Propoxy, i-Propoxy; n-Butoxy, i-Butoxy, sec-Butoxy, tert-Butoxy, -NH₂, -NH(CH₃), -N(CH₃)₂, -NH(C₂H₅) und -N(C₂H₅)₂, Carbonyl und Carboxyl. Es kann ein oder es können mehrere derartige Substituent(en), die gleich oder verschieden voneinander sein können, an eine Aryl-Gruppe eines Aralkyl-Rest gemäß der vorliegenden Erfindung gebunden sein. Die substituierte(n) Position(en) am Aryl-Ring (-System) kann/können beliebig gewählt sein.

In bevorzugten Ausführungsformen der Erfindung sind Heteroarylalkyl-Reste solche Reste, in denen der Heteroaryl-Rest der Heteroarylalkyl-Reste gemäß der Erfindung substituiert ist und die Alkylen-Gruppe geradkettig oder verzweigt ist und 1 bis 6 Kohlenstoff-Atome aufweist. Die Ringstruktur eines solchen Heteroaryl-Restes kann eine fünfgliedrige, sechsgliedrige oder siebengliedrige Ringstruktur mit einem Ring oder eine aus zwei oder mehreren aneinander gebundenen ("annellierten") Ringen gebildete Struktur sein, wobei die annellierten Ringe die gleiche oder eine unterschiedliche Zahl von Ringgliedern haben können. Das/die Heteroatom(e) kann/können allein in einem oder auch in mehreren der Ringe des Ringsystems vorkommen. Die Heteroaryl-Reste der Heteroarylalkyl-Reste bestehen vorzugsweise aus einem oder zwei Ringen. Bei aus mehreren aneinander kondensierten Ringen bestehenden Heteroarylalkyl-Systemen sind benzokondensierte Ringe besonders bevorzugt, d. h. Ringsysteme, in denen zumindest einer der Ringe ein aromatischer carbocyclischer Sechsring ist. Besonders bevorzugte Heteroarylalkyl-Reste sind gewählt aus Furanylmethyl und -ethyl, Thiophenylmethyl und -ethyl, Pyridylmethyl und -ethyl, Indolylmethyl und -ethyl, Cumaronylmethyl und -ethyl, Thionaphthenylmethyl und -ethyl, Chinolinyl- (Benzopyridyl-) methyl und -ethyl, Chinazolinyl- (Benzopyrimidinyl-) und Chinoxylinyl- (Benzopyrazinyl)-methyl und -ethyl.

Mögliche Substituenten an diesen Heteroaryl-Gruppen der Heteroarylalkyl-Reste können vorzugsweise gewählt sein aus der obigen Gruppe von Substituenten für lineare Alkyl-Gruppen, ohne die Erfindung auf diese Substituenten zu beschränken. Besonders bevorzugte Substituenten für Heteroaryl-Gruppen sind die Substituenten -Cl, -Br, Methyl, Ethyl, n-Propyl, i-Propyl, n-Butyl, i-Butyl, sec-Butyl oder tert-Butyl, Methoxy, Ethoxy, n-Propoxy, i-Propoxy; n-Butoxy, i-Butoxy, sec.Butoxy, tert-Butoxy, -NH₂, -NH(CH₃), -N(CH₃)₂, -NH(C₂H₅) und -N(C₂H₅)₂, Carbonyl und Carboxyl. Es kann ein oder es können mehrere derartige Substituent(en), die gleich oder verschieden voneinander sein können, an einen Heteroarylalkyl-Rest gemäß der vorliegenden Erfindung gebunden sein. Die substituierte(n) Position(en) am Heteroaryl-Ring (-System) kann/können beliebig gewählt sein.

In bevorzugten Ausführungsformen der Erfindung sind Heterocycloalkyl-Reste Cycloalkyl-Reste gemäß der obigen allgemeinen oder speziellen Definition, die ein oder mehrere Heteroatom(e) enthalten, das/die gewählt ist/sind aus -O-, -S- und -NRₓ-, worin Rₓ für Wasserstoff oder einen (wie oben definierten) Alkyl-Rest mit 1 bis 6 Kohlenstoff-Atomen steht, und die Alkyl(en)-Gruppen der Heterocycloalkyl-Reste sind geradkettig oder verzweigt und weisen 1 bis 6 Kohlenstoff-Atome auf. Bei mehreren in den/die Ring(e) eingebundenen HeteroAtomen können diese gleich oder verschieden sein. Vorzugsweise ist ein Heteroatom in den Ring eingebunden. Bevorzugte Beispiele für Heteroatome enthaltende Cycloalkyl-Reste, die auch als Heterocycloalkyl-Reste bezeichnet werden, sind in weiteren Ausführungsformen der Erfindung die Reste Tetrahydrofuranyl, Pyrrolidinyl, Pyrazolidinyl, Imidazolidinyl, Piperidinyl, Piperazinyl und Morpholinyl.

Mögliche Substituenten an diesen Heterocycloalkyl-Resten können vorzugsweise gewählt sein aus der obigen Gruppe von Substituenten für lineare Alkyl-Gruppen, ohne die Erfindung auf diese Substituenten zu beschränken. Besonders bevorzugte Substituenten für Heteroaryl-Gruppen sind die Substituenten -Cl, -Br, Methyl, Ethyl, n-Propyl, i-Propyl, n-Butyl, i-Butyl, sec-Butyl oder tert-Butyl, Methoxy, Ethoxy, n-Propoxy, i-Propoxy; n-Butoxy, i-Butoxy, sec-Butoxy, tert-Butoxy, -NH₂, -NH(CH₃), -N(CH₃)₂, -NH(C₂H₅) und -N(C₂H₅)₂, Carbonyl und Carboxyl. Es kann ein oder es können mehrere derartige Substituent(en), die gleich oder verschieden voneinander sein können, an einen Heterocycloalkyl-Rest gemäß der vorliegenden Erfindung gebunden sein. Die substituierte(n) Position(en) am Heterocycloalkyl-Ring(-System) kann/können beliebig gewählt sein.

In der vorliegenden Beschreibung und in den Patentansprüchen werden unter dem Begriff "Arylamidoalkyl-Rest" und "Heteroarylamidoalkyl-Rest" Alkyl-Reste (bzw. - genauer gesagt - Alkylen-Reste) im Sinne der obigen allgemeinen und spezifischen Definition verstanden, die an einer ihrer Bindungen mit einem Arylamido-Rest oder Heteroarylamido-Rest der allgemeinen Formel Ar-NRₓ-C(=O)- oder der allgemeinen Formel Ar-C(=O)-NRₓ- substituiert sind, worin Rₓ für Wasserstoff oder einen Alkyl-Rest mit 1 bis 6 Kohlenstoff-Atomen steht und Ar für einen beliebigen Aryl-Rest oder Heteroaryl-Rest gemäß der obigen allgemeinen oder speziellen Definition steht. Diese Aryl- oder Heteroaryl-Reste können unsubstituiert oder substituiert sein. Bevorzugte Beispiele für einen Arylamidoalkyl-Rest - ohne die Erfindung insoweit zu beschränkten - sind 2-, 3- oder 4-Benzoesäure-amido-n-butyl-Reste oder 2-Nitro-3-, -4-, -5- oder -6-Benzoesäure-amido-n-butyl-Reste; bevorzugte, jedoch nicht beschränkende Beispiele für Heteroarylamidoalkyl-Reste sind 2-, 4-, 5- oder 6-Pyridin-3-carbonsäureamido-n-butyl-Reste.

Mögliche Substituenten an diesen Arylamidoalkyl-Resten und Heteroarylamidoalkyl-Resten können vorzugsweise gewählt sein aus der obigen Gruppe von Substituenten für lineare Alkyl-Gruppen, ohne die Erfindung auf diese Substituenten zu beschränken. Besonders bevorzugte Substituenten für Aryl-Gruppen bzw. Heteroaryl-Gruppen der Arylamidoalkyl-Reste und Heteroarylamidoalkyl-Reste sind die Substituenten -Cl, -Br, Methyl, Ethyl, n-Propyl, i-Propyl, n-Butyl, i-Butyl, sec-Butyl oder tert-Butyl, Methoxy, Ethoxy, n-Propoxy, i-Propoxy; n-Butoxy, i-Butoxy, sec.Butoxy, tert-Butoxy, -NH₂, -NH(CH₃), -N(CH₃)₂, -NH(C₂H₅) und -N(C₂H₅)₂, Carbonyl und Carboxyl. Es kann ein oder es können mehrere derartige Substituent(en), die gleich oder verschieden voneinander sein können, an eine Aryl- oder Heteroaryl-Gruppe der Arylamidoalkyl-Reste oder Heteroarylamidoalkyl-Reste gemäß der vorliegenden Erfindung gebunden sein. Die substituierte(n) Position(en) am aromatischen Ring(-System) kann/können beliebig gewählt sein.

Eine vergleichbare Definition wie bei den Aralkyl-Resten, Heteroarylalkyl-Resten, Heterocycloalkyl-Resten, Arylamidoalkyl-Resten und Heteroarylamidoalkyl-Resten gilt im Rahmen der vorliegenden Beschreibung und der Patentansprüche bezüglich der Definition der Begriffe "Aralkylen-Rest", "Heteroarylalkylen-Rest", "Heterocycloalkylen-Rest", "Arylamidoalkylen-Rest" und "Heteroarylamidoalkylen-Rest": Hierunter werden jeweils zweiwertige Reste verstanden, deren grundsätzlicher Aufbau und deren Auswahl und deren Substituent(en) mit den vorstehenden Angaben zur Definition der "Aralkyl-Rest", "Heteroarylalkyl-Rest", "Heterocycloalkyl-Rest", "Arylamidoalkyl-Rest" und "Heteroarylamidoalkyl-Rest" vergleichbar sind, nur dass es sich jeweils um einen zweiwertigen Rest handelt, dessen Einbindung an beliebigen zwei Kohlenstoff-Atomen des Ringes bzw. Ringsystems oder der Alkylen-Gruppe oder auch an einem Stickstoff-Atom des Heteroaryl- oder Heterocyclyl-Ring-Systems erfolgen kann.

In den allgemeinen Formeln (1) und (2) steht der Rest D für - S - S - oder - Se - Se -. Diese beiden S- bzw. Se-Atome bilden eine Brücke zwischen zwei Teilen der Moleküle der Verbindungen der allgemeinen Formeln (1) und (2) bildet, die unter natürlichen, insbesondere unter reduzierenden Bedingungen gespalten werden kann. Dabei werden zwei Molekül-Teile freigesetzt, die eine inhibierende Wirkung für die Dipeptidylpeptidase IV (DP IV) und für Peptidasen mit analoger enzymatischer Wirkung sowie für die Alanyl-Aminopeptidase N (APN) und für Peptidasen mit analoger enzymatischer Wirkung entfalten.

In der obigen allgemeinen Formel (2) steht E für die Gruppe - CH₂ - C*H (NH₂) - R⁹, worin R⁹ für einen O, N oder S enthaltenden oder nicht enthaltenden unsubstituierten oder substituierten unverzweigten oder verzweigten Alkyl-Rest, Cycloalkyl-Rest, Aralkyl-Rest, Heterocycloalkyl-Rest, Heteroarylalkyl-Rest, Arylamidoalkyl-Rest, Heteroarylamidoalkyl-Rest, unsubstituierten oder einfach oder mehrfach substituierten Aryl-Rest oder Heteroaryl-Rest mit einem oder mehreren fünf-, sechs- oder siebengliedrigen Ringen steht. Bezüglich der erfindungsgemäß verwendbaren bzw. bevorzugten Beispiele der Alkyl-Reste, Cycloalkyl-Reste, Aralkyl-Reste, Heterocycloalkyl-Reste, Heteroarylalkyl-Reste, Arylamidoalkyl-Reste, Heteroarylamidoalkyl-Reste, unsubstituierten oder einfach oder mehrfach substituierten Aryl-Reste oder Heteroaryl-Reste mit einem oder mehreren fünf-, sechs- oder siebengliedrigen Ringen sowie der für diese Reste denkbaren und bevorzugten Substituenten kann auf die obigen Definitionen der entsprechenden Reste und ihrer bevorzugten Ausführungsformen Bezug genommen werden; diese Definitionen sind in gleicher Weise auch für die Reste in der allgemeinen Formel (2) anwendbar, für die E steht.

In der obigen Formel für E bedeutet * am mit der Amino-Gruppe substituierten Kohlenstoff-Atom ein chirales Kohlenstoff-Atom. In weiter bevorzugten Ausführungsformen der Erfindung stellen solche Verbindungen der allgemeinen Formel (2) Prodrugs zu besonders wirksamen Inhibitoren dar, in denen das mit * bezeichnete chirale Kohlenstoff-Atom eine S- bzw. L-Konfiguration aufweist.

Erfindungsgemäß besonders bevorzugt steht E für substituierte 2-Aminoalkylen-Reste, beispielsweise einen 2-Amino-3-phenylpropyl-Rest, oder für unsubstituierte oder durch Heteroatome wie -S-, -S(=O)-, -N- oder -0- substituierte 2-Aminoalkylen-Reste, beispielsweise einen 2-Amino-4-methylpentyl-Rest, einen 2-Amino-4-methylthiobutyl-Rest oder einen 2-Amino-4-methylsulfoxybutyl-Rest.

In weiter bevorzugten Ausführungsformen der Erfindung stehen in den allgemeinen Formeln (1) und (2) die Reste B und/oder B' für einen Rest R¹, welcher steht für einen geradkettigen oder verzweigten Alkylen-Rest mit 1 bis 6 Kohlenstoff-Atomen. Besonders bevorzugte Verbindungen der allgemeinen Formeln (1) und (2) umfassen Reste B und/oder B' in Form einer oder mehrerer der Gruppen gewählt aus -CH₂- (Methylen), -CH₂-CH₂- (Ethylen) oder (H₃C)₂-C< (2,2-Propylen).

In alternativen, ebenfalls weiter bevorzugten Ausführungsformen stehen B und/oder B' für einen Rest -(CH₂)ₙ - R² - R³ - R⁴ -, worin n für eine ganze Zahl von 1 bis 5 steht; R² für -NH- oder -NH-C(=NH)-NH- steht, wenn R³ für O = C< oder -SO₂- steht, oder worin R² für O = C< steht, wenn R³ für - NH - steht; R⁴ für einen O, N oder S enthaltenden oder nicht enthaltenden unsubstituierten oder substituierten unverzweigten oder verzweigten Alkylen-Rest, Cycloalkylen-Rest, Aralkylen-Rest, Heterocycloalkylen-Rest, Heteroarylalkylen-Rest, unsubstituierten oder einfach oder mehrfach substituierten Arylen-Rest oder Heteroarylen-Rest mit einem oder mehreren fünf-, sechs- oder siebengliedrigen Ringen steht. Weiter bevorzugt steht n für 1 bis 5, so dass bevorzugte Beispiele des oben genannten Restes eine Methylen-Gruppe, Ethylen-Gruppe, PropylenGruppe, Butylen-Gruppe und Pentylen-Gruppe umfassen; R² und R³ bilden vorzugsweise zusammen eine Amido-Gruppe -C (=O) -NH - oder - NH - C (=O) -. Weiter bevorzugt sind solche Verbindungen der allgemeinen Formeln (1) und (2) mit Resten B und/oder B', worin B für die vorgenannte Formel steht und R⁴ einen Amino-substituierten Alkylen-Rest, beispielsweise einen Aminoethylen-Rest, oder einen unsubstituierten oder (beispielsweise mit einer Nitro-Gruppe) substituierten Phenylen-Rest oder einen unsubstituierten oder substituierten Pyridyl-2,5-en-Rest bedeutet.

In alternativen, ebenfalls weiter bevorzugten Ausführungsformen stehen B und/oder B' für einen Rest der Formel -R⁷ - R⁸ -, worin R⁷ für einen einfach oder mehrfach substituierten Benzylenrest steht und R⁸ für eine Einfachbindung oder für einen O, N oder S enthaltenden oder nicht enthaltenden unsubstituierten oder substituierten unverzweigten oder verzweigten Alkylen-Rest, Cycloalkylen-Rest, Aralkylen-Rest, Heterocycloalkylen-Rest oder Heteroarylalkylen-Rest steht, der vorzugsweise eine oder mehrere Amino-Gruppen, Carbonyl-Gruppen oder Carboxyl-Gruppen als funktionelle Gruppen aufweisen kann, oder einen unsubstituierten oder einfach oder mehrfach substituierten Arylen-Rest oder Heteroarylen-Rest mit einem oder mehreren fünf-, sechs- oder siebengliedrigen Ringen steht. Hinsichtlich der Definition der vorgenannten Reste und ihrer denkbaren erfindungsgemäßen Substituenten kann auf die vorangehenden allgemeinen oder speziellen Definitionen der jeweiligen Reste und Substituenten zurückgegriffen werden.

Weiter bevorzugt sind erfindungsgemäß Verbindungen der allgemeinen Formeln (1) oder (2), in denen B und B' gleich oder verschieden sein können und für einen Rest -(CH₂)ₙ - R² - R³ - R⁴ stehen, worin R² für -NH- oder -NH-C(=NH) -NH- steht, wenn R³ für O = C< oder -SO₂- steht, oder worin R² für O = C< steht, wenn R³ für - NH - steht, und worin R⁴ steht für
- CH(COOH)-R¹-, worin R¹ die oben angegebene Bedeutung hat, wenn R² für O = C < steht und R³ für -NH- steht; oder
- 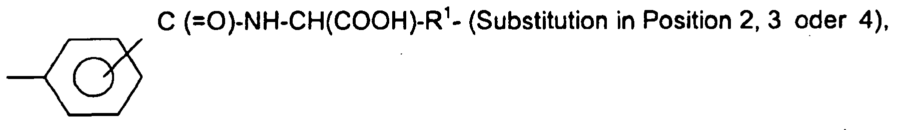 worin R¹ die oben angegebene Bedeutung hat, wenn R² für O = C < steht und R³ für -NH- steht; oder
- -CH (NHR⁵) -R¹-, wenn R² für -NH- oder -NH-C(=NH) -NH- steht und R³ für O = C< steht, worin R⁵ für H oder einen Acyl-Rest steht, vorzugsweise für einen Benzyloxycarbonyl-Rest, einen Fluoren-9-ylmethoxycarbonyl-Rest, einen tert-Butyloxycarbonyl-Rest oder einen Benzoyl-Rest steht; oder
- 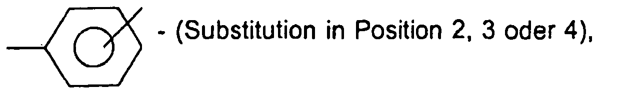 worin R⁴ für Phenylen steht und R² für -NH- oder -NH-C(=NH)-NH- steht, wenn R³ für O = C< oder -SO₂- steht, oder worin R² für O = C < steht, wenn R³ für -NH- steht; oder
- 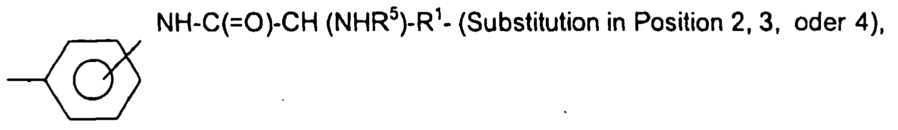 worin R⁵ für H oder einen Acyl-Rest steht, vorzugsweise für einen Benzyloxycarbonyl-Rest, einen Fluoren-9-ylmethoxycarbonyl-Rest oder einen Benzoyl-Rest steht und R² für -NH- oder -NH-C(=NH) -NHsteht, wenn R³ für O = C< oder -SO₂- steht, oder worin R² für O = C < steht, wenn R³ für -NH- steht; oder
- 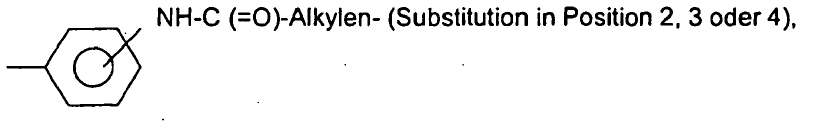 worin Alkylen für einen unverzweigten oder verzweigten Alkylen-Rest mit 1 bis 6 Kohlenstoff-Atomen steht und R² für -NH- oder -NH-C(=NH)-NH- steht, wenn R³ für O = C< oder -SO₂- steht, oder worin R² für O = C < steht, wenn R³ für -NH- steht; oder
- 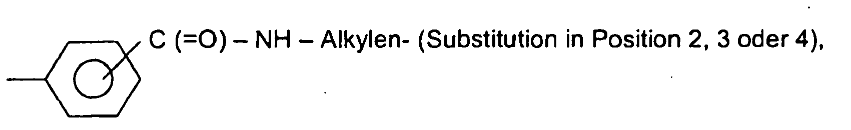 worin Alkylen für einen unverzweigten oder verzweigten Alkylen-Rest mit 1 bis 6 Kohlenstoff-Atomen steht und R² für -NH- oder -NH-C(=NH)-NH- steht, wenn R³ für O = C< oder -SO₂- steht, oder worin R² für O = C < steht, wenn R3 für-NH- steht; oder
- 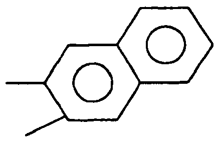 worin und R² für -NH- oder -NH-C(=NH) -NH- steht, wenn R³ für O = C< oder -SO₂- steht, oder worin R² für O = C < steht, wenn R³ für -NHsteht; oder
- 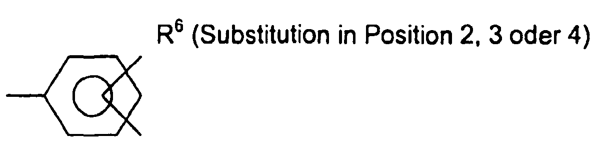 (Substitution am Ring in Abhängigkeit von der Position von R⁶), worin R⁶ für H, NO₂, CN, Halogen oder einen Acyl-Rest steht und R² für -NH- oder -NH-C(=NH) -NH- steht, wenn R³ für O = C< oder -SO₂- steht, oder worin R² für O = C < steht, wenn R³ für -NH- steht; oder
- 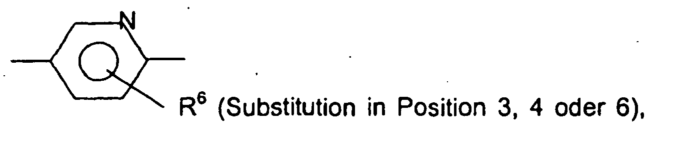 worin R⁶ für H, NO₂, CN, Halogen oder einen Acyl-Rest steht und R² für -NH- oder -NH-C(=NH) -NH- steht, wenn R³ für O = C< oder -SO₂- steht, oder worin R² für O = C < steht, wenn R³ für -NH- steht; oder
- 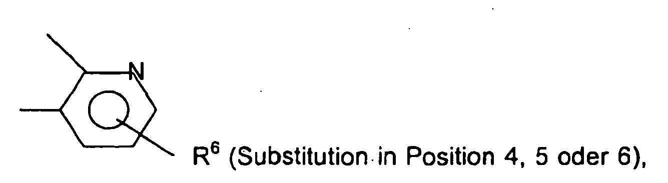 -R⁶ worin R⁶ für H, NO₂, CN, Halogen oder einen Acyl-Rest steht und R² für -NH- oder -NH-C(=NH) -NH- steht, wenn R³ für O = C< oder -SO₂- steht, oder worin R² für O = C < steht, wenn R³ für -NH- steht; oder
- 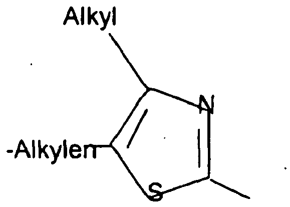 worin und R² für -NH- oder -NH-C(=NH) -NH- steht, wenn R³ für O = C < oder -SO₂- steht, oder worin R² für O = C < steht, wenn R³ für -NHsteht.

Alternativ dazu sind erfindungsgemäß Verbindungen der allgemeinen Formeln (1) und (2) weiter bevorzugt, in denen B und B' gleich oder verschieden sein können und für einen Rest - R⁷ - R⁸ - stehen, worin R⁷ und R⁸ in Kombination stehen für einen Rest
- 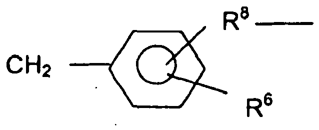 (in dem R⁷ für den obigen Rest ohne R⁸ steht und die Stellung von R⁶ in Abhängigkeit von der Position von R⁸ ist), worin R⁸ und R⁶ die oben angegebenen Bedeutungen haben, d. h. worin R⁶ für H, NO₂, CN, Halogen oder einen Acyl-Rest steht und worin R⁸ für eine Einfachbindung oder für einen O, N oder S enthaltenden oder nicht enthaltenden unsubstituierten oder substituierten unverzweigten oder verzweigten Alkylen-Rest, Cycloalkylen-Rest, Aralkylen-Rest, Heterocycloalkylen-Rest oder Heteroarylalkylen-Rest steht, der vorzugsweise eine oder mehrere Amino-Gruppen, Carbonyl-Gruppen oder Carboxyl-Gruppen als funktionelle Gruppen aufweisen kann, oder für einen unsubstituierten oder einfach oder mehrfach substituierten Arylen-Rest oder Heteroarylen-Rest mit einem oder mehreren fünf-, sechs- oder siebengliedrigen Ring(en) steht.

Noch weiter bevorzugte Verbindungen der allgemeinen Formeln (1) und (2) sind solche, in denen B und B' gleich oder verschieden sind und unabhängig voneinander für einen Rest - R⁷- R⁸ - stehen, worin R⁷ für einen einfach oder mehrfach substituierten Benzylen-Rest der vorstehenden Formel (ohne _{R}⁸) steht und R⁸ steht für
- NH - oder -C₁- bis C₆-Alkylen - NH - in Kombination mit
- -C(=O)-C₁₋ bis C₆-Alkylen - oder
- -C(=O)- Arylen - oder
- SO₂-C₁- bis C₆-Alkylen - oder
- -SO₂- Arylen - oder
- 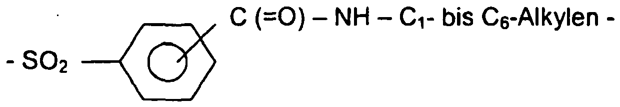
(Substitution in der Position 2, 3 oder 4) oder
- -C(=O)-CH(NHR⁵)- R¹, worin R¹ und R⁵ die oben angegebenen Bedeutungen haben; oder
- O = C< in Kombination mit
- -NH-C₁₋ bis C₆-Alkylen - oder
- -NH-Arylen - oder
- -NH-CH(COOH)- R¹ -, worin R¹ die oben genannten Bedeutungen aufweist; oder
- -O-C₁- bis C₆-Alkylen - oder
- -O-Arylen - oder
- -O-C₁- bis C₆-Alkylen -NH-C(=O)-CH(NH₂)-R¹-, worin R¹ die oben genannten Bedeutungen aufweist, oder
- -O-C₁- bis C₆-Alkylen-C(=O)-NH-CH(COOH)-R¹ -, worin R¹ die oben genannten Bedeutungen aufweist.

Erfindungsgemäß liegen die Verbindungen der allgemeinen Formeln (1) und/oder (2) in Form neutraler Moleküle vor und finden als solche erfindungsgemäße Verwendung. Alternativ dazu können die Verbindungen der allgemeinen Formeln (1) und/oder (2) auch in Form ihrer Säureadditionssalze mit anorganischen und/oder organischen Säuren vorliegen. Derartige Säureadditionssalze werden aufgrund der Präsenz basischer Zentren (meist von basischen Stickstoff-Atomen) im Molekül durch Anlagerung eines oder mehrerer Moleküle von H-aciden Verbindungen (Brönstedt-Säuren), bevorzugt eines Moleküls einer H-aciden Verbindung, gebildet und sorgen für eine verbesserte Löslichkeit der Moleküle in polaren Medien, wie beispielsweise in Wasser. Die letztgenannte Eigenschaft ist von besonderer Bedeutung für solche Verbindungen, die pharmakologische Wirkungen entfalten.

In bevorzugten Ausführungsformen der Erfindung sind die Säureadditionssalze Salze pharmazeutisch annehmbarer Säuren und sind mit Vorteil (jedoch ohne Beschränkung für die vorliegende Erfindung) gewählt aus der Gruppe, die besteht aus Hydrochloriden, Trifluoracetaten, Tartraten, Succinaten, Formiaten und/oder Citraten der Verbindungen der allgemeinen Formeln (1) oder (2).

Besonders bevorzugte und mit Vorteil verwendbare Verbindungen der allgemeinen Formel (1) sind gekennzeichnet durch die allgemeine Formel (1a) 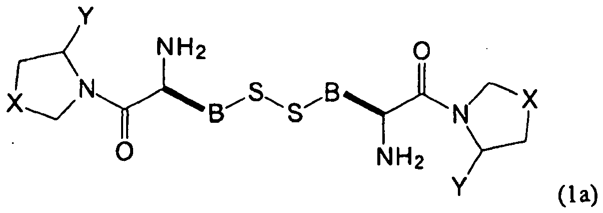 worin X, Y und B die oben angegebenen Bedeutungen haben. Mit besonderem Vorteil verwendbar und damit auch Gegenstand der Erfindung sind Säureadditionssalze der Verbindung der allgemeinen Formel (1a), bevorzugt ihre Säureadditionssalze mit pharmazeutisch annehmbaren anorganischen und/oder organischen Säuren, insbesondere mit Säuren aus der vorstehend genannten Gruppe.

Ganz besonders bevorzugte Verbindungen der allgemeinen Formel (1a) ergeben sich aus der nachfolgenden Tabelle 1, ohne dass die Erfindung auf diese Verbindungen beschränkt ist:

**Tabelle 1**

| **Beispiele Verbindungen der allgemeinen Formel A - B - D - B' - A' (1)** | | | | |
|---|---|---|---|---|
| **Nr.** | **B** | **X** | **Y** | **Summen-Formel** |
| I | -CH₂- | -CH₂- | H | C₁₄H₂₆N₄O₂S₂ |
| II | -CH₂- | S | H | C₁₂H₂₂N₄O₂S₄ |
| III | -CH₂- | -CH₂- | CN | C₁₆H₂₄N₆O₂S₂ |
| IV | 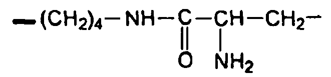 | S | H | C₂₄H₄₆N₈O₄S₄ |
| V | 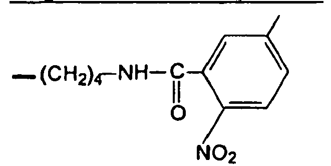 | S | H | C₃₂H₄₂N₈O₈S₄ |
| VI | 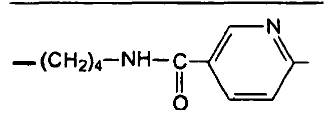 | S | H | C₃₀H₄₂N₈O₄S₄ |

und ihre Säureadditionssalze, bevorzugt ihre Säureadditionssalze mit pharmazeutisch annehmbaren anorganischen und/oder organischen Säuren, vorzugsweise aus der o. g. Gruppe pharmazeutisch annehmbarer Säuren.

Besonders bevorzugte und mit Vorteil verwendbare Verbindungen der allgemeinen Formel (2) sind gekennzeichnet durch die allgemeine Formel (2a) 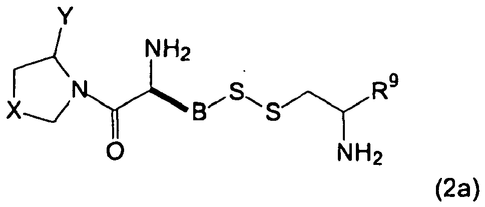 worin X, Y, R⁹ und B die oben angegebenen Bedeutungen haben, und ihre Säureadditionssalze, bevorzugt ihre Säureadditionssalze mit pharmazeutisch annehmbaren anorganischen und/oder organischen Säuren, vorzugsweise aus der o. g. Gruppe pharmazeutisch annehmbarer Säuren.

Ganz besonders bevorzugte Verbindungen der allgemeinen Formel (2a) ergeben sich aus der nachfolgenden Tabelle 2, ohne dass die Erfindung auf diese Verbindungen beschränkt ist:

**Tabelle 2**

| **Beispiele Verbindungen der allgemeinen Formel A - B - D - E (2)** | | | | | |
|---|---|---|---|---|---|
| **Nr.** | **B** | **R⁹** | **X** | **Y** | **Summen-Formel** |
| VII | -CH₂- | 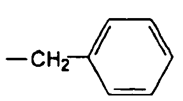 | S | H | C₁₅H₂₃N₃OS₃ |
| VIII | 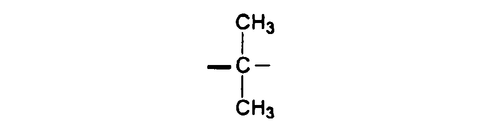 | 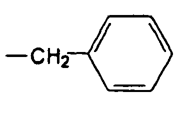 | S | H | C₁₇H₂₇N₃OS₃ |
| IX | 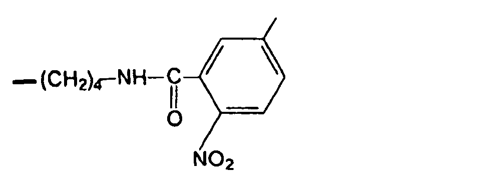 | 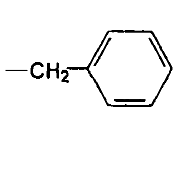 | S | H | C₂₅H₃₃N₅O₄S₃ |
| X | 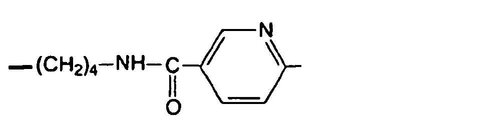 | 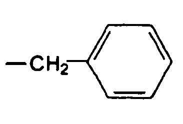 | S | H | C₂₄H₃₃N₅O₂S₃ |
| XI | 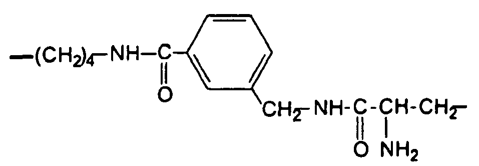 | 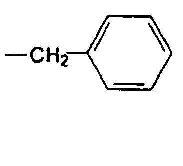 | S | H | C₂₉H₄₂N₆O₃S₃ |
| XII | 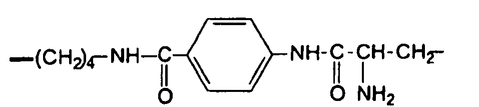 | 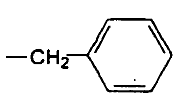 | S | H | C₂₈H₄₀N₆O₃S₃ |
| XIII | 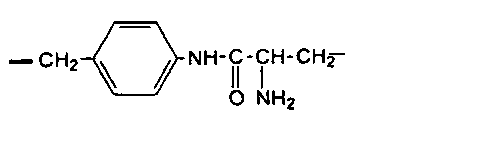 | 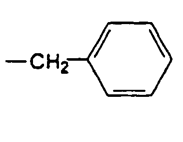 | S | H | C₂₄H₃₃N₅O₂S₃ |

und ihre Säureadditionssalze, bevorzugt ihre Säureadditionssalze mit pharmazeutisch annehmbaren anorganischen und/oder organischen Säuren.

Die Erfindung betrifft auch ein Verfahren zur Herstellung von Verbindungen der allgemeinen Formeln

A-B-D-B'-A' (1)

und

A-B-D-E (2).

In den vorgenannten Formeln (1) und (2) haben A, A', B, B', D und E die oben im einzelnen angegebenen Bedeutungen. Bei dem Verfahren zur Herstellung der neuen Verbindungen (1) und/oder (2) geht man so vor, dass man gemäß dem oben angegebenen Syntheseschema 1 entweder
- Verbindungen der allgemeinen Formel 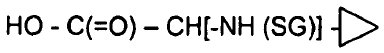 worin (SG) eine Schutzgruppe bedeutet und 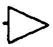 für ein Strukturelement von B steht, mit einer heterocyclischen Verbindung der allgemeinen Formel 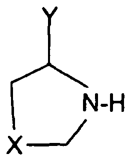 umsetzt, worin X für S, O, CH₂, CH₂CH₂. CH₂O oder CH₂NH steht und Y für H oder CN steht; das erhaltene Kondensationsproduktder Formel 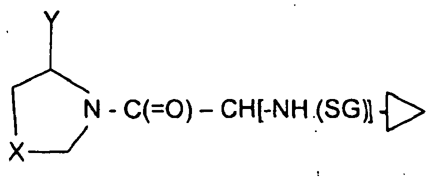 mit einer Verbindung der allgemeinen Formel 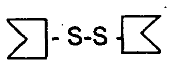 umsetzt, worin 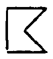 für ein Strukturelement von B steht; und das erhaltene Reaktionsprodukt unter Abspaltung der Schutzgruppen (SG) in eine Verbindung der allgemeinen Formel A - B - D - B' - A' (1) überführt, in der A und A' gleich oder verschieden sein können und B und B' gleich oder verschieden sein können und worin A, A', B, B' und D die oben angegebenen Bedeutungen haben können; oder
- Verbindungen der allgemeinen Formel 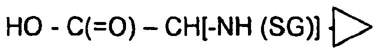 worin (SG) eine Schutzgruppe bedeutet und 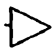 für ein Strukturelement von B steht, mit einer heterocyclischen Verbindung der allgemeinen Formel 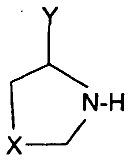 umsetzt, worin X für S, O, CH₂, CH₂CH₂, CH₂O oder CH₂NH steht und Y für H oder CN steht; das erhaltene Kondensationsprodukt der Formel 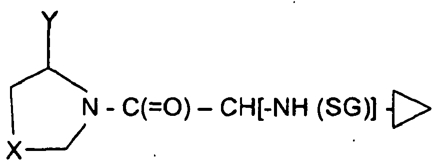 mit einer Verbindung der allgemeinen Formel 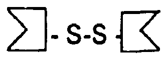 umsetzt, worin 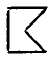 für ein Strukturelement von B steht; und das erhaltene Reaktionsprodukt mit einer Verbindung der allgemeinen Formel

   HS - CH₂ - CH [- NH - (SG)] - (_{R}⁹)

   und nach Abspaltung der Schutzgruppen (SG) zu einer Verbindung der allgemeinen Formel A - B - D - E (2) umsetzt, worin A, B, D und E die oben angegebenen Bedeutungen haben können; oder
- eine Verbindung der allgemeinen Formel 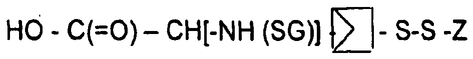 worin (SG) für eine Schutzgruppe steht, 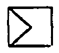 für ein Strukturelement von B steht und Z für einen Rest steht, der eine -S-S-Gruppe für einen Thiolaustausch aktiviert, mit einer heterocyclischen Verbindung der allgemeinen Formel 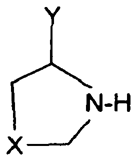 umsetzt, worin X für S, O, CH₂, CH₂CH₂, CH₂O oder CH₂NH steht und Y für H oder CN steht; das erhaltene Kondensationsprodukt der Formel 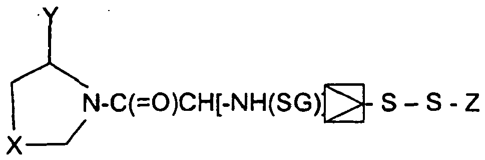 mit einer mit einer Verbindung der allgemeinen Formel

   HS - CH₂ - CH [- NH - (SG)] - (R⁹)

   und nach Abspaltung der Schutzgruppen (SG) zu einer Verbindung der allgemeinen Formel A - B - D - E (2) umsetzt, worin A, B, D und E die oben angegebenen Bedeutungen haben können; oder
- eine Verbindung der allgemeinen Formel 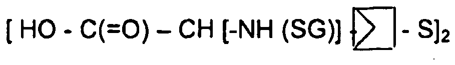 worin SG für eine Schutzgruppe steht und 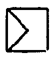 für ein Strukturelement von B steht, mit einer heterocyclischen Verbindung der allgemeinen Formel 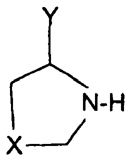 umsetzt, worin X für S, O, CH₂, CH₂CH₂, CH₂O oder CH₂NH steht und Y für H oder CN steht; und das erhaltene Reaktionsprodukt mit einer Verbindung der allgemeinen Formel

   HS - CH₂ - CH [- NH - (SG)] - R⁹

   zu einer Verbindung der allgemeinen Formel A - B - D - E (2) umsetzt, worin A, B, D und E die oben angegebenen Bedeutungen haben können; oder
- eine Verbindung der allgemeinen Formel 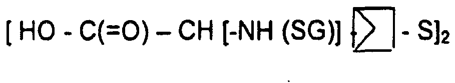 worin SG für eine Schutzgruppe steht und 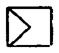 für ein Strukturelement von B steht, mit einer heterocyclischen Verbindung der allgemeinen Formel 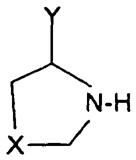 umsetzt, worin X für S, O, CH₂, CH₂CH₂, CH₂O oder CH₂NH steht und Y für H oder CN steht; und das erhaltene Reaktionsprodukt unter Abspaltung der Schutzgruppen SG in eine Verbindung der allgemeinen Formel A-B-D - B' - A' (1) überführt, in der A und A' gleich oder verschieden sein können und B und B' gleich oder verschieden sein können und worin A, A', B, B' und D die oben angegebenen Bedeutungen haben können; oder
- Verbindungen der allgemeinen Formel 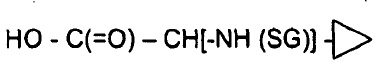 worin (SG) eine Schutzgruppe bedeutet und 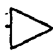 für ein Strukturelement von B steht, mit einer heterocyclischen Verbindung der allgemeinen Formel 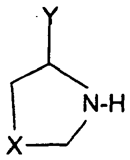 umsetzt, worin X für S, O, CH₂, CH₂CH₂, CH₂O oder CH₂NH steht und Y für H oder CN steht; das erhaltene Kondensationsprodukt der Formel 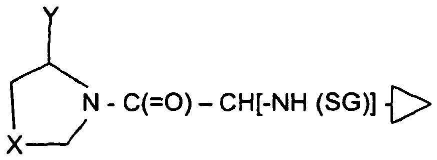 mit einer Verbindung der allgemeinen Formel 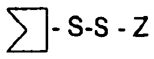 umsetzt, worin 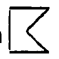 für ein Strukturelement von B steht und Z für einen Rest steht, der eine -S-S-Gruppe für einen Thiolaustausch aktiviert, und (SG) für eine Schutzgruppe steht; und das erhaltene Reaktionsprodukt der Formel 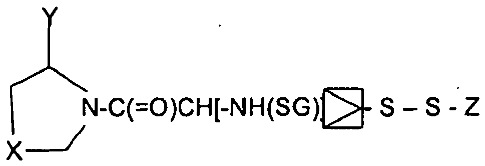 mit einer mit einer Verbindung der allgemeinen Formel

   HS-CH₂-CH[-NH-(SG)]-(R⁹)

   und nach Abspaltung der Schutzgruppen (SG) zu einer Verbindung der allgemeinen Formel A - B - D - E (2) umsetzt, worin A, B, D und E die oben angegebenen Bedeutungen haben können.

Die im Rahmen der vorstehenden Verfahrensschritte in Synthese-Zwischenstufen der Verbindungen der allgemeinen Formeln (1) und (2) eingeführten Schutzgruppen ("SG") können beliebige Schutzgruppen sein, die ein Fachmann im Bereich der organischen Synthese aus der praktischen Erfahrung kennt, und sie unterliegen erfingdungsgemäß keinen Beschränkungen. Bevorzugte Schutzgruppen sind Schutzgruppen zum Schutz von Aminosäure-Seitenketten wie beispielsweise Urethan-Schutzgruppen, z. B. Benzyloxycarbonyl-Reste, Fluoren-9-ylmethoxycarbonyl-Reste, tert-Butyloxycarbonyl-Reste usw..

Die im Rahmen der vorstehenden Verfahrensschritte in Synthese-Zwischenstufen der Verbindungen der allgemeinen Formeln (1) und (2) eingeführten Gruppen Z stehen für Reste, die ein eine -S-S-Gruppe enthaltendes Molekül für einen Thiol-Austausch aktivieren, also einen Austausch einer an das S-Atom gebundenen Gruppe gegen ein (gegebenenfalls mit einem Substituenten versehenes) S-Atom eines anderen Moleküls. Solche Gruppen sind dem Fachmann auf dem Gebiet der organischen Synthese hinlänglich bekannt, und die Erfindung ist nicht auf bestimmte Gruppen beschränkt, die für einen Thiol-Austausch aktivieren. Bevorzugte, nicht jedoch beschränkende Beispiele sind die Gruppen 3-Nitro-2-pyridyl, 5-Nitro-2-pyridyl, 2-, 3- oder 4-Pyridyl, 2-Nitrophenyl, Methoxycarbonyl oder N-Methyl-N-phenylcarbamoyl.

Die in dem allgemeinen Reaktionsschema für die zu den Verbindungen der allgemeinen Formeln (1) und (2) führenden Synthesen sowie in den vorstehenden und in den nachfolgenden Beschreibungen der Verfahrensschritte, die zu Verbindungen der allgemeinen Formeln (1) und (2) führen, verwendeten Symbole 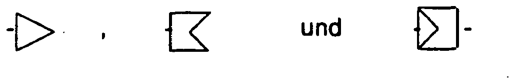 bedeuten Strukturelemente der Reste B bzw. B' in den Verbindungen der allgemeinen Formeln (1) und (2). Unter "Strukturelementen" wird in diesem Zusammenhang verstanden, dass die Reste B bzw. B' aus Molekülteilen mit jeweils einer reaktiven Gruppe aufgebaut sind, die im Rahmen einer organischen Synthese unter Bildung einer kovalenten Bindung zwischen den beiden reaktiven Gruppen zusammengefügt werden, die dadurch benachbarte Bindungspartner im neu gebildeten Molekül werden. Besondere, nicht jedoch beschränkende Beispiele für solche Strukturelemente sind eine Carboxyl-Gruppe in einem und eine Hydroxy-Gruppe im zweiten Molekül, die unter Bildung einer Ester-Gruppierung unter Abspaltung von Wasser zusammengefügt werden, oder eine Carboxyl-Gruppe in einem und eine Amino-Gruppe im zweiten Molekül, die unter Bildung einer Amido-Gruppe unter Abspaltung von Wasser zusammengefügt werden. Dies wird durch die Zusammenfügung der beiden vorstehend links aufgeführten Symbole zu dem oben rechts aufgeführten symbolisiert.

Mit besonderem Vorteil kann man die Verbindungen (1) und/oder (2) nach Verfahren herstellen, die in den folgenden Synthese-Schemata 2 bis 17 angegeben sind:

Eine Verbindung der allgemeinen Formel

HO-C(=O)-CH[-NH(SG)¹]-(CH₂)ₙ-R²-(SG)²

worin (SG)¹ eine Schutzgruppe für die Aminogruppe bedeutet, (SG)² eine Schutzgruppe am Substituenten R² bedeutet und R² und n die oben angegebenen allgemeinen oder speziellen Bedeutungen haben können, wird mit einer heterocyclischen Verbindung der allgemeinen Formel 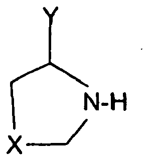 umgesetzt, worin X für S, O, CH₂, CH₂CH₂, CH₂O oder CH₂NH steht und Y für H oder CN steht; das erhaltene Kondensationsprodukt der Formel 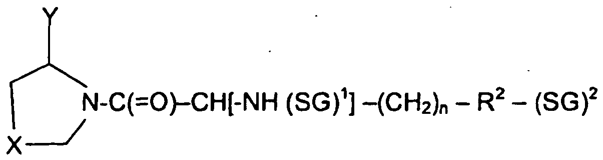 wird nach Abspaltung der Schutzgruppe (SG)² mit einer Verbindung der allgemeinen Formel

[F¹-R³-R⁴-S-]₂

umgesetzt, worin R³ und R⁴ die oben angegebenen allgemeinen und speziellen Bedeutungen haben können und F¹ für einen Teil einer funktionellen Gruppe steht, die mit einem Teil einer anderen funktionellen Gruppe reagieren kann; und das erhaltene Reaktionsprodukt wird unter Abspaltung der Schutzgruppe (SG)¹ in eine Verbindung der allgemeinen Formel A - B - D - B' - A' (1) überführt, in der A und A' gleich oder verschieden sein können und B und B' gleich oder verschieden sein können und worin A, A', B, B' und D die oben angegebenen Bedeutungen haben können; im Fall des nachfolgenden Reaktionsschemas 2 sind A und A' gleich und stehen für die oben angegebene, X und Y enthaltende heterocyclische Verbindung mit am Stickstoff gebundener Carbonylamino-Gruppe; B und B' sind ebenfalls gleich und stehen für die Gruppierung der Formel -(CH₂)ₙ-R²-R³-R⁴- mit den oben angegebenen allgemeinen oder speziellen Bedeutungen für n, R², R³ und R⁴, und D steht für -S-S-.

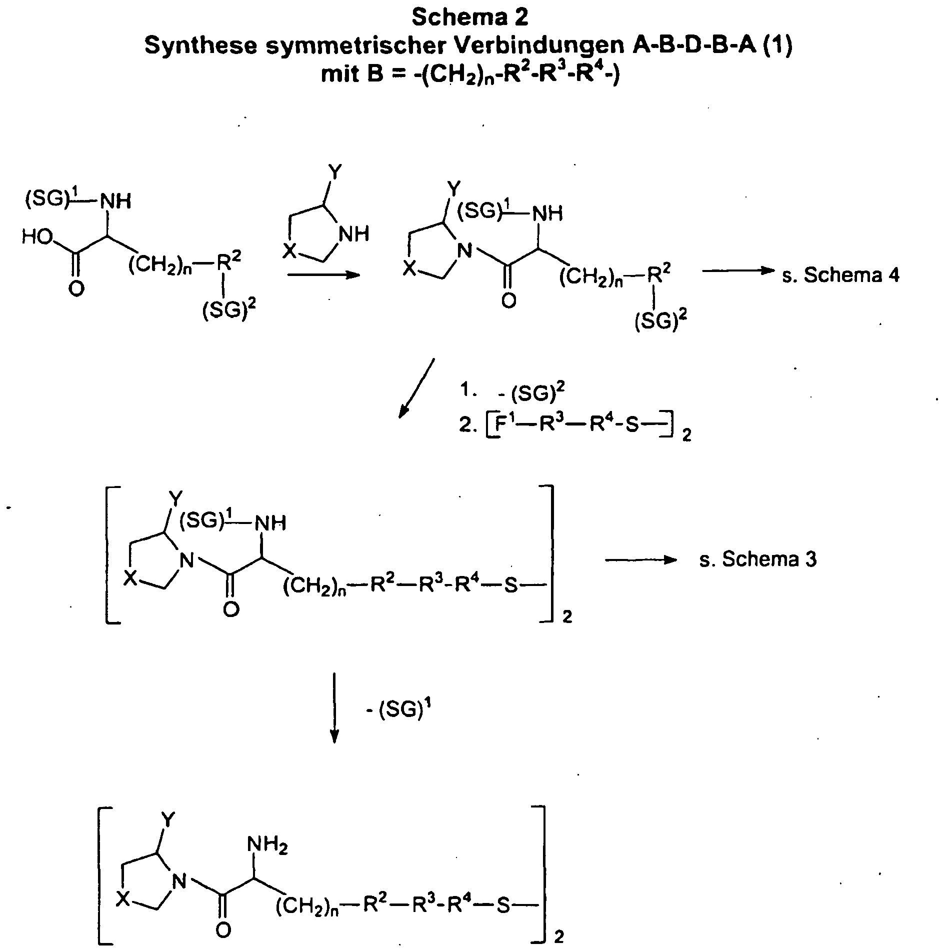

Die Schutzgruppen (SG)¹ und (SG)² sind allgemein Schutzgruppen, wie sie der Fachmann im Bereich der organischen Synthese für den zwischenzeitlichen Schutz bestimmter Gruppen in organischen Molekülen kennt, und sind bevorzugterweise Urethanschutzgruppen, die beispielsweise als Aminosäure-Schutzgruppen üblicherweise verwendet werden. Nicht beschränkende Beispiele sind ein Benzyloxycarbonyl-Rest, ein Fluoren-9-ylmethoxycarbonyl-Rest oder ein tert-Butyloxycarbonyl-Rest.

Unter Verwendung der ersten Reaktionsstufen des vorstehend gezeigten Reaktionsschemas 2 kann man - ausgehend von dem durch Umsetzung mit der Verbindung der Formel [F¹ - R³ - R⁴ - S -]₂ entstandenen Dimer - durch Umsetzung mit einer Verbindung der allgemeinen Formel HS - CH₂ - CH (R⁹) - NH (SG)³ , worin R⁹ die oben angegebene allgemeine und spezielle Bedeutung hat und (SG)³ eine Schutzgruppe für die Aminogruppe ist, unter Abspaltung der Schutzgruppe (SG)³ unsymmetrische Verbindungen der Formel A - B - D - E (2) erhalten, wie das nachfolgende Reaktionsschema 3 zeigt:

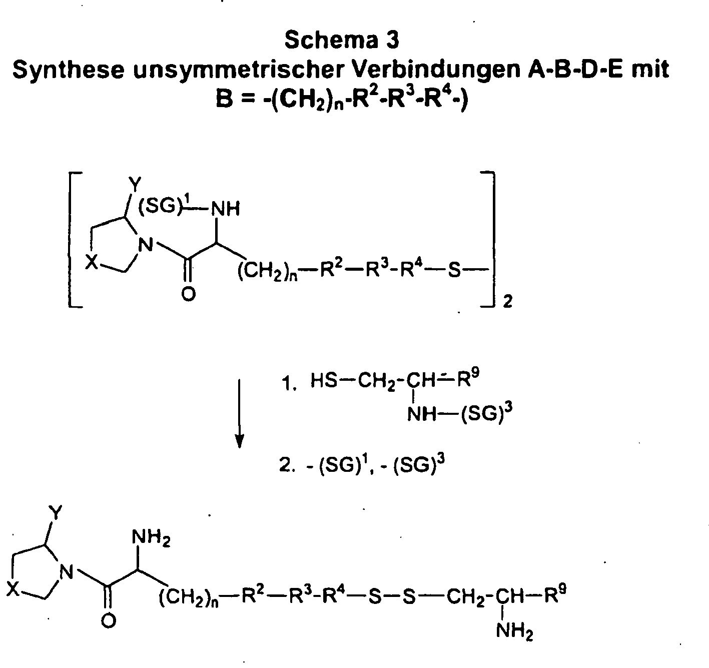

In der erhaltenen unsymmetrischen Verbindung der allgemeinen Formel A - B - D - E (2) steht A für die oben angegebene, X und Y enthaltende heterocyclische Verbindung mit am Stickstoff gebundenem α-Aminocarbonyl-Rest; B steht für die Gruppierung der Formel - (CH₂)ₙ - R² - R³ - R⁴ - mit den oben angegebenen allgemeinen oder speziellen Bedeutungen für n, R², R³ und R⁴, D steht für -S-S- und E steht für die Gruppierung - CH₂ - CH (NH₂) - R⁹, worin R⁹ die oben angegebenen allgemeinen und speziellen Bedeutungen aufweist.

In einem zweiten Syntheseweg zu unsymmetrischen Verbindungen wird - ausgehend von der ersten Zwischenstufe des obigen Reaktionsschemas 2 - der Rest B in mehreren Reaktionsschritten unter Erhalt desselben Endproduktes wie in der Reaktion nach Reaktionsschema 3 aufgebaut, wie sich aus dem nachfolgenden Reaktionsschema 4 ergibt:

Die vorgenannte erste Zwischenstufe (siehe Reaktionsschema 2) wird unter Abspaltung der Schutzgruppe (SG)² in eine Verbindung mit geeigneter Abgangsgruppe F² für die nachfolgende Reaktion mit der Verbindung der allgemeinen Formel F³ - R³ - R^{4a} - (SG)⁴ überführt; darin bedeuten F² und F³ Teile funktioneller (Abgangs-) Gruppen, hat R³ die oben angegebene allgemeine und spezielle Bedeutung, steht R^{4a} für einen Teil der Gruppe R⁴ und ist (SG)⁴ eine Schutzgruppe. Letztere wird nach der Abspaltung in eine für die nachfolgende Reaktion geeignete Abgangsgruppe F⁴ überführt. Die Umsetzung mit einer Verbindung der allgemeinen Formel F⁵ - R^{4b} - S - S - Z, worin F⁵ eine geeignete funktionelle Abgangsgruppe bedeutet, R^{4b} für einen zweiten Teil von R⁴ steht und Z ein Rest ist, der das Molekül für einen Thiolaustausch aktiviert, und die anschließende Umsetzung mit dem Thiol der allgemeinen Formel HS - CH₂ - CH (R⁹) - NH (SG)³ führen nach Abspaltung der beiden Schutzgruppen (SG)¹ und (SG)³ zur unsymmetrischen Verbindung der allgemeinen Formel A - B - D - E (2), worin A für die oben angegebene, X und Y enthaltende heterocyclische Verbindung mit am Stickstoff gebundenem α-Aminocarbonyl-Rest steht; B steht für die Gruppierung der Formel - (CH₂)ₙ - R² - R³ - R⁴ - mit den oben angegebenen allgemeinen oder speziellen Bedeutungen für n, R², R³ und R⁴, D steht für -S-S- und E steht für die Gruppierung - CH₂ - CH (NH₂) - R⁹, worin R⁹ die oben angegebenen allgemeinen und speziellen Bedeutungen aufweist.

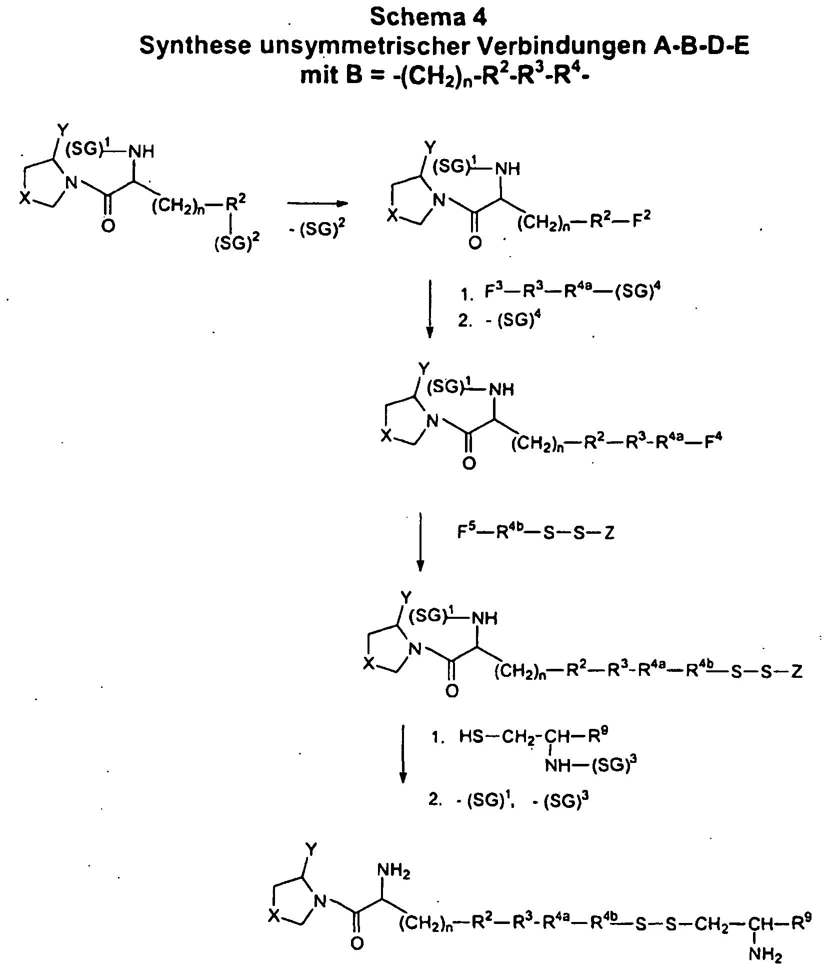

In dem vorangehenden Reaktionsschema 4 stehen (SG)¹, (SG)², (SG)³ und (SG)⁴ allgemein für Schutzgruppen, wie sie der Fachmann im Bereich der organischen Synthese für den zwischenzeitlichen Schutz bestimmter Gruppen in organischen Molekülen kennt, und sind bevorzugterweise Urethanschutzgruppen, die beispielsweise als Aminosäure-Schutzgruppen üblicherweise verwendet werden. Nicht beschränkende Beispiele sind ein Benzyloxycarbonyl-Rest, ein Fluoren-9-ylmethoxycarbonyl-Rest oder ein tert-Butyloxycarbonyl-Rest.

Die Reste F², F³, F⁴ und F⁵ sind Teile von funktionellen Gruppen und sind insbesondere geeignete, aus dem Molekül abspaltbare Abgangsgruppen wie beispielsweise -H, -OH, -Cl usw.. Z steht für Reste, die ein eine -S-S-Gruppe enthaltendes Molekül für einen Thiol-Austausch aktivieren, also einen Austausch einer an das S-Atom gebundenen Gruppe gegen ein (gegebenenfalls mit einem Substituenten versehenes) S-Atom eines anderen Moleküls. Solche Gruppen sind dem Fachmann auf dem Gebiet der organischen Synthese hinlänglich bekannt, und die Erfindung ist nicht auf bestimmte Gruppen beschränkt, die für einen Thiol-Austausch aktivieren. Bevorzugte, nicht jedoch beschränkende Beispiele sind die Gruppen 3-Nitro-2-pyridyl, 5-Nitro-2-pyridyl, 2-, 3- oder 4-Pyridyl, 2-Nitrophenyl, Methoxycarbonyl oder N-Methyl-N-phenylcarbamoyl. In dem obigen Schema bedeuten die Reste R^{4a} und R^{4b} gemeinsam den Rest R⁴ mit der oben angegebenen allgemeinen und speziellen Bedeutung.

Die Ausgangsverbindung des nachfolgenden Reaktionsschemas 5, worin R¹ die oben allgemein und im Detail angegebene Bedeutung hat, kann mit der oben angegebenen heterocyclischen Verbindung der allgemeinen Formel 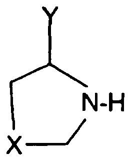 unter Abspaltung der Schutzgruppe (SG)¹ direkt zu symmetrischen Verbindungen der allgemeinen Formel A - B - D - B' - A' (1) mit B = R¹ umgesetzt werden, worin X, Y und (SG)¹ die oben allgemein und speziell angegebenen Bedeutungen haben:

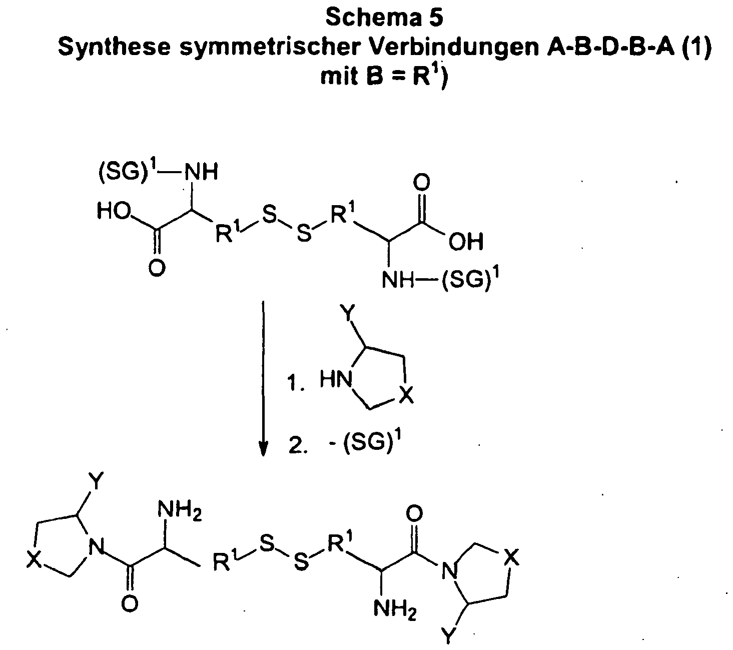

Auf dem vorgenannten Reaktionsweg ist beispielsweise ein Endprodukt zugänglich, in dem Y für CN steht, indem man die vorgenannte Reaktion mit der heterocyclischen Verbindung 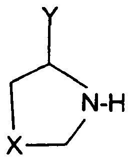 durchführt, in der Y für - C (=0) - NH₂ steht, und das symmetrische Reaktions produkt, in dem Y nach wie vor für - C (=0) - NH₂ steht, Bedingungen aussetzt, unter denen sich die Carbonylamino-Gruppe unter Wasserabspaltung in einen Cyano-Rest umwandelt. Diese Reaktion ergibt sich aus dem nachfolgenden Reaktionsschema 5a:

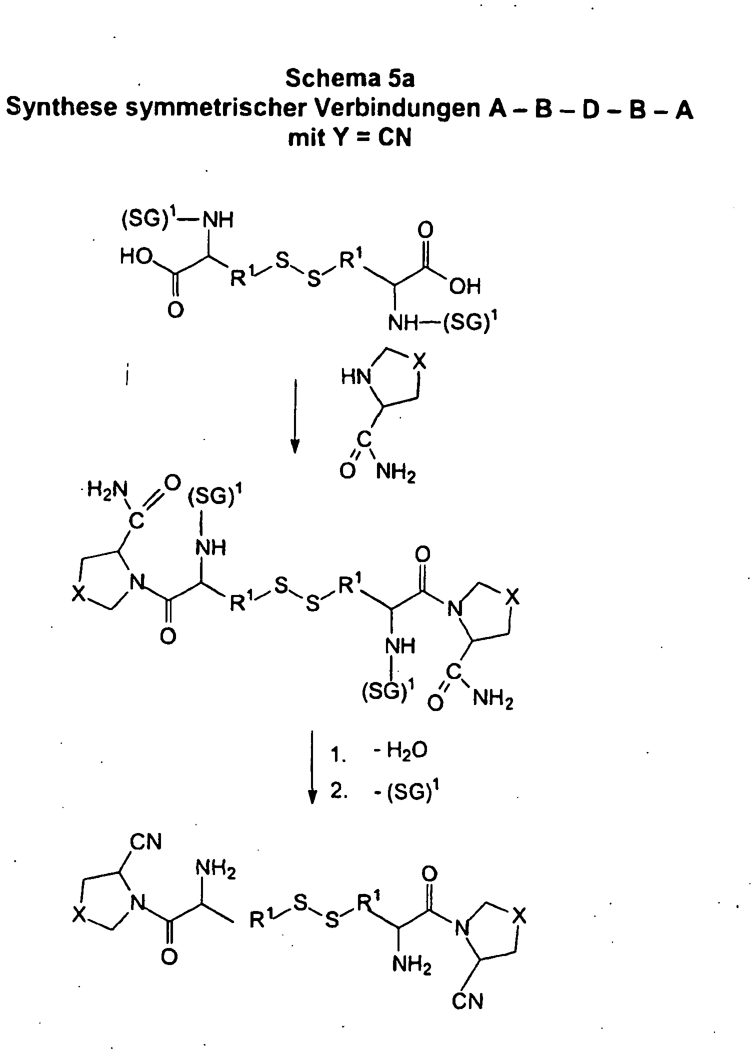

Unsymmetrische Verbindungen der allgemeinen Formel A - B - D - E, in denen B für R¹ steht und R¹ die oben angegebenen allgemeinen und speziellen Bedeutungen hat, sind aus dem N-geschützten Reaktionsprodukt des Reaktionsschemas 5 (siehe oben) zugänglich durch Umsetzung mit einer ThiolVerbindung der allgemeinen Formel HS - CH₂ - CH [- NH (SG)³] - R⁹ und Abspaltung der Schutzgruppen, wie in Reaktionsschema 6 gezeigt ist:

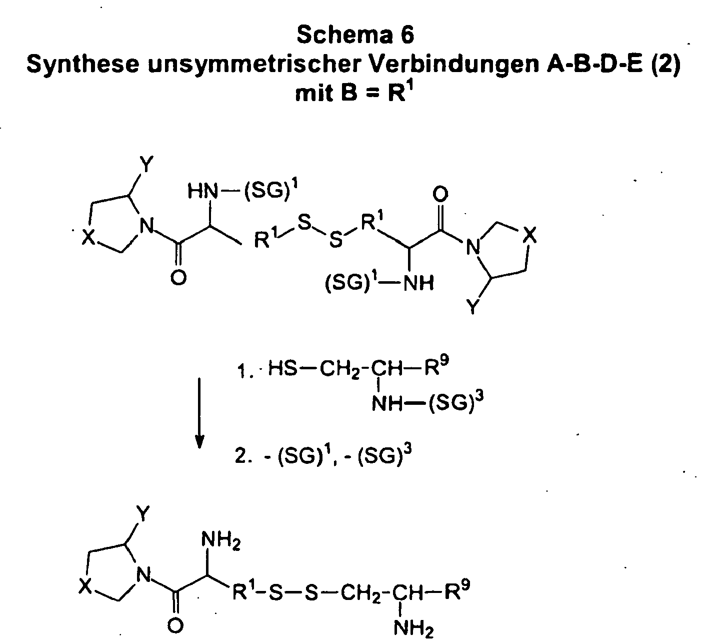

Dieselben unsymmetrischen Verbindungen A - B - D - E (2) mit B = R¹ wie in Reaktionsschema 6 sind auch auf dem in Reaktionsschema 7 angegebenen Syntheseweg zugänglich, in dem die Ausgangsverbindung zuerst mit der heterocyclischen Verbindung 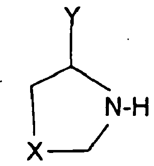 umgesetzt wird. Die nachfolgende Umsetzung mit der Thiolverbindung der allgemeinen Formel HS - CH₂ - CH [- NH (SG)³] - R⁹ und Abspaltung der Schutzgruppen (SG)¹ und (SG)³ führt ebenfalls zu den unsymmetrischen Verbindungen A - B - D - E (2) mit B = R¹, worin R¹ die oben angegebenen allgemeinen und speziellen Bedeutungen haben kann. 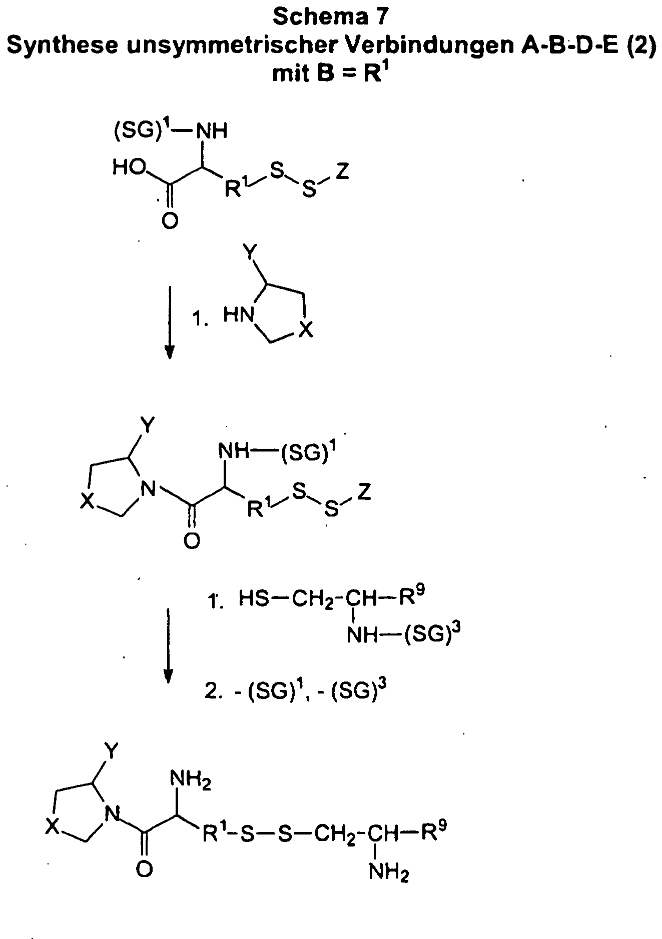 Die nachfolgenden Synthese-Schemata 8 und 9 zeigen Wege zur Synthese symmetrischer Verbindungen A-B-D-B-A(1) mit B=-R⁷-R⁸- (Schema 8) und - ausgehend vom N-geschützten Produkt der Reaktion des Schemas 8 - zur Synthese unsymmetrischer Verbindungen A-B-D-E-(2) auf Synthesewegen, die ähnlich den oben bereits beschriebenen sind:

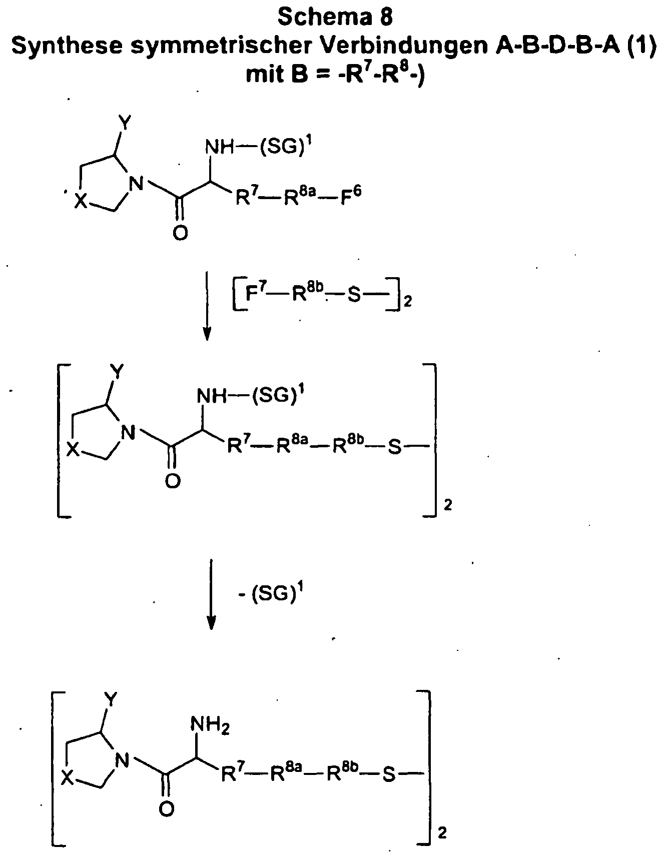

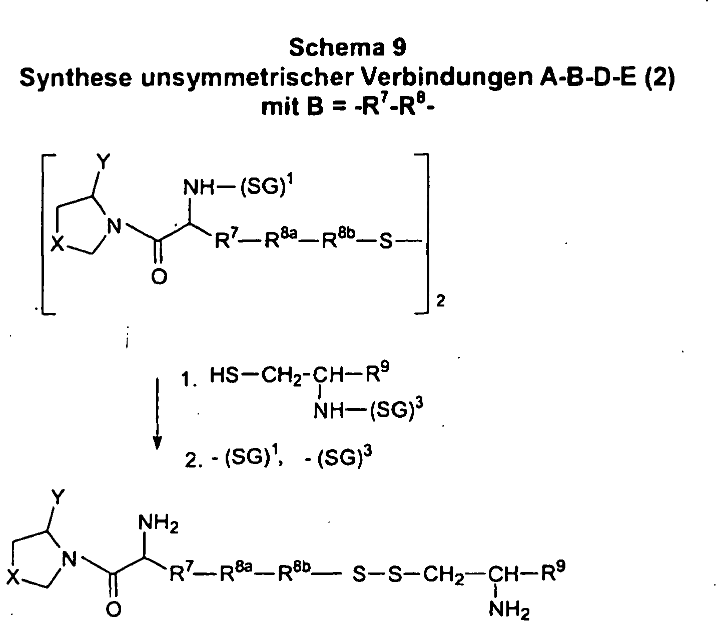

In den Reaktionsschemata 8 und 9 bedeuten die Reste R^{8a} und R^{8b} Reste, die sich zu R⁸ im Wege zweier Syntheseschritte zusammensetzen.

Zwei weitere Synthesewege, die zu den auch im Wege der Synthese nach Reaktionsschema 9 zugänglichen unsymmetrischen Verbindungen A - B - D - E (2) mit B = - R⁷ - R⁸ - führen und mit den oben bereits beschriebenen Synthesereaktionen vergleichbar sind, werden in den beiden nachfolgenden Synthese-Schemata 10 und 11 gezeigt:

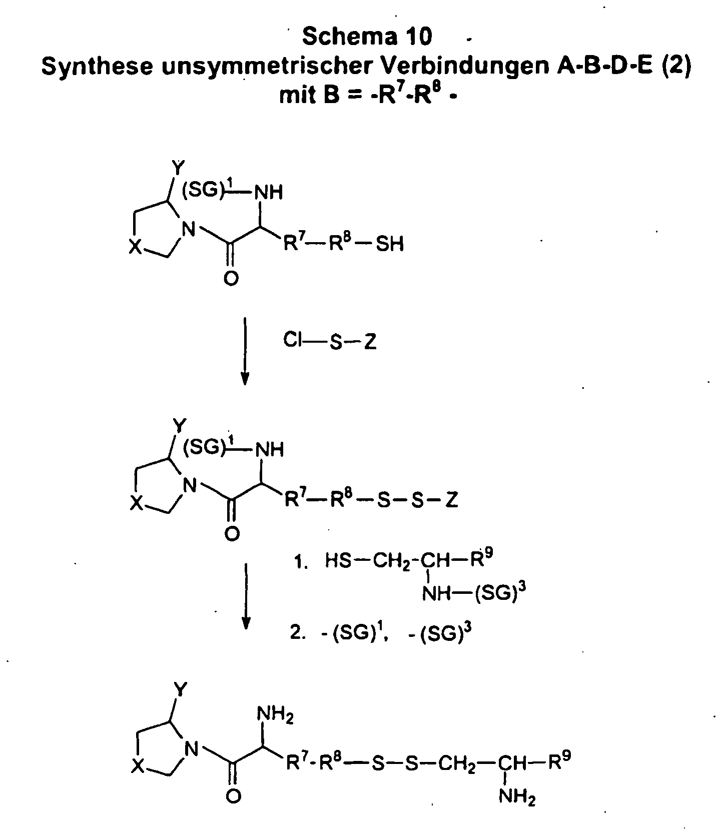

In allen angegebenen Reaktionsschemata sind die Reste F², F³, F⁴, F⁵ und F⁶ Teile von funktionellen Gruppen und sind insbesondere geeignete, aus dem Molekül abspaltbare Abgangsgruppen wie beispielsweise -H, -OH, -Cl usw.. Z steht für Reste, die ein eine -S-S-Gruppe enthaltendes Molekül für einen Thiol-Austausch aktivieren, also einen Austausch einer an das S-Atom gebundenen Gruppe gegen ein (gegebenenfalls mit einem Substituenten versehenes) S-Atom eines anderen Moleküls. Solche Gruppen sind dem Fachmann auf dem Gebiet der organischen Synthese hinlänglich bekannt, und die Erfindung ist nicht auf bestimmte Gruppen beschränkt, die für einen Thiol-Austausch aktivieren. Bevorzugte, nicht jedoch beschränkende Beispiele sind die Gruppen 3-Nitro-2-pyridyl, 5-Nitro-2-pyridyl, 2-, 3- oder 4-Pyridyl, 2-Nitrophenyl, Methoxycarbonyl oder N-Methyl-N-phenylcarbamoyl. In dem obigen Schema bedeuten die Reste R^{4a} und R^{4b} gemeinsam den Rest R⁴ und die Reste R^{8a} und R^{8b} gemeinsam den Rest R⁸, beide jeweils mit den oben angegebenen allgemeinen und speziellen Bedeutungen.

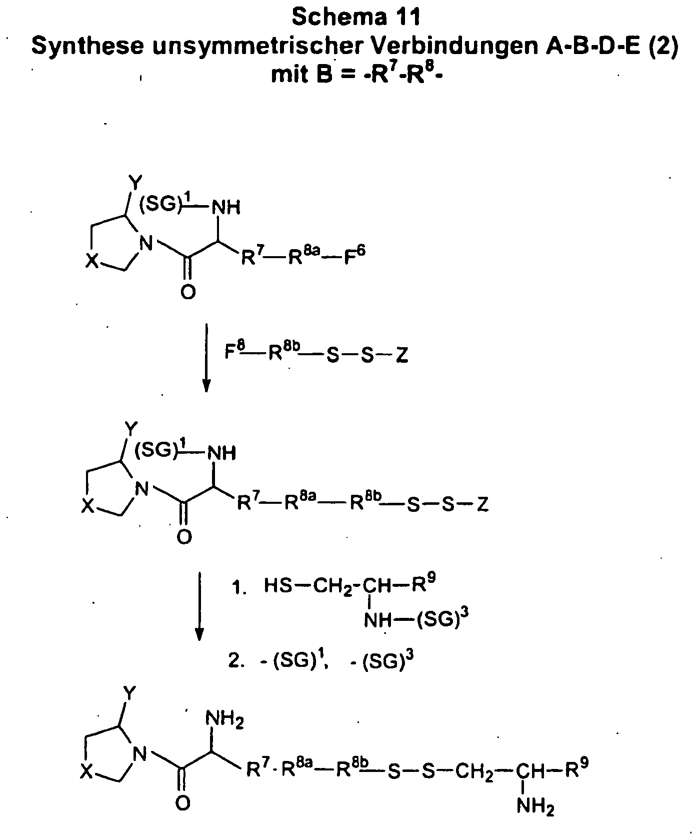

Die vorgenannten und in den Reaktionsschemata angegebenen Reaktionen zu den erfindungsgemäßen Verbindungen der allgemeinen Formeln (1) und (2) werden unter dem Fachmann auf dem Gebiet der organischen Synthese hinlänglich bekannten Bedingungen nach an sich bekannten Verfahren durchgeführt und bedürfen diesbezüglich keiner weiteren Erläuterung. Typische verwendete Lösungsmittel sind polare organische Lösungsmittel oder Mischungen solcher Lösungsmittel. Insbesondere kommen Ether wie THF, Ester wie Essigsäureethylester oder DMF infrage, ohne dass die Erfindung hierauf beschränkt ist. Die Reaktionstemperatur richtet sich nach der durchzuführenden Umsetzung und dem verwendeten Lösungsmittel, liegt jedoch üblicherweise im Bereich von - 20 °C bis 50 °C, vorzugsweise bei 10 °C bis 40 °C.

Die erfindungsgemäßen Verbindungen der allgemeinen Formeln (1) und (2) fallen bei ihrer Synthese nach einem der vorstehend beschriebenen Verfahren insbesondere dann, wenn es um ihre genaue Charakterisierung und anschlie-βende Verwendung in wässrigem Milieu geht, in Form von Säureadditionssalzen an. Bevorzugt werden für die Charakterisierung und spätere Verwendung der Verbindungen der allgemeinen Formeln (1) und (2) Säureadditionssalze mit bestimmten, physiologisch (d. h. pharmakologisch und/oder kosmetisch) annehmbaren anorganischen oder organischen Säuren hergestellt. In bevorzugten Ausführungsformen der Erfindung werden als Säureadditionssalze Salze pharmazeutisch annehmbarer Säuren aus der Gruppe hergestellt, die besteht aus Hydrochloriden, Trifluoracetaten, Tartraten, Succinaten, Formiaten und/ oder Citraten der Verbindungen der allgemeinen Formeln (1) oder (2).

Die oben im allgemeinen (anhand der Formeln (1) und (2)) und im einzelnen beschriebenen Verbindungen, die beispielsweise nach einem der vorstehend beschriebenen Verfahren hergestellt werden können, ohne dass die Herstellung der Verbindungen auf die oben genannten Verfahren beschränkt ist, sind für zahlreiche Zwecke verwendbar. Die Erfindung hat überraschend gefunden, dass die Verbindungen im Bereich der Medizin verwendet werden können. Insbesondere wurde gefunden, dass die neuen Verbindungen gemäß der vorliegenden Erfindung selbst Inhibitoren der Dipeptidylpeptidase IV oder von Enzymen mit analoger enzymatischer Wirkung und der Alanyl-Aminopeptidase N oder von Enzymen mit analoger enzymatischer Wirkung sind.

In bevorzugten Ausführungsformen der Erfindung können die Verbindungen erfolgreich als Inhibitor-Vorstufen oder als Inhibitor-Prodrugs verwendet werden. Zur Definition der Begriffe "Inhibitor", "Vorstufe" und "Prodrug" wird auf die obigen Definitionen verwiesen.

Weiter bevorzugte Verbindungen gemäß der Erfindung dienen der Verwendung als Prodrugs für Inhibitoren der Dipeptidylpeptidase IV (DPIV) und Peptidasen mit analoger enzymatischer Wirkung sowie der Alanyl-Aminopeptidasen N (APN) und Peptidasen mit analoger enzymatischer Wirkung. Von diesen Verbindungen sind diejenigen noch weiter bevorzugt, die einer Verwendung als Prodrugs für Inhibitoren der Dipeptidylpeptidase IV (DPIV) und Alanyl-Aminopeptidase N (APN) dienen. Überraschend wurde nämlich gefunden, dass die erfindungsgemäßen Verbindungen unter physiologischen oder pathologischen Bedingungen, insbesondere unter reduzierenden Bedingungen, wie sie an Zellen und Geweben vorliegen, zu solchen Verbindungen reagieren können, die hochwirksame Inhibitoren für die Dipeptidylpeptidase IV (DPIV) und Peptidasen mit analoger enzymatischer Wirkung sowie für die Alanyl-Aminopeptidase N (APN) und Peptidasen mit analoger enzymatischer Wirkung sind. Erfindungsgemäß kann eine der neuen Verbindungen gemäß der obigen Formeln (1) oder (2) entsprechend der allgemeinen oder speziellen Beschreibung Verwendung finden, oder es können mehrere der genannten Verbindungen in Kombination Verwendung finden, wobei Kombinationen von mehreren Verbindungen entweder mehrere Verbindungen der allgemeinen Formel (1) oder mehrere Verbindungen der allgemeinen Formel (2) oder mehrere Verbindungen umfassen können, die beliebig aus der Gruppe der Verbindungen der allgemeinen Formeln (1) und (2) in Kombination gewählt sind. Es ist bevorzugt, allein eine der Verbindungen der allgemeinen Formeln (1) oder (2) einzusetzen.

Die Erfindung betrifft auch die Verwendung einer oder mehrerer Verbindung(en), mindestens jedoch einer Verbindung, besonders bevorzugt genau einer Verbindung der allgemeinen Formeln (1) und/oder (2) nach der obigen allgemeinen und detaillierten Beschreibung zur Herstellung eines Medikaments zur Prophylaxe und Therapie von Erkrankungen mit überschießender Immunantwort und entzündlicher Genese, einschließlich Arteriosklerose, neuronalen Erkrankungen, zerebralen Schädigungen, Hauterkrankungen, Tumorerkrankungen und Virus-bedingten Erkrankungen sowie Diabetes Typ II.

Die Erfindung betrifft weiter die Verwendung einer oder mehrerer Verbindung(en), mindestens jedoch einer Verbindung, besonders bevorzugt genau einer Verbindung der allgemeinen Formeln (1) und/oder (2) nach der obigen allgemeinen und detaillierten Beschreibung zur Herstellung eines Medikaments oder einer kosmetischen Zubereitung zur Prophylaxe und Therapie von Erkrankungen mit überschießender Immunantwort und entzündlicher Genese, einschließlich Arteriosklerose, neuronalen Erkrankungen, zerebralen Schädigungen, Hauterkrankungen, Tumorerkrankungen und Virus-bedingten Erkrankungen sowie Diabetes Typ II.

In bevorzugten Ausführungsformen der Erfindung erfolgt eine Verwendung der Verbindungen der allgemeinen Formeln (1) und/oder (2) im allgemeinen und bevorzugt der Verbindungen gemäß den beiden vorstehenden Tabellen einzeln oder in Kombination, oder auch eine Verwendung von pharmazeutischen oder kosmetischen Zusammensetzungen, die eine oder mehrere der genannten Verbindungen umfassen, zur Prophylaxe und Therapie von Erkrankungen wie beispielsweise Multiple Sklerose, Morbus Crohn, Colitis ulcerosa, und anderen Autoimmunerkrankungen sowie entzündlichen Erkrankungen, Asthma bronchiale und anderen allergischen Erkrankungen, Haut- und Schleimhauterkrankungen, beispielsweise Psoriasis, Akne sowie dermatologischen Erkrankungen mit Hyperproliferation und veränderten Differenzierungszuständen von Fibroblasten, benigner fibrosierender und sklerosierender Hauterkrankungen und maligner fibroblastärer Hyperproliferationszustände, akuten neuronalen Erkrankungen, wie beispielsweise Ischämie-bedingter zerebraler Schädigungen nach einem ischämischen oder hämorrhagischen Schlaganfall, Schädel/Hirn-Trauma, Herzstillstand, Herzinfarkt oder als Folge von herzchirurgischen Eingriffen, von chronischen neuronalen Erkrankungen, beispielsweise von Morbus Alzheimer, der Pick'schen Erkrankung, der Progressiven Supranukleären Palsy, der kortikobasalen Degeneration, der frontotemporalen Demenz, von Morbus Parkinson, insbesondere Parkinsonismus gekoppelt an Chromosom 17, von Morbus Huntington, von durch Prionen bedingten Krankheitszuständen und von Amyotropher Lateralsklerose, von Artherosklerose, arterieller Entzündung, Stent-Restenose, von Chronisch Obstruktiven Lungenerkrankungen (COPD), von Tumoren, Metastasierungen, von Prostatakarzinom, von Schwerem Akutem Respiratorischen Syndrom (SARS) und von Sepsis und Sepsis-ähnlichen Zuständen sowie Diabetes Typ II.

In einer weiteren bevorzugten Ausführungsform der Erfindung erfolgt eine Verwendung der Verbindungen der allgemeinen Formeln (1) und/oder (2) im allgemeinen und bevorzugt die Verbindungen gemäß den obigen beiden Tabellen einzeln oder in Kombination, oder auch der pharmazeutischen oder kosmetischen Zusammensetzungen, die eine oder mehrere der genannten Verbindungen umfassen, zur Prophylaxe und Therapie der Abstoßung von transplantierten Geweben und Zellen. Als ein Beispiel einer solchen Anwendung kann die Verwendung einer oder mehrerer der vorgenannten Verbindungen oder einer pharmazeutischen Zusammensetzung, die eine oder mehrere der genannten Verbindungen enthält, bei allogenen oder xenogenen transplantierten Organen, Geweben und Zellen, wie Nieren-, Herz-, Leber-, Pankreas-, Haut- oder Stammzelltransplantation sowie Graft versus Host-Erkrankungen genannt werden.

In einer weiteren bevorzugten Ausführungsform der Erfindung erfolgt eine Verwendung der Verbindungen der allgemeinen Formeln (1) und/oder (2) im allgemeinen und bevorzugt der Verbindungen gemäß den beiden obigen Tabellen einzeln oder in Kombination, oder auch der pharmazeutischen oder kosmetischen Zusammensetzungen, die eine oder mehrere der genannten Verbindungen umfassen, zur Prophylaxe und Therapie der Abstoßungs- oder Entzündungsreaktionen an oder durch in einen Organismus implantierte medizinische Gegenstände ("medical devices"). Dies können beispielsweise Stents, Gelenkimplantate (Kniegelenk-Implantate, Hüftgelenk-Implantate), Knochen-Implantate, Herz-Schrittmacher oder andere Implantate sein. In einer weiteren bevorzugten Ausführungsform der Erfindung erfolgt eine Verwendung der Verbindungen der allgemeinen Formeln (1) und/oder (2) im allgemeinen und bevorzugt die Verbindungen gemäß den beiden obigen Tabellen einzeln oder in Kombination, oder auch der pharmazeutischen oder kosmetischen Zusammensetzungen, die eine oder mehrere der genannten Verbindungen umfassen, in der Weise, daß die Verbindung(en) oder Zusammensetzung(en) in Form einer Beschichtung oder Benetzung auf den Gegenstand bzw. die Gegenstände aufgebracht werden oder mindestens eine der Verbindungen oder Zusammensetzungen stofflich dem Material des Gegenstandes / der Gegenstände beigemischt wird. Auch in diesem Fall ist natürlich möglich, mindestens eine der Verbindungen oder Zusammensetzungen - gegebenenfalls zeitlich abgestuft oder parallel - lokal oder systemisch zu verabreichen.

In gleicher Weise wie vorstehend beschrieben - und für die vergleichbaren Zwecke bzw. zur Prophylaxe und Therapie der vorstehend beispielhaft, jedoch nicht abschließend genannten Erkrankungen und Zustände - können die Verbindungen der allgemeinen Formeln (1) und (2) im allgemeinen und die Verbindungen gemäß den beiden obigen Tabellen in bevorzugten Ausführungsformen, sowie die nachstehend beschriebenen, die genannten Verbindungen enthaltenden pharmazeutischen und kosmetischen Zusammensetzungen allein oder in Kombination mehrerer von ihnen zur Herstellung von Medikamenten zur Behandlung der o. g. Krankheiten oder Zustände verwendet werden. Diese können die genannten Verbindungen in den nachstehend genannten Mengen umfassen, gegebenenfalls zusammen mit an sich bekannten Träger-, Hilfs- und/oder Zusatzstoffen.

In bevorzugten Ausführungsformen umfasst die Verwendung der Verbindungen der obigen allgemeinen Formeln (1) und/oder (2) solche Fälle, in denen die verwendete(n) Verbindung(en) mindestens einen Inhibitor der Dipeptidylpeptidase IV (DPIV) und Peptidasen mit analoger enzymatischer Wirkung sowie der Alanyl-Aminopeptidase N (APN) und Peptidasen mit analoger enzymatischer Wirkung generiert/generieren. Eine Generierung mindestens eines derartigen Inhibitors, bevorzugt zweier oder mehrerer derartiger Inhibitoren, besonders bevorzugt eines Inhibitors oder zweier Inhibitoren, aus einer Verbindung führt zu einer überraschenden Spezifität des Einsatzes der erfindungsgemäßen Verbindungen im medizinischen Bereich, insbesondere als Inhibitoren eines oder mehrerer Enzyme aus den vorgenannten Gruppen.

In einer noch weiter bevorzugten Ausführungsform führt die Verwendung einer oder mehrerer der Verbindungen der obigen allgemeinen Formeln (1) und/oder (2) oder von kosmetischen oder pharmazeutischen Zubereitungen, die mindestens eine der Verbindungen der allgemeinen Formeln (1) und/oder (2) umfassen, zu mindestens einem Inhibitor, vorzugsweise zu einem Inhibitor oder zu zwei Inhibitoren von Enzymen aus der vorgenannten Gruppe, wenn reduzierende Bedingungen herrschen, insbesondere wenn reduzierende Bedingungen an der Stelle herrschen, an der die Verbindung(en), die entsteht/entstehen, zur pharmakologischen Wirkung kommen.

Unter "reduzierenden Bedingungen" werden in diesem Zusammenhang Bedingungen einer chemischen Reaktion verstanden, in denen die Verbindung oder die Verbindungen der allgemeinen Formel (1) oder die Verbindung oder die Verbindungen der allgemeinen Formel (2) oder eine oder mehrere Verbindungen der allgemeinen Formeln (1) und (2) in Kombination Reaktionsbedingungen am Ort der Wirkung ausgesetzt sind, in denen ein Elektronen-Donor Elektronen zur Aufnahme durch die Verbindung(en) der allegemeinen Formeln (1) und/oder (2) bereitstellt. Wie erfindungsgemäß gefunden wurde, werden die Verbindungen der allgemeinen Formeln (1) und/oder (2) unter reduzierenden Bedingungen unter Spaltung der -S-S- - oder -Se-Se- - Brücke, für die D in den allgemeinen Formeln (1) und (2) steht, in zwei Bausteine mit -SH - Endgruppe bzw. -SeH-Endgruppe überführt.

Auf welchem Wege die reduzierenden physiologischen Reaktionsbedingungen zustande kommen, ist für die Erfindung ohne entscheidende Bedeutung, solange sie zur zuverlässigen Generierung der jeweiligen Moleküle mit -SH- bzw. -Se-Endgruppe führen. Die erfindungsgemäßen Verbindungen treffen regelmä-βig auf genau die Bedingungen, die zur in-vivo-Generierung der als Inhibitoren der vorgenannten Enzyme geeigneten Verbindungen führen.

Die Mengen mindestens einer der Verbindungen der allgemeinen Formeln (1) und/oder (2) im allgemeinen bzw. der Verbindungen gemäß den beiden obigen Tabellen im Rahmen der erfindungsgemäßen Verwendung liegen im Bereich von 0,01 bis 1000 mg mindestens einer Verbindung der allgemeinen Formel (1) und/oder (2) pro Verabreichungseinheit, vorzugsweise im Bereich von 0,1 bis 100 mg pro Verabreichungseinheit.

Im Rahmen der erfindungsgemäßen Verwendung kann eine Verabreichung der mindestens einen Verbindung der allgemeinen Formeln (1) und/oder (2) auf jedem an sich bekannten Weg erfolgen, den ein Fachmann mit üblichen Kenntnissen in diesem technischen Bereich kennt. Die Verabreichung der Verbindungen der allgemeinen Formeln (1) und/oder (2) im allgemeinen und noch weiter bevorzugt der Verbindungen gemäß den beiden obigen Tabellen bzw. pharmazeutischer oder kosmetischer Zubereitungen, die eine oder mehrere der vorgenannten Verbindungen zusammen mit an sich üblichen Träger-, Hilfs- und/oder Zusatzstoffen umfassen, erfolgt einerseits als topische Applikation in Form von z.B. Cremes, Salben, Pasten, Gelen, Lösungen, Sprays, Liposomen und Nanosomen, Schüttelmixturen, "pegylierten" Formulierungen, degradierbaren (d. h. unter physiologischen Bedingungen abbaubaren) Depot-Matrices, Hydrokolloidverbänden, Pflastern, Mikroschwämmen, Prepolymeren und ähnlichen neuen Trägersubstraten, Jet-Injektion bzw. anderen dermatologischen Grundlagen/Vehikeln einschließlich instillativer Applikation, und andererseits als systemische Applikation zur oralen, transdermalen, intravenösen, subcutanen, intracutanen, intramuskulären, intrathekalen Anwendung in geeigneten Rezepturen bzw. in geeigneter Galenik, also beispielsweise Tabletten, Dragees, Lutschtabletten, Kapseln, Aerosole, Sprays, Lösungen, Emulsionen und Suspensionen.

Die Erfindung betrifft auch die Verwendung einer Verbindung der allgemeinen Formeln (1) oder (2) in einem Verfahren zur Hemmung sowohl der Aktivität der Alanyl-Aminopeptidase N oder von Peptidasen mit analoger enzymatischer Wirkung als auch der Aktivität der Dipeptidylpeptidase IV oder von Peptidasen mit analoger enzymatischer Wirkung, und zwar allein oder in Kombination mit anderen Inhibitoren der Alanyl-Aminopeptidase N oder Inhibitoren von Peptidasen mit analoger enzymatischer Wirkung und/oder anderen Inhibitoren der DPIV oder Inhibitoren von Peptidasen mit analoger enzymatischer Wirkung durch Verabreichung mindestens einer Verbindung der allgemeinen Formel (1) und/oder (2) oder einer pharmazeutischen oder kosmetischen Zusammensetzung, die mindestens eine Verbindung der allgemeinen Formel (1) und/oder (2) umfasst, gemäß der obigen detaillierten Beschreibung in einer für die Hemmung der Enzymaktivität erforderlichen Menge. Die Mengen einer der Verbindungen der allgemeinen Formeln (1) und/oder (2) im allgemeinen bzw. der Verbindungen gemäß den beiden obigen Tabellen liegen - wie oben angegeben - im Bereich von 0,01 bis 1000 mg einer mindestens Verbindung pro Verabreichungseinheit, vorzugsweise im Bereich von 0,1 bis 100 mg pro Verabreichungseinheit.

Weiter betrifft die Erfindung auch die Verwendung einer Verbindung der allgemeinen Formeln (1) oder (2) in einem Verfahren zur topischen Beeinflussung sowohl der Aktivität der Alanyl-Aminopeptidase N oder von Peptidasen mit analoger enzymatischer Wirkung als auch der Aktivität der Dipeptidylpeptidase IV oder von Peptidasen mit analoger enzymatischer Wirkung, und zwar allein oder in Kombination mit anderen Inhibitoren der Alanyl-Aminopeptidase N oder Inhibitoren von Peptidasen mit analoger enzymatischer Wirkung und/oder anderen Inhibitoren der DPIV oder Inhibitoren von Peptidasen mit analoger enzymatischer Wirkung durch Verabreichung mindestens einer Verbindung der allgemeinen Formeln (1) und/oder (2) oder von einer pharmazeutischen oder kosmetischen Zusammensetzung gemäß der nachstehenden detaillierten Beschreibung in einer für die Beeinflussung der Enzymaktivität erforderlichen Menge. Auch in diesen Fällen bewegen sich die Mengen der Verbindung(en) der allgemeinen Formeln (1) und/oder (2) im oben angegebenen Bereich.

Die Erfindung betrifft auch die Verwendung einer Verbindung der allgemeinen Formeln (1) oder (2) in einem Verfahren zur Generierung von mindestens einem Inhibitor der Dipeptidylpeptidase IV (DPIV) und Peptidasen mit analoger enzymatischer Wirkung sowie der Alanyl-Aminopeptidase N (APN) und Peptidasen mit analoger enzymatischer Wirkung aus mindestes einer Verbindung der allgemeinen Formeln (1) und (2). Das erfindungsgemäße Verfahren umfasst den Schritt, dass man mindestens eine Verbindung der allgemeinen Formeln (1) und (2) gemäß der obigen Beschreibung reduzierenden Bedingungen aussetzt. Wie oben bereits ausgeführt, sind dem Fachmann hinsichtlich der reduzierenden Bedingungen bei der Umsetzung mindestens einer der Verbindungen der allgemeinen Formeln (1) und/oder (2), die insoweit auch als Zwischenstufen in der Synthese erfindungsgemäßer Inhibitoren der Dipeptidylpeptidase IV (DPIV) und Inhibitoren von Peptidasen mit analoger enzymatischer Wirkung sowie Inhibitoren der Alanyl-Aminopeptidase N (APN) und Inhibitoren von Peptidasen mit analoger enzymatischer Wirkung angesehen werden können, zu den eigentlichen Inhibitoren der Dipeptidylpeptidase IV (DPIV) und Inhibitoren von Peptidasen mit analoger enzymatischer Wirkung sowie Inhibitoren der Alanyl-Aminopeptidase N (APN) und Inhibitoren von Peptidasen mit analoger enzymatischer Wirkung keine limitierenden Bedingungen gesetzt.

Weiter betrifft die Erfindung auch die Verwendung einer Verbindung der allgemeinen Formeln (1) oder (2) in einem Verfahren zur Prophylaxe und Therapie einer Vielzahl von Erkrankungen, beispielsweise Erkrankungen mit überschie-βender Immunantwort (Autoimmunerkrankungen, Allergien und Transplantatrejektionen), von anderen chronisch-entzündlichen Erkrankungen, neuronalen Erkrankungen und zerebralen Schädigungen, Hauterkrankungen (u. a. Akne und Schuppenflechte), Tumorerkrankungen und speziellen Virusinfektionen (u. a. SARS) sowie Diabetes Typ II und insbesondere der oben im einzelnen genannten Erkrankungen. Dies schließt ein: Verfahren zur Prophylaxe und Therapie von Erkrankungen wie beispielsweise Multiple Sklerose, Morbus Crohn, Colitis ulcerosa, und anderen Autoimmunerkrankungen sowie entzündlichen Erkrankungen, Asthma bronchiale und anderen allergischen Erkrankungen, Haut- und Schleimhauterkrankungen, beispielsweise Psoriasis, Akne sowie dermatologischen Erkrankungen mit Hyperproliferation und veränderten Differenzierungszuständen von Fibroblasten, benigner fibrosierender und sklerosierender Hauterkrankungen und maligner fibroblastärer Hyperproliferationszustände, akuten neuronalen Erkrankungen, wie beispielsweise Ischämie-bedingter zerebraler Schädigungen nach einem ischämischen oder hämorrhagischen Schlaganfall, SchädeI/Hirn-Trauma, Herzstillstand, Herzinfarkt oder als Folge von herzchirurgischen Eingriffen, von chronischen neuronalen Erkrankungen, beispielsweise von Morbus Alzheimer, der Pick'schen Erkrankung, der Progressiven Supranukleären Palsy, der kortikobasalen Degeneration, der frontotemporalen Demenz, von Morbus Parkinson, insbesondere Parkinsonismus gekoppelt an Chromosom 17, von Morbus Huntington, von durch Prionen bedingten Krankheitszuständen und von Amyotropher Lateralsklerose, von Artherosklerose, arterieller Entzündung, Stent-Restenose, von Chronisch Obstruktiven Lungenerkrankungen (COPD), von Tumoren, Metastasierungen, von Prostatakarzinom, von Schwerem Akutem Respiratorischen Syndrom (SARS) und von Sepsis und Sepsis-ähnlichen Zuständen. Das Verfahren umfasst eine Verabreichung mindestens einer Verbindung oder pharmazeutischen Zusammensetzung gemäß der nachstehenden detaillierten Beschreibung in einer für die Prophylaxe oder Therapie der jeweiligen Erkrankung erforderlichen Menge. Auch in diesen Fällen bewegen sich die Mengen der Verbindung(en) im oben angegebenen Bereich von 0,01 bis 1000 mg einer Verbindung pro Verabreichungseinheit, vorzugsweise im Bereich von 0,1 bis 100 mg pro Verabreichungseinheit.

Die Erfindung betrifft auch pharmazeutische Zubereitungen, die wenigstens eine Verbindung wenigstens einer der allgemeinen Formeln (1) und (2) nach der obigen allgemeinen und detaillierten Beschreibung, gegebenenfalls zusammen mit einem oder mehreren pharmazeutisch annehmbaren Träger(n), Hilfsstoff(en) und/oder Adjuvant(ien), umfassen.

Weiter betrifft die Erfindung auch kosmetische Zubereitungen, die wenigstens eine Verbindung wenigstens einer der allgemeinen Formeln (1) und (2) nach der obigen allgemeinen und detaillierten Beschreibung, gegebenenfalls zusammen mit einem oder mehreren kosmetisch annehmbaren Träger(n), Hilfsstoff(en) und/oder Adjuvant(ien), umfassen.

Die in derartigen Zubereitungen möglichen pharmazeutisch oder kosmetisch annehmbaren Träger, Hilfsstoffe und/oder Adjuvantien sind dem Fachmann auf dem pharmazeutischen oder kosmetischen Fachgebiet zur Genüge bekannt und bedürfen hier keiner ins Einzelne gehenden Erwähnung.

Die genannten pharmazeutischen bzw. kosmetischen Zubereitungen können die mindestens eine Verbindung der allgemeinen Formel (1) oder (2), vorzugsweise eine oder zwei Verbindungen der allgemeinen Formeln (1) und/oder (2) in solcher Menge / in solchen Mengen enthalten, die für die gewünschte Wirkung im pharmazeutischen oder kosmetischen Bereich erforderlich ist/sind. Die Menge(n) unterliegt/unterliegen keinen besonderen Beschränkungen und ist/sind abhängig von einer Anzahl von Parametern wie beispielsweise vom Verabreichungsweg, von dem speziellen Krankheitsbild bzw. kosmetischen Status, von der Konstitution des Empfängers, der ein Säuger wie beispielsweise ein Mensch sein kann, von der Bioverfügbarkeit der verwendeten Verbindung(en) usw.. In besonders bevorzugten Ausführungsformen enthält eine pharmazeutische Verabreichungseinheit bzw. eine kosmetische Anwendungseinheit eine Menge an mindestens einer Verbindung der allgemeinen Formel (1) und/oder (2), die im Bereich von 0,01 bis 1000 mg einer Verbindung pro Verabreichungseinheit liegt, vorzugsweise im Bereich von 0,1 bis 100 mg pro Verabreichungseinheit liegt. Üblicherweise können die Verabreichungseinheiten so beschaffen sein (und auch solche Konzentrationen an mindestens einer Verbindung der allgemeinen Formel (1) und/oder (2) umfassen), dass die Verabreichung einer oder weniger, weiter bevorzugt einer, zweier oder dreier, Verabreichungseinheiten pro Tag ausreichend ist, um eine für eine zielgerichtete phamazeutische oder kosmetische Behandlung erforderliche Menge an mindestens einer der Verbindungen (1) und/oder (2) an den Empfänger, wie z. B. einen Säuger, speziell einen Menschen, zu verabreichen.

Die Erfindung wird nachfolgend durch spezielle bevorzugte Ausführungsbeispiele näher erläutert. Die nachfolgenden Ausführungsbeispiele dienen jedoch nicht der Beschränkung der Erfindung, sondern ausschließlich deren beispielhafter Erläuterung.

### Beispiele

### Beispiel 1

### Herstellung von Verbindungen der allgemeinen Formel (1)

Es wurden Verbindungen der allgemeinen Formel (1) nach folgendem Verfahren hergestellt:

### (a) Verbindung II (Schema 12)

440 mg (1 mmol) (Boc-Cys-OH)₂ 1 wurden in 5 ml trockenem THF gelöst. Zu dieser Lösung fügte man unter Argon 158 µl (2 mmol) Thiazolidin und nach 25 min bei 0°C portionsweise 460 mg (2,4 mmol) N'-(3-Dimethylaminopropyl)-N-ethylcarbodiimid (EDC) hinzu. Die Umsetzung wurde bei RT mittels DC kontrolliert. Nach 6h wurde mit 460 mg (2,4 mmol) EDC und 79 µl (1 mmol) Thiazolidin bei 0°C nachaktiviert. Nach 20 h destillierte man das THF ab und nahm den festen Rückstand in Essigester auf. Die Essigesterphase wusch man 3x mit 5%-iger KHSO₄-Lösung, 1x mit NaCl-Lösung, 3x mit gesättigter NaHCO₃-Lösung und 3x mit NaCl-Lösung. Es wurde über Na₂SO₄ getrocknet, abfiltriert und eingeengt.

Das gewonnene Rohprodukt wurde durch Kristallisation aus einem Gemisch aus Essigester/ Petrolether gereinigt.

Man erhielt 468 mg (80%) der entsprechenden Boc-geschützten Verbindung. Zur Abspaltung der Schutzgruppen wurden 468 mg (0,8 mmol) dieser Verbindung in 1,2 ml (16 mmol) Trifluoressigsäure und 12 ml Methylenchlorid gelöst. Man kontrollierte die Reaktion mittels DC und versetzte nach beendeter Umsetzung den Ansatz mit trockenem Ether. Der ausfallende Feststoff wurde abfiltriert und mehrfach mit Ether nachgewaschen.
Ausbeute: 446 mg (91%) II.

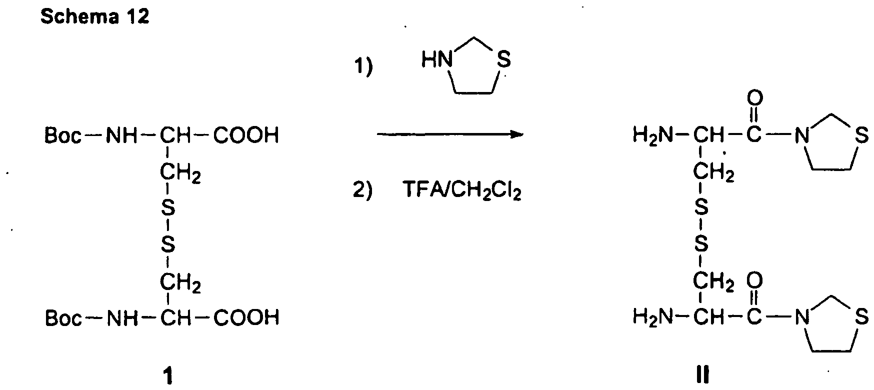

### (b) Verbindung VI (Schema 13)

### (i) Boc-Lys-thiazolidid 3

1,4g (3 mmol) Boc-Lys(Fmoc)-OH **2** und 382 µl (3 mmol) 4-Ethylmorpholin wurden in 7 ml trockenem THF gelöst. Man kühlte auf -15°C und versetzte bei dieser Temperatur mit 390 µl (3 mmol) Chlorameisensäureisobutylester. Nach 15 minütiger Reaktionszeit bei -15°C gab man 284 µl (3,6 mmol) Thiazolidin zu, rührte eine weitere Stunde bei -15°C und danach über Nacht bei RT.

Zur Vervollständigung der Umsetzung wurde mit der Hälfte der oben angegebenen Mengen nochmals nachaktiviert, wobei der Ansatz nach der Zugabe des 4-Ethylmorpholins wieder auf -15°C gekühlt wurde und Chlorameisensäureisobutylester und Thiazolidin, wie beschrieben, zugegeben wurden. Nach weiteren 4h Reaktionszeit bei RT wurde zur Trockne eingeengt, und der Rückstand wurde in Essigester aufgenommen..

Die Essigesterphase wurde 3x mit 5%-iger KHSO₄-Lösung, 1x mit NaCl-Lösung, 3x mit gesättigter NaHCO₃-Lösung und anschließend mit NaCl-Lösung neutral gewaschen.

Nach dem Trocknen über Na₂SO₄. wurde die Essigesterphase eingeengt und der Rückstand zur Abspaltung der Fmoc-Schutzgruppe in 30 ml Morpholin gelöst.

Nach 1,5 h Reaktionszeit bei RT wurde das Morpholin abdestilliert. Zum Rückstand gab man ca. 6 ml eiskaltes Methanol, filtrierte vom schwer löslichen 4-Fluoren-9-ylmethylmorpholin ab und engte die Lösung ein.
Ausbeute: 875 mg (92 %) **3**

### (ii) Synthese von VI

Zur Lösung von 181 mg (0,5 mmol) 4 in 3 ml DMF wurden bei 0°C portionsweise 240 mg (1,25 mmol) EDC gegeben, und nachfolgend wurden 317 mg (1 mmol) 3 zugesetzt.

Man rührte 1h bei 0°C und danach 6h bei RT. Nach Zugabe von weiteren 120 mg (0,63 mol) EDC und 6 stündiger Reaktionszeit wurde das DMF abdestilliert, und der Rückstand wurde in Essigester aufgenommen. Die Essigesterphase wurde 3x mit 5%-iger KHSO₄-Lösung, 1x mit NaCl-Lösung, 3x mit gesättigter NaHCO₃-Lösung und anschließend mit NaCl-Lösung neutral gewaschen.

Nach dem Trocknen über Na₂SO₄ und Abdestillieren des Essigesters verblieben 440 mg Rohprodukt.

Durch chromatographische Reinigung an Kieselgel mit dem Laufmittel Chloroform/Methanol 92/8 erhielt man 250 mg (55 %) **8**.

Zur Abspaltung der Schutzgruppen wurden 20 mg (0,022 mmol) **8** in 220 µl 1 M HCl in Eisessig gelöst. Nach 6-stündigem Stehen bei RT wurde zum Ansatz trockener Ether zugegeben, und das ausfallende Produkt wurde abfitriert und mit Ether gewaschen.
Ausbeute: 15 mg (88%) **VI**

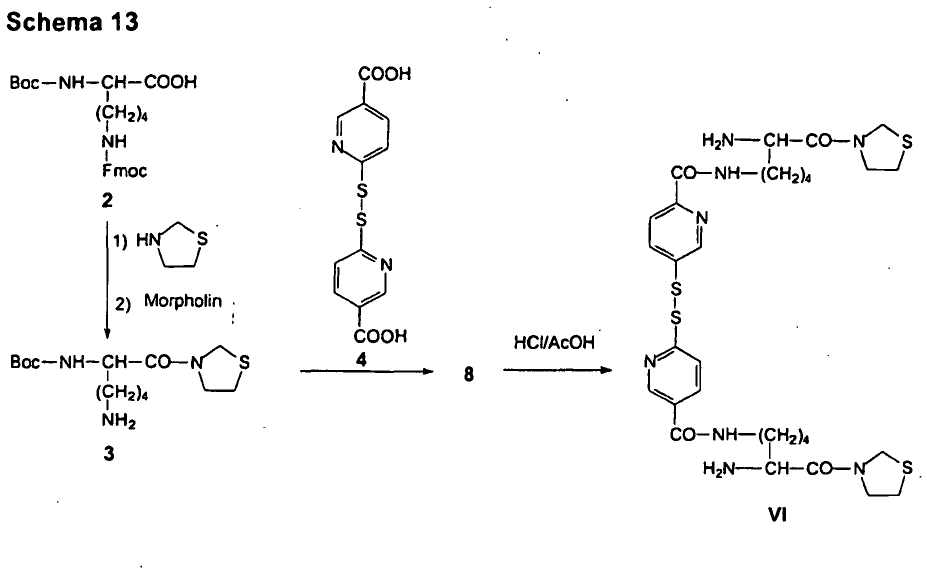

Die erhaltenen Verbindungen wurden durch ESI-MS charakterisiert. Die nachfolgende Tabelle 3 zeigt 6 Beispiele von Verbindungen der allgemeinen Formel (1) gemäß der Erfindung.

**Tabelle 3**

| Beispiele für Verbindungen der allgemeinen Formel **A - B - D - B' - A' (1)** | | | | | | |
|---|---|---|---|---|---|---|
| 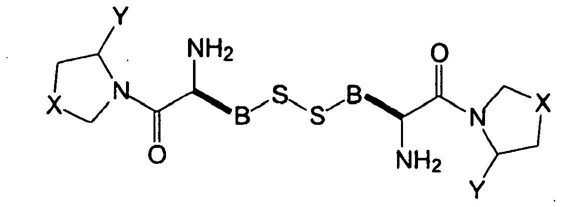 | | | | | | |
| **Nr.** | **B** | **X** | **Y** | **Summenformel** | **Mol.-gewicht** | **ESI-MS [m/e]** |
| I | - CH₂- | -CH₂- | H | C₁₄H₂₆N₄O₂S₂ | 346,5 | 347,2 |
| II | - CH₂- | S | H | C₁₂H₂₂N₄O₂S₄ | 382,6 | 383,0 |
| III | - CH₂- | -CH₂- | CN | C₁₆H₂₄N₆O₂S₂ | 396,5 | 397,1 |
| IV | 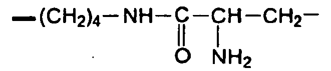 | S | H | C₂₄H₄₆N₈O₄S₄ | 638,9 | 639,2 |
| V | 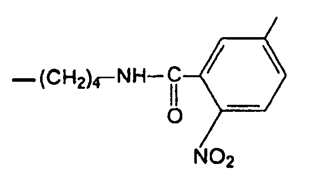 | S | H | C₃₂H₄₂N₈O₈S₄ | 795,0 | 795,2 |
| VI | 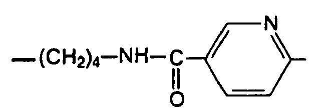 | S | H | C₃₀H₄₂N₈O₄S₄ | 707,0 | 707,2 |

### Beispiel 2

### Herstellung von Verbindungen der allgemeinen Formel (2)

Es wurden Verbindungen der allgemeinen Formel (2) nach folgenden Verfahren hergestellt:

### (a) Verbindung VII (Schema 14)

### (i) Synthese von 6

188 mg (0,5 mmol) Boc-Cys(Npys)-OH **5** wurden in 2,5 ml trockenem THF gelöst. Unter Argon wurden bei 0°C 40 µl (0,5 mmol) Thiazolidin und portionsweise 115 mg (0.6 mmol) EDC hinzugefügt. Die Reaktion wurde bei RT mittels DC verfolgt und nach ca. 4 h beendet. Hierfür wurde der Ansatz eingeengt, der feste Rückstand in Essigester aufgenommen und die organische Phase anschließend 3x mit 5%-iger KHSO₄-Lösung, 1x mit NaCl-Lösung, 3x mit gesättigter NaHCO₃-Lösung und 3x mit NaCl-Lösung gewaschen. Nach dem Trocknen über Na₂SO₄ wurde abfiltriert, und die Essigesterphase wurde eingeengt.
Ausbeute: 191 mg (86%) **6.**

### (ii) Synthese von VII

29 mg (0,065 mmol) **6** wurden in 5 ml ACN/ Phosphatpuffer (1:1), pH 8,0 gelöst. Zu dieser Lösung fügte man unter Argon bei RT eine Lösung aus 18 mg (0,065 mmol) β-Aminothiol **7** (synthetisiert in drei Stufen ausgehend von (S)-Phenylalaninol nach "Fournier-Zaluski, M. C.; Coric, P.; Turcaud, S.; Bruetschy, L.; Lucas, E.; Noble, F.; Roques, B. P. J. Med.Chem. 1992, 35, 1259-1266"; "Fournier-Zaluski, M. C.; Coric, P.; Turcaud, S.; Lucas, E.; Noble, F.; Maldonado, R.; Roques, B. P. J. Med.Chem. 1992, 35, 2473-2481") in 5 ml ACN/ Phosphatpuffer hinzu. Nach ca. 3 h Reaktionszeit (HPLC-Kontrolle) beendete man die Reaktion, indem man den Ansatz einengte, den festen Rückstand in Essigester aufnahm und diese Phase 3x mit NaCl-Lösung wusch. Es wurde über Na₂SO₄ getrocknet, abfiltriert und eingeengt. Das gewonnene Rohprodukt wurde an Kieselgel mit Essigester/ Petrolether chromatographisch gereinigt. Man erhielt 20 mg (55%) an entsprechender Boc-geschützter Verbindung. Zur Abspaltung der Schutzgruppen wurden 10 mg (0,018 mmol) dieser Verbindung in 28 µl (0,36 mmol) Trifluoressigsäure und 280 µl Methylenchlorid gelöst. Die Reaktion wurde mittels DC verfolgt und beendet, indem der Ansatz mit trockenem Ether versetzt wurde. Das ausfallende Produkt wurde mehrfach mit Ether nachgewaschen. Ausbeute: 8 mg (76%) VII.

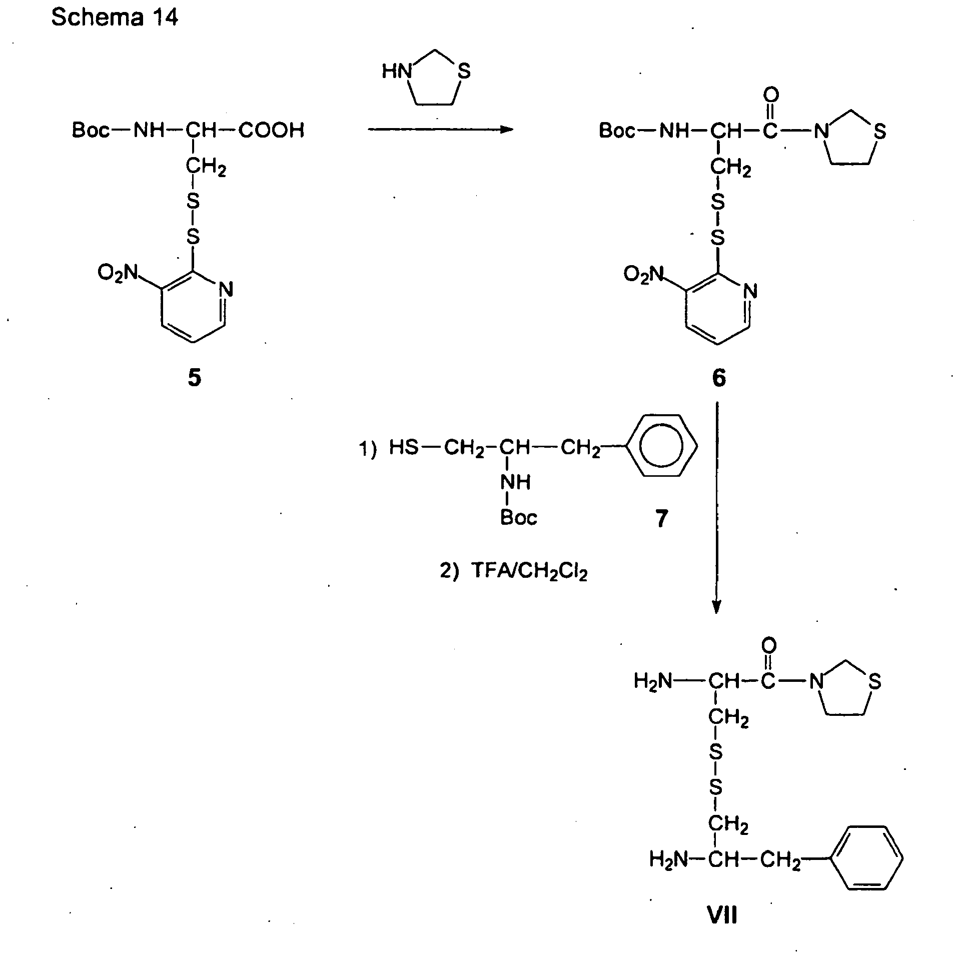

### (b) Verbindung X (Schema 15)

73 mg (0,08 mmol) 8 und 20 mg (0,075 mmol) β-Aminothiol 7 wurden in 3 ml Methanol 24 Stunden bei RT gerührt. Nach dem Abdestillieren des Methanols wurde das Rohprodukt an Kieselgel mit dem Laufmittel Essigester/Petrolether 95/5 gereinigt. Man erhielt 34 mg Boc-geschütztes Disulfid (63%), das zur Abspaltung der Schutzgruppen in 750 µl Methylenchlorid gelöst und mit 75 µl Trifluoressigsäure versetzt wurde. Nach 6 stündigem Stehen bei RT wurde trockener Ether zugegeben, und das ausfallende Produkt wurde abfitriert und mit Ether gewaschen. Ausbeute: 29 mg (83%) X.

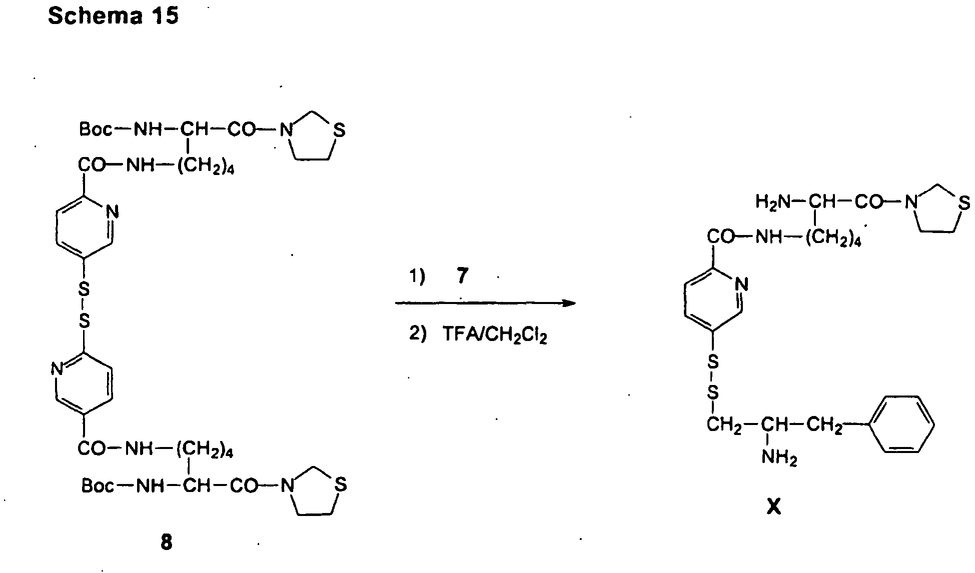

### (c) Verbindung XI (Schema 16)

### (i) Synthese von 10

190 mg (0,5 mmol) 3-(Fmoc-aminomethyl)-benzoesäure 9 und 64 µl (0,5 mmol) 4-Ethylmorpholin wurden in 1 ml DMF gelöst und unter Argon auf -15°C gekühlt. Zu dieser Lösung gab man 65 µl (0,5 mmol) Chlorameisensäureisobutylester , rührte 20 min bei -15°C, fügte anschließend eine Lösung aus 159 mg (0,5 mmol) Boc-Lys-thiazolidid 3 in 1,25 ml DMF hinzu und rührte noch 1 h bei -15°C.

Nach 20 h bei RT wurde erneut mit 32 µl (0,25 mmol) 4-Ethyl-morpholin und 33 µl (0,25 mmol) Chlorameisensäureisobutylester bei -15°C nachaktiviert.

Man destillierte das DMF nach 40 h (DC-Kontrolle) ab und nahm in Essigester auf. Die Essigesterphase wusch man 3x mit 5%-iger KHSO₄-Lösung, 1 x mit NaCl-Lösung, 3x mit gesättigter NaHCO₃-Lösung und 3x mit NaCl-Lösung. Es wurde über Na₂SO₄ getrocknet, abfiltiert und anschließend eingeengt. Das gewonnene Rohprodukt wurde an Kieselgel mit Essigester chromatographisch gereinigt.

Man erhielt 174 mg (52%) der entsprechenden Fmoc-geschützten Verbindung. Zur Abspaltung der Schutzgruppe wurden 150 mg (0,22 mmol) dieser Verbindung in 2,2 ml Morpholin gelöst, und nach 2 h wurde das überschüssige Morpholin abdestilliert. Den Rückstand nahm man in 1 ml eiskaltem Methanol auf, filtrierte vom unlöslichen 4-Fluoren-9-ylmethylmorpholin ab und engte ein.
Ausbeute: 92 mg (93%) 10.

### (ii) Synthese von 11

78 mg (0,21 mmol) Boc-Cys(Npys)-OH 5 und 27 µl (0,21 mmol) 4-Ethylmorpholin wurden in 0,4 ml THF gelöst und unter Argon auf -15°C gekühlt. Zu dieser Lösung gab man 28 µl (0,21 mmol) Chlorameisensäureisobutylester, rührte 20 min bei -15°C und fügte danach eine Lösung aus 95 mg (0.21 mmol) 10 in 0,75 ml THF hinzu. Nach 1 h bei -15°C und 20 h bei RT wurde mit 13 µl 4-Ethylmorpholin (0,10 mmol) und 13 µl (0,10 mmol) Chlorameisensäureisobutylester bei -15°C nachaktiviert.

Man destillierte das THF nach 24 h (DC-Kontrolle) ab und arbeitete den Ansatz wie bei Verbindung 10 beschrieben auf.

Das gewonnene Rohprodukt wurde anschließend an Kieselgel mit Methanol/Chloroform chromatographisch gereinigt. Ausbeute: 100 mg (60%) 11.

### (iii) Synthese von XI

67 mg (0,08 mmol) 11 wurden in 8 ml Acetonitril/ Phosphatpuffer (1:1), pH 8,0 gelöst. Unter Argon gab man bei RT eine Lösung aus 21 mg (0,08 mmol) 7 in 8 ml Acetonitril/Phosphatpuffer hinzu. Man verfolgte die Reaktion mittels HPLC und engte nach beendeter Reaktion ein. Den festen Rückstand nahm man in Essigester auf, wusch 3x mit NaCl-Lösüng, trocknete über Na₂SO₄, filtrierte ab und engte ein. Das Rohprodukt wurde anschließend an Kieselgel mit Essigester/Petrolether chromatographisch gereinigt. Man erhielt 29 mg (40%) der entsprechenden Boc-geschützten Verbindung. Zur Abspaltung der Schutzgruppen wurden 22 mg (0,024 mmol) dieser Verbindung in 55 µl Trifluoressigsäure und 550 µl Methylenchlorid gelöst. Die Reaktion wurde mittels DC verfolgt, und der Ansatz wurde nach Beendigung der Abspaltung mit Ether versetzt. Das ausfallende Produkt wurde abfiltriert und mehrfach mit Ether nachgewaschen. Ausbeute: 16 mg (70%) XI.

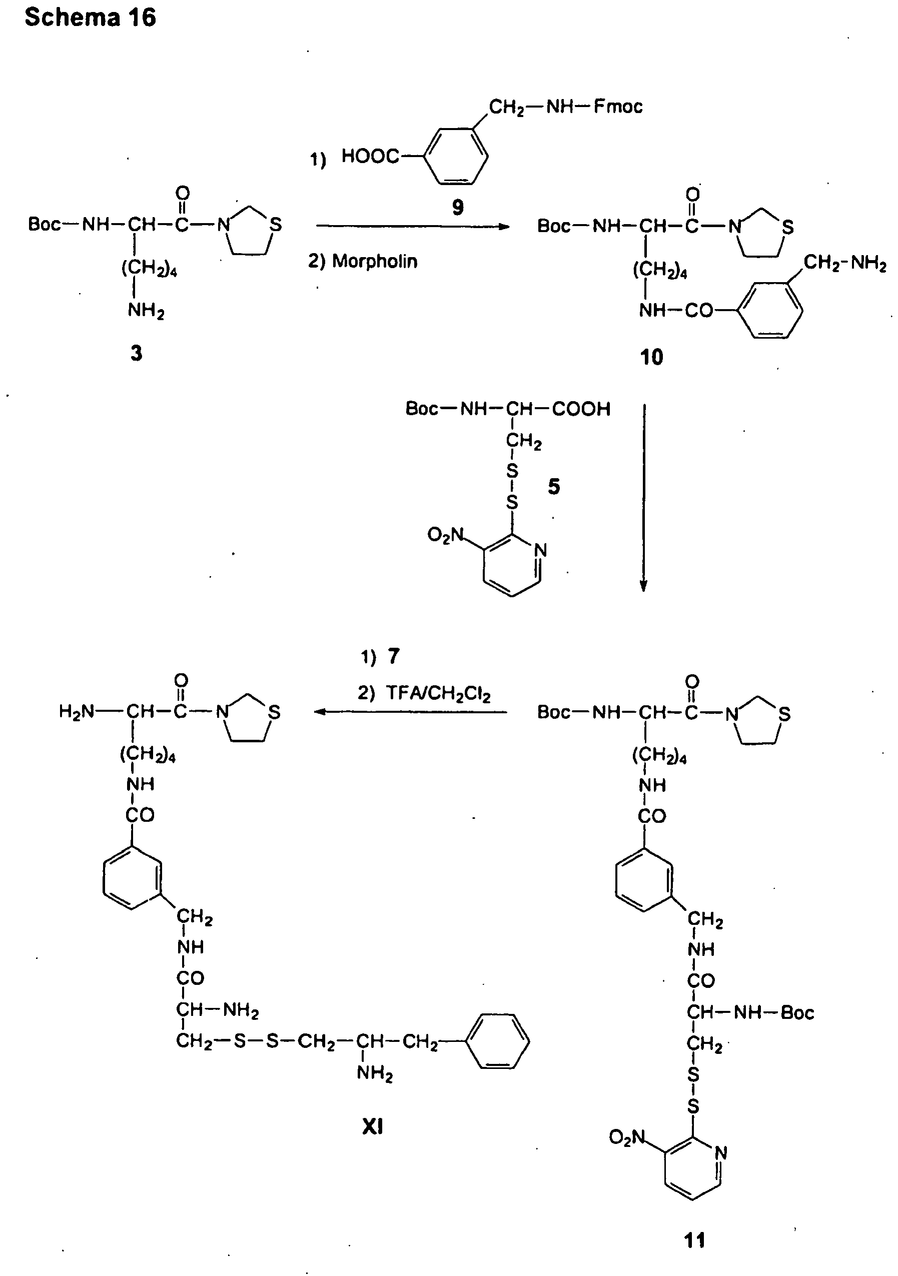

### (d) Verbindung XIII (Schema 17)

### (i) Synthese von 13

2,5 g (5 mmol) Boc-p-Amino-Phe(Fmoc)-OH **12** wurden in 25 ml trockenem THF gelöst. Unter Argon gab man bei 0°C 394 µl (5 mmol) Thiazolidin und portionsweise 1,15 g (6 mmol) EDC hinzu. Die Reaktion wurde mittels DC bei RT verfolgt. Nach 6 h wurde mit 197 µl (2,5 mmol) Thiazolidin und 954 mg (5 mmol) EDC bei 0°C nachaktiviert. Man beendete die Reaktion nach weiteren 2 h bei RT, indem man den Ansatz einengte, den festen Rückstand in Essigester aufnahm und die organische Phase 3x mit 5%-iger KHSO₄-Lösung, 1 x mit NaCl-Lösung, 3x mit gesättigter NaHCO₃-Lösung und 3x mit NaCl-Lösung wusch. Anschließend trocknete man über Na₂SO₄, filtrierte ab und engte ein.

Man erhielt 2,58 g (90%) der entsprechenden Fmoc-geschützten Verbindung, welche zur Abspaltung der Schutzgruppe in 45 ml Morpholin gelöst wurde. Nach 2h bei RT wurde das überschüssige Morpholin abdestilliert, und der Rückstand wurde in 6 ml eiskaltem Methanol aufgenommen. Man filtrierte vom unlöslichen 4-Fluoren-9-ylmethyl-morpholin ab und engte ein.
Ausbeute: 1.23 g (78%) **13**

### (ii) Synthese von 14

375 mg (1 mmol) **5** und 127 µl (1 mmol) 4-Ethylmorpholin wurden in 3 ml trockenem THF gelöst und unter Argon auf -15°C gekühlt. Zu dieser Lösung fügte man 131 µl (1 mmol) Chlorameisensäure-isobutylester und nach 20 min bei -15°C eine Lösung aus 351 mg (1 mmol) **13** in 0,5 ml THF hinzu. Nach 1 h bei -15°C und 20 h bei RT (DC-Kontrolle) wurde mit 64 µl (0,5 mmol) 4-Ethylmorpholin und 66 µl (0,5 mmol) Chlorameisensäureisobutylester bei -15°C nachaktiviert. Man beendete nach weiteren 2 h die Reaktion, indem man den

Ansatz einengte, den festen Rückstand in Essigester aufnahm und die Essigester-Phase wie bei Verbindung **13** beschrieben aufarbeitete.

Das gewonnene Rohprodukt wurde an Kieselgel mit Essigester chromatographisch gereinigt. Ausbeute: 350 mg (50%) **14.**

### (iii) Synthese von XIII

65 mg (0,09 mmol) **14** wurden in 9 ml Acetonitril/ Phosphatpuffer (1:1), pH 8,0 gelöst. Unter Argon fügte man bei RT eine Lösung von 25 mg (0,09 mmol) 7 in 9 ml Acetonitril/ Phosphatpuffer hinzu. Die Reaktion wurde mittels HPLC verfolgt, und der Ansatz wurde nach ca. 3 h aufgearbeitet. Nach dem Einengen wurde der feste Rückstand in Essigester aufgenommen, und die organische Phase wurde anschließend 3 x mit NaCl-Lösung gewaschen. Man trocknete über Na₂SO₄, filtrierte ab und engte ein. Das Rohprodukt wurde dann an Kieselgel mit Essigester/ Petrolether (60:40) chromatographisch gereinigt. Man erhielt 35 mg (47%) der entsprechenden Boc-geschützten Verbindung. Zur Abspaltung der Schutzgruppen wurden 30 mg dieser Verbindung in 84 µl (1,1 mmol) Trifluoressigsäure und 840 µl Methylenchlorid gelöst. Die Reaktion wurde mittels DC kontrolliert. Das Produkt wurde nach Beendigung mit trockenem Ether versetzt, und der ausfallende Feststoff wurde mehrfach mit Ether gewaschen. Ausbeute: 22,5 mg (70%) **XIII**.

Die nachfolgende Tabelle 4 zeigt 7 Beispiele von Verbindungen der allgemeinen Formel (2) gemäß der Erfindung.

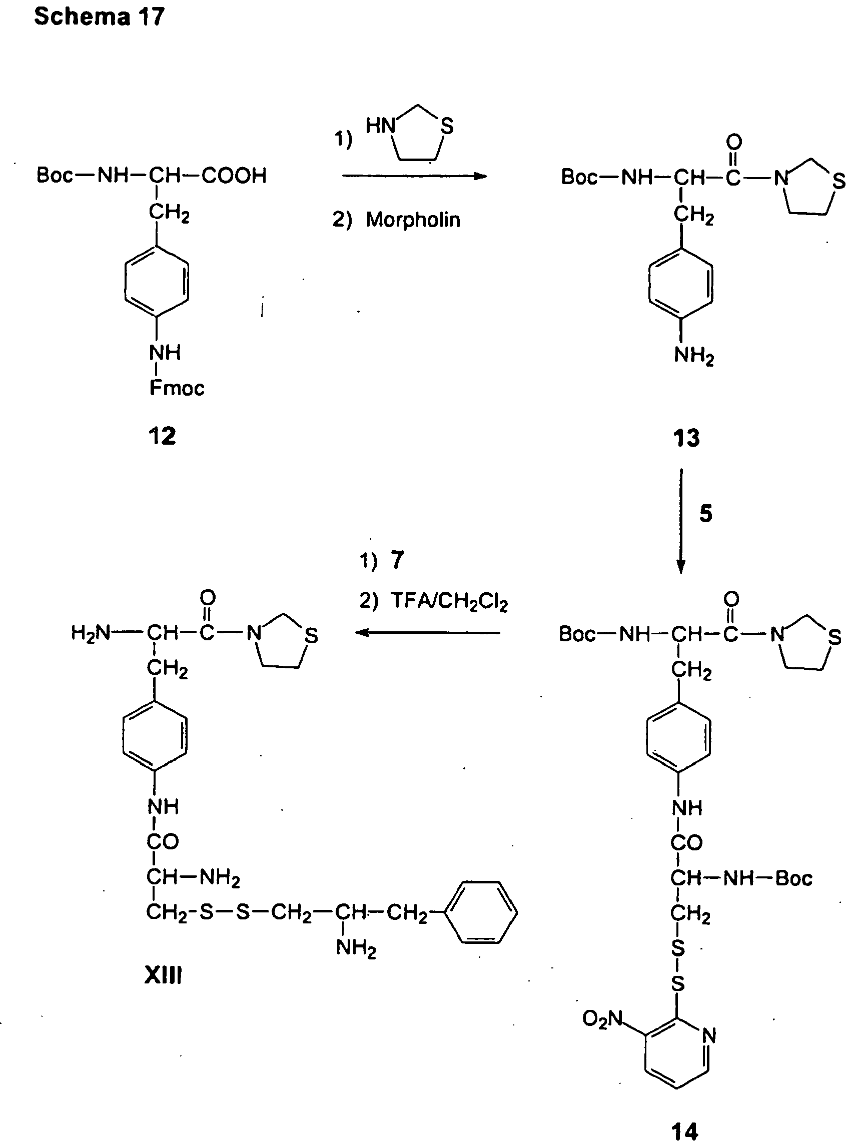

**Tabelle 4**

| Beispiele für Verbindungen der allgemeinen Formel **A - B - D - E (2)** | | | | | | | |
|---|---|---|---|---|---|---|---|
| 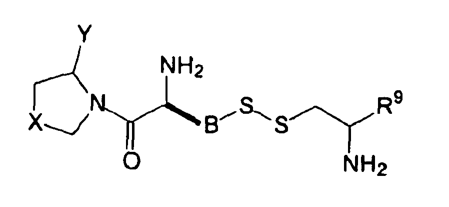 | | | | | | | |

| **Nr.** | **B** | **R⁹** | **X** | **Y** | **Summen formel** | **Mol.- gewicht** | **ESI-MS [m/e]** |
|---|---|---|---|---|---|---|---|
| VII | - CH₂- | 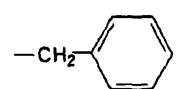 | S | H | C₁₅H₂₃N₃OS₃ | 357,6 | 358,0 |
| VIII | 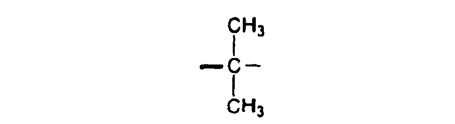 | 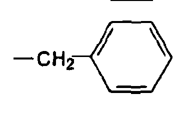 | S | H | C₁₇H₂₇N₃OS₃ | 385,6 | 386,0 |
| IX | 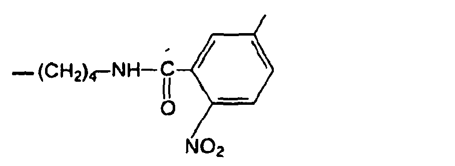 | 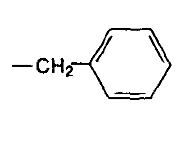 | S | H | C₂₅H₃₃N₅O₄S₃ | 563,8 | 564,1 |
| X | 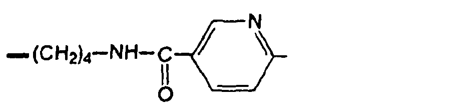 | 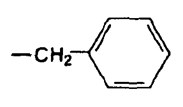 | S | H | C₂₄H₃₃N₅O₂S₃ | 519,8 | 520,1 |
| XI | 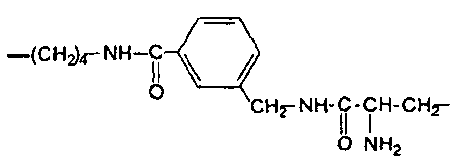 | 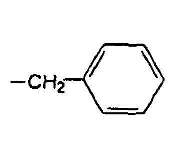 | S | H | C₂₉H₄₂N₆O₃S₃ | 618,9 | 619,2 |
| XII | 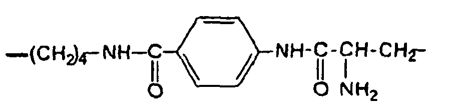 | 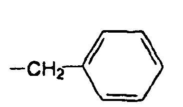 | S | H | C₂₈H₄₀N₆O₃S₃ | 604,8 | 605,2 |
| XIII | 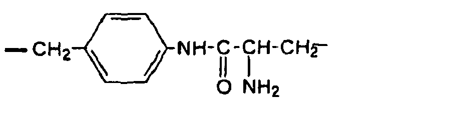 | 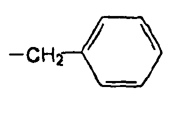 | S | H | C₂₄H₃₃N₅O₂S₃ | 519,8 | 520,2 |

### Duale Hemmung von APN und DPIV nach Freisetzung der Inhibitoren aus den Prodrugs der allgemeinen Formeln (1) und (2)

Von den in den vorgenannten Beispielen 1 und 2 hergestellten Verbindungen wurden 8 ausgewählt (Verbindungen I und II der allgemeinen Formel (1) und Verbindungen VII, VIII, X, XI, XII und XIII der allgemeinen Formel (2)). Die genannten Verbindungen wurden nach folgendem Verfahren mit Dithiothreitol (DTT) reduktiv behandelt, um die Inhibitoren aus den Prodrugs freizusetzen.

### Durchführung der Reduktion mit DTT:

Gleiche Volumina einer 7·10⁻² M Lösung des Inhibitors in Wasser bzw. DMF und einer 0,35M wässrigen DTT-Lösung (5 Equivalente) wurden 4 Stunden bei RT geschüttelt.

Im Anschluß an die reduktive Behandlung der Prodrugs erfolgte die Bestimmung der IC₅₀-Werte nach folgendem Verfahren:

### Bestimmmung der IC₅₀-Werte nach Reduktion:

Dipeptidylpeptidase IV aus Schweineniere (EC 3.4.14.5), lyophilisiertes Pulver (Sigma);
Substrat: Ala-Pro-AMC, [S]= 3·10⁻⁵ M (2 Km) im Ansatz
Puffer: 40 mM Tris/HCl-Puffer, pH 7,6, I = 0,125 im Ansatz

Leucin Aminopeptidase, microsomal, aus Schweineniere (EC 3.4.11.2), Suspension in 3,5 M (NH₄)₂SO₄ Lösung (Sigma)
Substrat: Leu-AMC, [S]= 1,5·10⁻⁴ M (2 Km) im Ansatz
Puffer: 40 mM Tris/HCl-Puffer, pH 7,2 im Ansatz

### Durchführung:

Aus dem Reduktionsansatz wurden durch Verdünnung mit Wasser 10 bis 12 Lösungen mit definierter Inhibitorkonzentration hergestellt. Diese wurden so gewählt, dass sie im relevanten Konzentrationsbereich, d.h. zwischen vollständiger Hemmung , als auch unbeeinflusster Enzymaktivität lagen.

Je 60 µl dieser Inhibitorlösungen bzw. Wasser (Standard) wurden mit 80 µl des jeweiligen verdünnten Enzyms in Puffer 15 Minuten bei 30°C inkubiert und danach 60 µl des entsprechenden AMC-Substrats in Wasser (Gesamtvolumen 200 µl) addiert. Die Freisetzung des 7-Amino-4-methyl-cumarins wurde mittels des Fluoreszenzplattenlesers *NOVOstar* der Firma BMG Labtechnologies über eine Zeitspanne von 20 Minuten bei 30°C kontinuierlich verfolgt. Die Enzymkonzentration wurde dabei so gewählt, daß während der Messzeit ein linearer Fluoreszenzanstieg erfolgte.

Die Excitations- bzw. Emissionswellenlängen betrugen 390 und 460 nm. Es wurden jeweils Doppelbestimmungen durchgeführt. Die Reaktionsgeschwindigkeiten (Ffuoreszenzanstiege/Minute) wurden gegen die Inhibitorkonzentration aufgetragen, und die IC₅₀-Werte wurden mit dem Computerprogramm *GraFit* berechnet.

Zum Vergleich wurden die IC₅₀-Werte etablierter Inhibitoren gegen DPIV (H-Lys[Z(NO₂)]-thiazolidid) und APN (Actinonin) bestimmt.

Die Ergebnisse sind der nachfolgenden Tabelle 5 zu entnehmen.

**Tabelle 5**

| **Duale Hemmung von APN und DP IV nach Freisetzung (Reduktion) der Inhibitoren aus den Prodrugs** | | |
|---|---|---|
| **Verbindung** | **APN (IC 50 [M])** | **DP IV (IC 50 [M])** |
| I | 1,84·10⁻⁴ | 2,25·10⁻⁵ |
| II | 1,08·10⁻⁴ | 8,07·10⁻⁶ |
| VII | 1,66·10⁻⁷ | 1,18·10⁻⁵ |
| VIII | 1,08·10⁻⁷ | 4,51·10⁻⁶ |
| X | 1,33·10⁻⁷ | 1,25·10⁻⁷ |
| XI | 1,90·10⁻⁷ | 2,80·10⁻⁷ |
| XII | 2,07·10⁻⁷ | 2,56·10⁻⁷ |
| XIII | 2,40·10⁻⁷ | 7,19·10⁻⁶ |
| Lys[Z(NO₂)]-thiazolidid | | 4,34·10⁻⁷ |
| Actinonin | 3,02·10⁻⁷ | |

### Beispiel 3

### Hemmung der Phytohämagglutinin-induzierten Proliferation mononukleärer Zellen (MNZ) gesunder Spender durch Verbindungen V bis XIII (Tabellen 3 und 4)

MNZ wurden aus dem peripheren Blut gesunder Spender mittels Dichtegradientenzentrifugation isoliert und in serumfreiem Medium (AIMV) mit 1µg/ml Phytohämagglutinin (PHA) stimuliert. Jeweils 5 x 10⁴ MNZ wurden in Anwesenheit verschiedener Inhibitorkonzentrationen (Dreifachbestimmungen) bei 37°C und 5% CO₂ in Mikrotestplatten für 48 Stunden inkubiert. Als Kontrollen wurden sowohl unstimulierte Zellen als auch PHA-stimulierte MNZ ohne Inhibitor unter identischen Bedingungen kultiviert. Die Proliferationsrate der Zellen wurde durch Einbau von Bromdesoxyuridin in neu synthetisierte DNS erfasst (Biotrak-Assay, GE Healthcare).

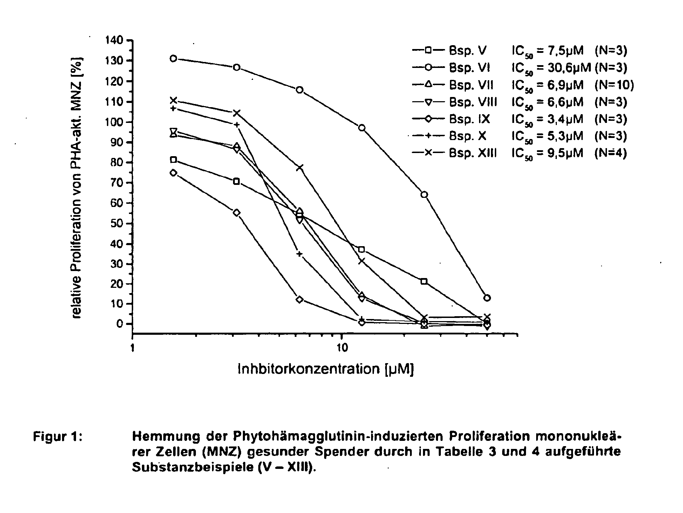

Die in Figur 1 gezeigten Kurven stellen jeweils die zusammengefassten Daten (relative Werte bezogen auf PHA-stimulierte Zellen in Abwesenheit von Inhibitor) von mindestens drei verschiedenen Spendern dar.

### Beispiel 4

### Therapeutische Wirkung der Verbindung VII (Tabelle 4) Im Mausmodell der Dextransulfat-induzierten Colitis

Das therapeutische Potential der Substanz(en) wurde in einem etablierten, chemisch induzierten Modell der Colitis getestet. In diesem Modell wird durch Dextransulfat-Natrium (DSS) eine Entzündungsreaktion im Colon der Tiere ausgelöst, deren histologisches Erscheinungsbild Ähnlichkeiten zur Colitis ulcerosa genannten, menschlichen Form der chronisch entzündlichen Darmerkrankung (akuter Schub) aufweist. Das Ausmaß der Entzündungsreaktion ist abhängig von der eingesetzten DSS-Konzentration. Die Erkrankungsstärke wird anhand eines etablierten Scoring-Systems erfasst, in das Veränderungen der Stuhlkonsistenz, der Nachweis von Blut im Kot sowie das Ausmaß des Gewichtsverlustes einfließen und mit Punkten bewertet werden.

Durch die Gabe des entzündungsauslösenden DSS während der gesamten Versuchsdauer kam es zu einem permanenten Fortschreiten der Erkrankung. Ab einer Punktzahl von 10 musste von einem potentiell tödlichen Erkrankungszustand ausgegangen werden. Der maximal erreichbare therapeutische Erfolg war das Aufhalten des Fortschreitens der Entzündungreaktion.

Weibliche Balb/c-Mäuse (8 Wochen alt) mit einem durchschnittlichen Gewicht von 20g erhielten 3% (w/v) Dextransulfat-Natrium mit dem Trinkwasser (ad libitum) während der gesamten Versuchsdauer. Bei allen Tieren waren nach 2 bis 3 Tagen Colitis-typische Symptome (blutige Durchfälle und Gewichtsverlust) nachweisbar. Jeweils 12 Tiere erhielten ab Versuchstag 3 täglich 100µg der Substanz VII, gelöst in physiologischer Kochsalzlösung (PBS), bzw. die entsprechende Menge des Lösungsmittels (PBS = Placebo-Kontrolle) durch intraperitoneale Injektion.

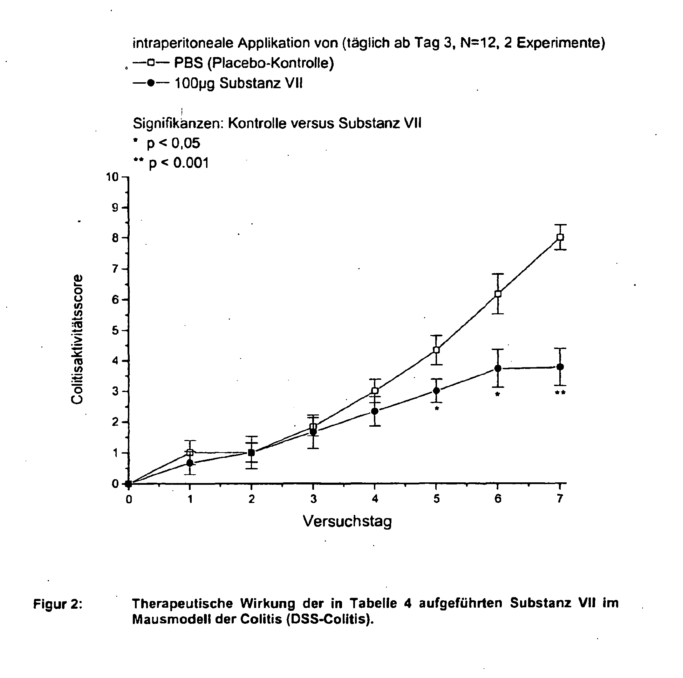

In der Grafik von Figur 2 ist die mittlere Erkrankungsstärke (Score-Punktzahl) der mit Substanz VII behandelten Mäuse im Vergleich zu Placebo-behandelten Tieren dargestellt. Die mittleren Score-Punktzahlen der mit Substanz VII behandelten Mäuse war an den Versuchstagen 5 bis 7 signifikant niedriger als die der Placebo-behandelten Tiere. Am Versuchsende erreichten die mit Substanz VII behandelten Tiere durchschnittlich etwa die halbe Score-Punktzahl der Kontrolltiere.

### Beispiel 5

### Therapeutischer Erfolg mehrerer in den Tabellen 3 und 4 aufgeführter Verbindungen der Formeln (1) und (2) im Mausmodell der DSS-induzierten Colitis

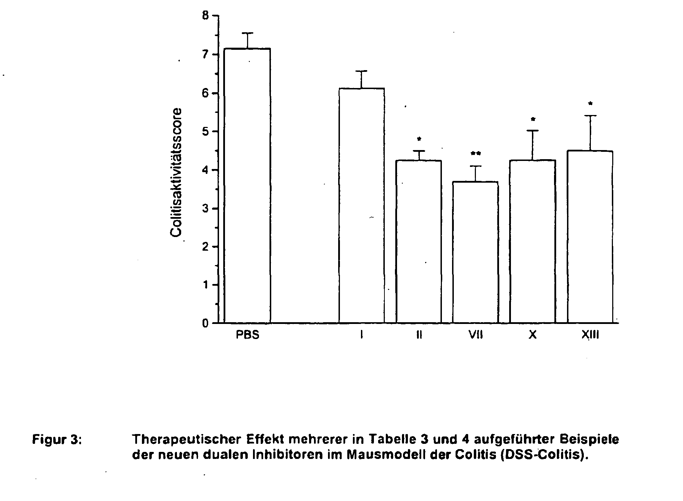

In Figur 3 ist das therapeutische Potential verschiedener Beispielsubstanzen am Versuchstag 7 (Versuchsende) im Mausmodell der DSS-induzierten Colitis dargestellt. Der Colitis-Aktivitätsscore wurde entsprechend der in Beispiel 4 beschriebenen Methodik bestimmt.

## Patentansprüche

1. Verbindungen der allgemeinen Formeln (1) oder (2)
A-B-D-B'-A' (1)
oder
A-B-D-E (2),
worin
- A und A' gleich oder verschieden sein können und für den Rest 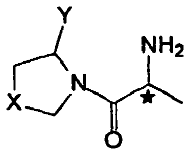 stehen, worin X für S, O, CH₂, CH₂CH₂, CH₂O oder CH₂NH steht und Y für H oder CN steht und * ein chirales Kohlenstoff-Atom vorzugsweise in der S-bzw. L-Konfiguration bezeichnet;
- B und B' gleich oder verschieden sein können und für einen O, N oder S enthaltenden oder nicht enthaltenden, unsubstituierten oder substituierten, unverzweigten oder verzweigten Alkylen-Rest, Cycloalkylen-Rest, Aralkylen-Rest, Heterocycloalkylen-Rest, Heteroarylalkylen-Rest, Arylamidoalkylen-Rest, Heteroarylamidoalkylen-Rest, unsubstituierten oder einfach oder mehrfach substituierten Arylen-Rest oder Heteroarylen-Rest mit einem oder mehreren fünf-, sechs- oder siebengliedrigen Ring(en) stehen;
- D für - S - S - oder - Se - Se - steht; und
- E für die Gruppe - CH₂ - CH (NH₂) - R⁹ bzw. - CH₂ - *CH (NH₂) - R⁹ steht, worin R⁹ für einen O, N oder S enthaltenden oder nicht enthaltenden unsubstituierten oder substituierten unverzweigten oder verzweigten Alkyl-Rest, Cycloalkyl-Rest, Aralkyl-Rest, Heterocycloalkyl-Rest, Heteroarylalkyl-Rest, Arylamidoalkyl-Rest, Heteroarylamidoalkyl-Rest, unsubstituierten oder einfach oder mehrfach substituierten Aryl-Rest oder Heteroaryl-Rest mit einem oder mehreren fünf-, sechs- oder siebengliedrigen Ringen steht und * ein chirales Kohlenstoff-Atom vorzugsweise in der S- bzw. L-Konfiguration bezeichnet;
- oder deren Säureadditionssalze mit organischen und/oder anorganischen Säuren.

2. Verbindungen der allgemeinen Formeln (1) oder (2) nach Anspruch 1, worin B und/oder B' stehen für
(a) einen Rest R¹, welcher steht für einen geradkettigen oder verzweigten Alkylen-Rest mit 1 bis 6 Kohlenstoff-Atomen, vorzugsweise welcher steht für -CH₂-, -CH₂-CH₂- oder (H₃C)₂-C<; oder
(b) einen Rest -(CH₂)ₙ - R² - R³ - R⁴ -, worin n für eine ganze Zahl von 1 bis 5 steht; R² für -NH- oder -(NH) - C (=NH) - NH - steht, wenn R³ für O = C< oder -SO₂- steht oder worin R² für O = C< steht, wenn R³ für - NH - steht; R⁴ für einen O, N oder S enthaltenden oder nicht enthaltenden unsubstituierten oder substituierten unverzweigten oder verzweigten Alkylen-Rest, Cycloalkylen-Rest, Aralkylen-Rest, Heterocycloalkylen-Rest, Heteroarylalkylen-Rest, unsubstituierten oder einfach oder mehrfach substituierten Arylen-Rest oder Heteroarylen-Rest mit einem oder mehreren fünf-, sechs- oder siebengliedrigen Ringen steht; oder
(c) einen Rest- R⁷- R⁸ -, worin R⁷ für einen einfach oder mehrfach substituierten Benzylenrest steht und R⁸ für eine Einfachbindung oder für einen O, N oder S enthaltenden oder nicht enthaltenden unsubstituierten oder substituierten unverzweigten oder verzweigten Alkylen-Rest, Cycloalkylen-Rest, Aralkylen-Rest, Heterocycloalkylen-Rest oder Heteroarylalkylen-Rest steht, der vorzugsweise eine oder mehrere Amino-Gruppen, Carbonyl-Gruppen oder Carboxyl-Gruppen als funktionelle Gruppen aufweisen kann, oder unsubstituierten oder einfach oder mehrfach substituierten Arylen-Rest oder Heteroarylen-Rest mit einem oder mehreren fünf-, sechs- oder siebengliedrigen Ringen steht.

3. Verbindungen der allgemeinen Formeln (1) und (2) nach Anspruch 1 oder Anspruch 2, worin B steht für einen Rest -(CH₂)ₙ - R² - R³ - R⁴ -, worin R⁴ steht für
- - CH(COOH)-R¹-, worin R¹ die oben angegebene Bedeutung hat, wenn R² für O = C < steht und R³ für -NH- steht; oder
- 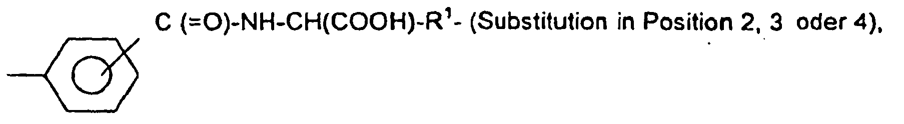 worin R¹ die oben angegebene Bedeutung hat, wenn R² für O = C < steht und R³ für -NH- steht; oder
- -CH (NHR⁵) -R¹-, wenn R² für -NH- oder -NH-C-(=NH)-NH- steht und R³ für O = C< steht, worin R⁵ für H oder einen Acyl-Rest steht, vorzugsweise für einen Benzyloxycarbonyl-Rest, einen Fluoren-9-ylmethoxycarbonyl-Rest, einen tert-Butyloxycarbonyl-Rest oder einen Benzoyl-Rest steht; oder
- 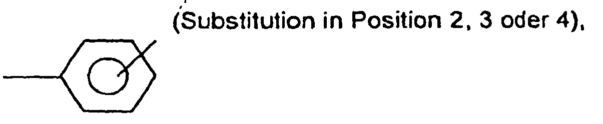 worin R⁴ für Phenylen
steht und R² für -NH- oder -NH-C(=NH)-NH- steht, wenn R³ für O = C< oder -SO₂- steht, oder worin R² für O = C < steht, wenn R³ für-NH- steht; oder
- 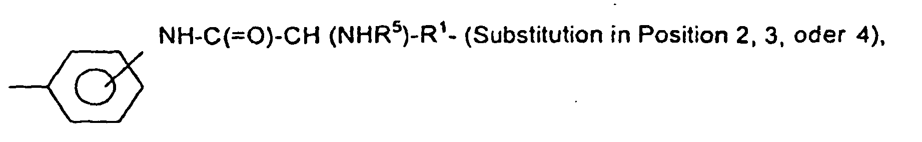 worin R⁵ für H oder einen Acyl-Rest steht, vorzugsweise für einen Benzyloxycarbonyl-Rest, einen Fluoren-9-ylmethoxycarbonyl-Rest oder einen Benzoyl-Rest steht, und R² für -NH- oder -NH-C(=NH)-NH- steht, wenn R³ für O = C< oder -SO₂- steht, oder worin R² für O = C < steht, wenn R³ für - NH- steht; oder
- 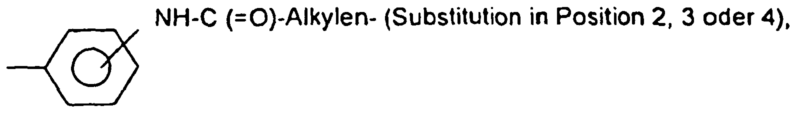 worin Alkylen für einen unverzweigten oder verzweigten Alkylen-Rest mit 1 bis 6 Kohlenstoff-Atomen steht und R² für -NH- oder -NH-C(=NH)-NH- steht, wenn R³ für O = C< oder -SO₂- steht, oder worin R² für O = C < steht, wenn R³ für -NH- steht; oder
- 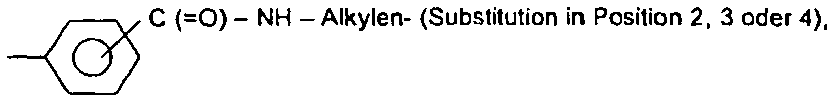 worin Alkylen für einen unverzweigten oder verzweigten Alkylen-Rest mit 1 bis 6 Kohlenstoff-Atomen steht und R² für -NH- oder -NH-C(=NH)-NH- steht, wenn R³ für O = C< oder -SO₂- steht, oder worin R² für O = C < steht, wenn R³ für -NH- steht; oder
- 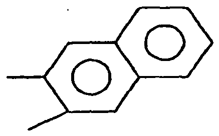 worin R² für -NH- oder -NH-C(=NH)-NH- steht, wenn R³ für O = C< oder -SO₂- steht, oder worin R² für O = C < steht, wenn R³ für -NH- steht; oder
- 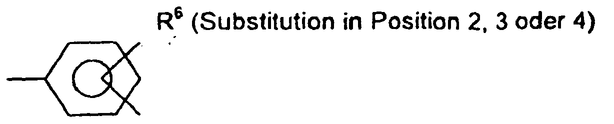 (Substitution am Ring in Abhängigkeit von der Position von R⁶), worin R⁶ für H, NO₂, CN, Halogen oder einen Acyl-Rest steht und R² für -NH- oder -NH-C(=NH)-NH- steht, wenn R³ für O = C< oder -SO₂- steht, oder worin R² für O = C< steht, wenn R³ für -NH- steht; oder
- 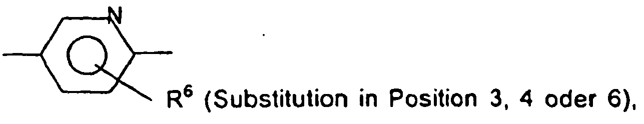 worin R⁶ für H, NO₂, CN, Halogen oder einen Acyl-Rest steht und R² für -NH- oder -NH-C(=NH)-NH- steht, wenn R³ für O = C< oder -SO₂- steht, oder worin R² für O = C < steht, wenn R³ für -NH- steht; oder
- 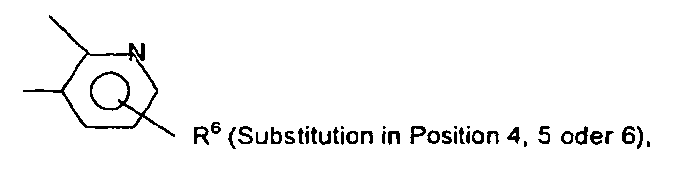 worin R⁶ für H, NO₂, CN, Halogen oder einen Acyl-Rest steht und R² für -NH- oder -NH-C(=NH)-NH- steht, wenn R³ für O = C< oder -SO₂- steht, oder worin R² für O = C < steht, wenn R³ für -NH- steht; oder
- 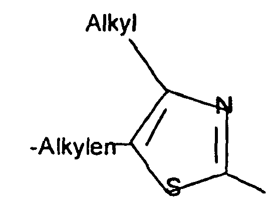 worin R² für -NH- oder -NH-C(=NH)-NH- steht, wenn R³ für O = C< oder -SO₂- steht, oder worin R² für O = C < steht, wenn R³ für -NH- steht.

4. Verbindungen der Formeln (1) oder (2) nach Anspruch 1 oder Anspruch 2, worin B steht für einen Rest - R⁷ - R⁸ -, worin R⁷ und R⁸ gemeinsam stehen für einen Rest
- 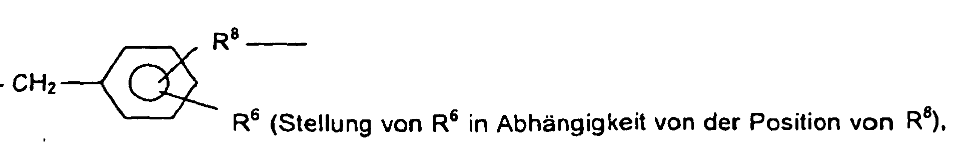 worin R⁸ und R⁶ die oben angegebenen Bedeutungen haben.

5. Verbindungen der Formeln (1) oder (2) nach Anspruch 1 oder Anspruch 2, worin B steht für einen Rest - R⁷ - R⁸ -, worin R⁷ für einen einfach oder mehrfach substituierten Benzylen-Rest steht und R⁸ steht für die folgenden Reste:
- NH - oder - C₁- bis C₆-Alkylen - NH - in Kombination mit
- - C (=O) - C₁- bis C₆-Alkylen - oder
- - C (=0) - Arylen - oder
- - SO₂ - Alkylen - oder
- - SO₂ - Arylen - oder
- -SO₂ 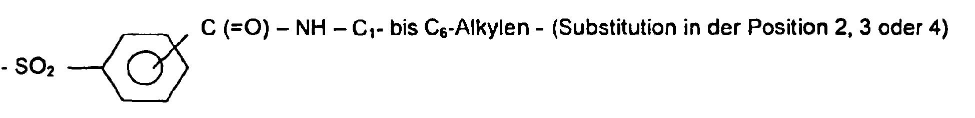 oder
- - C (=0) - CH (NHR⁵) - R¹, worin R¹ und R⁵ die oben angegebenen Bedeutungen haben; oder
- O = C< in Kombination mit
- - NH - C₁- bis C₆-Alkylen - oder
- - NH - Arylen - oder
- - NH - CH (COOH) - R¹ -, worin R¹ die oben genannten Bedeutungen aufweist; oder
- - 0 - C₁- bis C₆-Alkylen - oder
- - 0 - Arylen - oder
- - 0 - Alkylen - NH - C (=0) - CH (NH₂) - R¹ -, worin R¹ die oben genannten Bedeutungen aufweist, oder
- - 0 - C₁- bis C₆-Alkylen - C (=O) - NH - CH (COOH) - R¹ -, worin R¹ die oben genannten Bedeutungen aufweist.

6. Verbindungen der allgemeinen Formeln (1) oder (2) nach einem der Ansprüche 1 bis 5, worin die Säureadditionssalze Salze pharmazeutisch annehmbarer Säuren sind, bevorzugt worin die Säureadditionssalze Hydrochloride, Trifluoracetate, Tartrate, Succinate, Formiate und/oder Citrate sind.

7. Verbindungen der allgemeinen Formel (1) nach einem der Ansprüche 1 bis 6, nämlich Verbindungen der allgemeinen Formel (1 a) 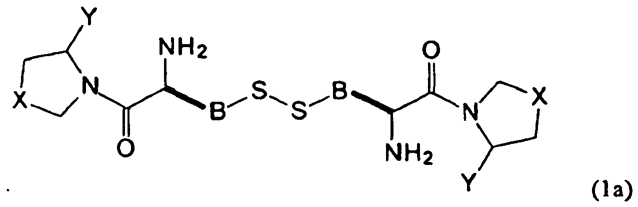 worin X, Y und B die oben angegebenen Bedeutungen haben, und ihre Säureadditionssalze, bevorzugt ihre Säureadditionssalze mit pharmazeutisch annehmbaren anorganischen und/oder organischen Säuren.

8. Verbindungen der allgemeinen Formel (1a) nach Anspruch 7, worin X, Y und B die nachfolgend angegebenen Bedeutungen haben
| **Nr.** | **B** | **X** | **Y** | **Summen-Formel** |
|---|---|---|---|---|
| I | - CH₂- | -CH₂- | H | C₁₄H₂₆N₄O₂S₂ |
| II | - CH₂- | S | H | C₁₂H₂₂N₄O₂S₄ |
| III | - CH₂- | -CH₂- | CN | C₁₆H₂₄N₆O₂S₂ |
| IV | 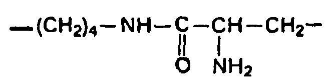 | S | H | C₂₄H₄₆N₈O₄S₄ |
| V | 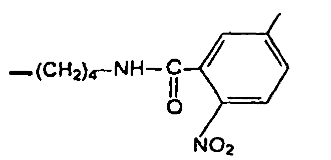 | S | H | C₃₂H₄₂N₈O₈S₄ |
| VI | 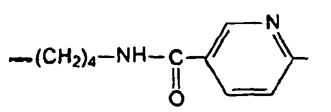 | S | H | C₃₀H₄₂N₈O₄S₄ |
und ihre Säureadditionssalze, bevorzugt ihre Säureadditionssalze mit pharmazeutisch annehmbaren anorganischen und/oder organischen Säuren.

9. Verbindungen der allgemeinen Formel (2) nach einem der Ansprüche 1 bis 6, nämlich Verbindungen der allgemeinen Formel (2a) 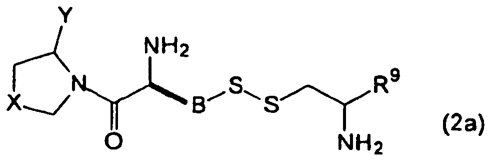 worin X, Y, R⁹ und B die oben angegebenen Bedeutungen haben, und ihre Säureadditionssalze, bevorzugt ihre Säureadditionssalze mit pharmazeutisch annehmbaren anorganischen und/oder organischen Säuren.

10. Verbindungen der allgemeinen Formeln (2a) nach Anspruch 9, worin X, Y, R⁹ und B die folgenden Bedeutungen haben:
| **Nr.** | **B** | **R⁹** | **X** | **Y** | **Summenformel** |
|---|---|---|---|---|---|
| VII | - CH₂- | 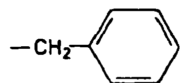 | S | H | C₁₅H₂₃N₃OS₃ |
| VIII | 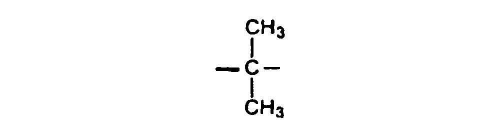 | 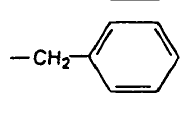 | S | H | C₁₇H₂₇N₃OS₃ |
| IX | 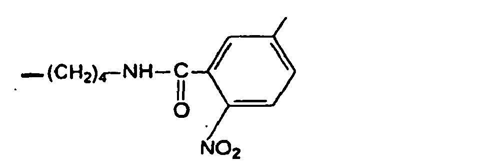 | 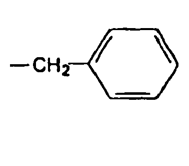 | S | H | C₂₅H₃₃N₅O₄S₃ |
| X | 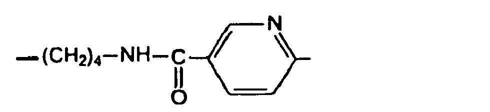 | 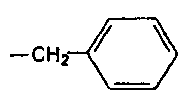 | S | H | C₂₄H₃₃N₅O₂S₃ |
| XI | 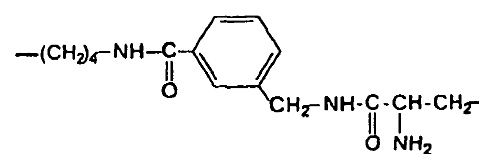 | 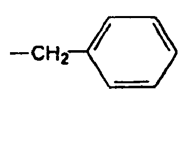 | S | H | C₂₈H₄₂N₈O₃S₃ |
| XII | 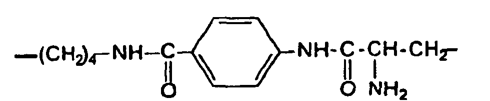 | 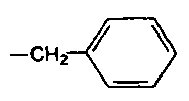 | S | H | C₂₈H₄₀N₆O₃S₃ |
| XIII | 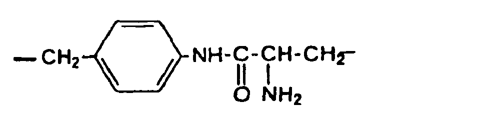 | 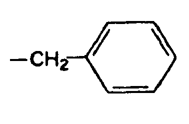 | S | H | C₂₄H₃₃N₅O₂S₃ |
und ihre Säureadditionssalze, bevorzugt ihre Säureadditionssalze mit pharmazeutisch annehmbaren anorganischen und/oder organischen Säuren.

11. Verfahren zur Herstellung von Verbindungen der allgemeinen Formeln
A - B - D - B^{I} - A^{I} (1)
oder
A - B - D - E (2),
worin
- A, B, D und E die in den Ansprüchen 1 bis 10 angegebenen Bedeutungen haben, worin man entsprechend Syntheseschema 1
- Verbindungen der allgemeinen Formel 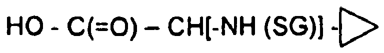 worin (SG) eine Schutzgruppe bedeutet und 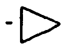 für ein Strukturelement von B steht, mit einer heterocyclischen Verbindung der allgemeinen Formel 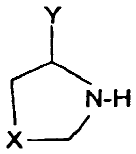 umsetzt, worin X für S, O, CH₂, CH₂CH₂. CH₂O oder CH₂NH steht und Y für H oder CN steht; das erhaltene Kondensationsprodukt der Formel 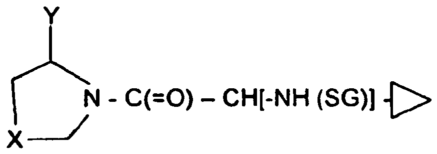 mit einer Verbindung der allgemeinen Formel 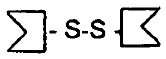 umsetzt, worin 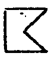 für ein Strukturelement von B steht; und das erhaltene Reaktionsprodukt unter Abspaltung der Schutzgruppen (SG) in eine Verbindung der allgemeinen Formel A - B - D - B' - A' (1) überführt, in der A und A' gleich oder verschieden sein können und B und B' gleich oder verschieden sein können und worin A, A'. B, B' und D die oben angegebenen Bedeutungen haben können; oder
- Verbindungen der allgemeinen Formel 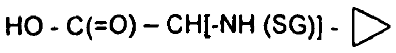 worin (SG) eine Schutzgruppe bedeutet und 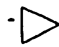 für ein Strukturelement von B steht, mit einer heterocyclischen Verbindung der allgemeinen Formel 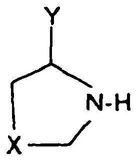 umsetzt, worin X für S, O, CH₂, CH₂CH₂, CH₂O oder CH₂NH steht und Y für H oder CN steht; das erhaltene Kondensationsprodukt der Formel 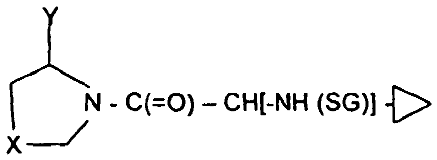 mit einer Verbindung der allgemeinen Formel 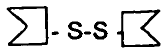 umsetzt, worin 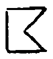 für ein Strukturelement von B steht; und das erhaltene Reaktionsprodukt mit einer Verbindung der allgemeinen Formel
HS - CH₂ - CH [- NH - (SG)] - (R⁹)
und nach Abspaltung der Schutzgruppen (SG) zu einer Verbindung der allgemeinen Formel A - B - D - E (2) umsetzt, worin A, B, D und E die oben angegebenen Bedeutungen haben können; oder
- eine Verbindung der allgemeinen Formel 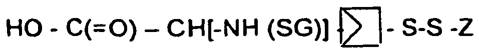 worin (SG) für eine Schutzgruppe steht, 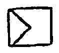 für ein Strukturelement von B steht und Z für einen Rest steht, der eine -S-S-Gruppe für einen Thiolaustausch aktiviert, mit einer heterocyclischen Verbindung der allgemeinen Formel 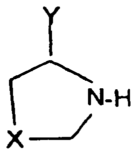 umsetzt, worin X für S, O, CH₂, CH₂CH₂, CH₂O oder CH₂NH steht und Y für H oder CN steht; das erhaltene Kondensationsprodukt der Formel 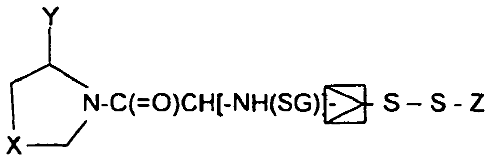 mit einer Verbindung der allgemeinen Formel
HS - CH₂ - CH [- NH - (SG)] - (R⁹)
und nach Abspaltung der Schutzgruppen (SG) zu einer Verbindung der allgemeinen Formel A - B - D - E (2) umsetzt, worin A, B, D und E die oben angegebenen Bedeutungen haben können; oder
- eine Verbindung der allgemeinen Formel 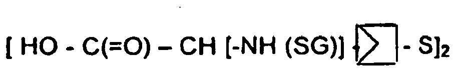 worin SG für eine Schutzgruppe steht und 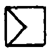 für ein Strukturelement von B steht, mit einer heterocyclischen Verbindung der allgemeinen Formel 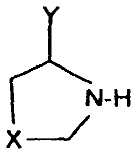 umsetzt, worin X für S, O, CH₂. CH₂CH₂, CH₂O oder CH₂NH steht und Y für H oder CN steht; und das erhaltene Reaktionsprodukt mit einer Verbindung der allgemeinen Formel
HS-CH₂-CH [- NH - (SG)] - R⁹
zu einer Verbindung der allgemeinen Formel A - B - D - E (2) umsetzt, worin A, B, D und E die oben angegebenen Bedeutungen haben können; oder
- eine Verbindung der allgemeinen Formel 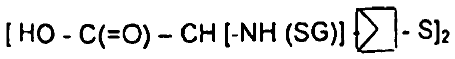 worin SG für eine Schutzgruppe steht und 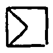 für ein Strukturelement von B steht, mit einer heterocyclischen Verbindung der allgemeinen Formel 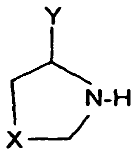 umsetzt, worin X für S, O, CH₂, CH₂CH₂, CH₂O oder CH₂NH steht und Y für H oder CN steht; und das erhaltene Reaktionsprodukt unter Abspaltung der Schutzgruppen SG in eine Verbindung der allgemeinen Formel A - B - D - B' - A' (1) überführt, in der A und A' gleich oder verschieden sein können und B und B' gleich oder verschieden sein können und worin A, A', B, B' und D die oben angegebenen Bedeutungen haben können; oder
- Verbindungen der allgemeinen Formel 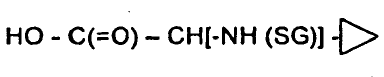 worin (SG) eine Schutzgruppe bedeutet und 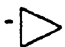 für ein Strukturelement von B steht, mit einer heterocyclischen Verbindung der allgemeinen Formel 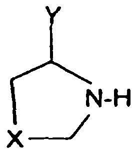 umsetzt, worin X für S, O, CH₂, CH₂CH₂, CH₂O oder CH₂NH steht und Y für H oder CN steht; das erhaltene Kondensationsprodukt der Formel 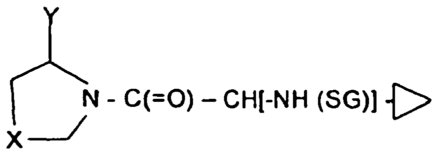 mit einer Verbindung der allgemeinen Formel 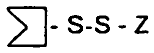 umsetzt, worin 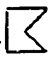 für ein Strukturelement von B steht und Z für einen Rest steht, der eine -S-S-Gruppe für einen Thiolaustausch aktiviert, und (SG) für eine Schutzgruppe steht; und das erhaltene Reaktionsprodukt der Formel 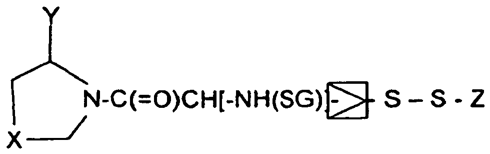 mit einer Verbindung der allgemeinen Formel
HS - CH₂ - CH [- NH - (SG)] - (R⁹)
und nach Abspaltung der Schutzgruppen (SG) zu einer Verbindung der allgemeinen Formel A - B - D - E (2) umsetzt, worin A, B, D und E die oben angegebenen Bedeutungen haben können.

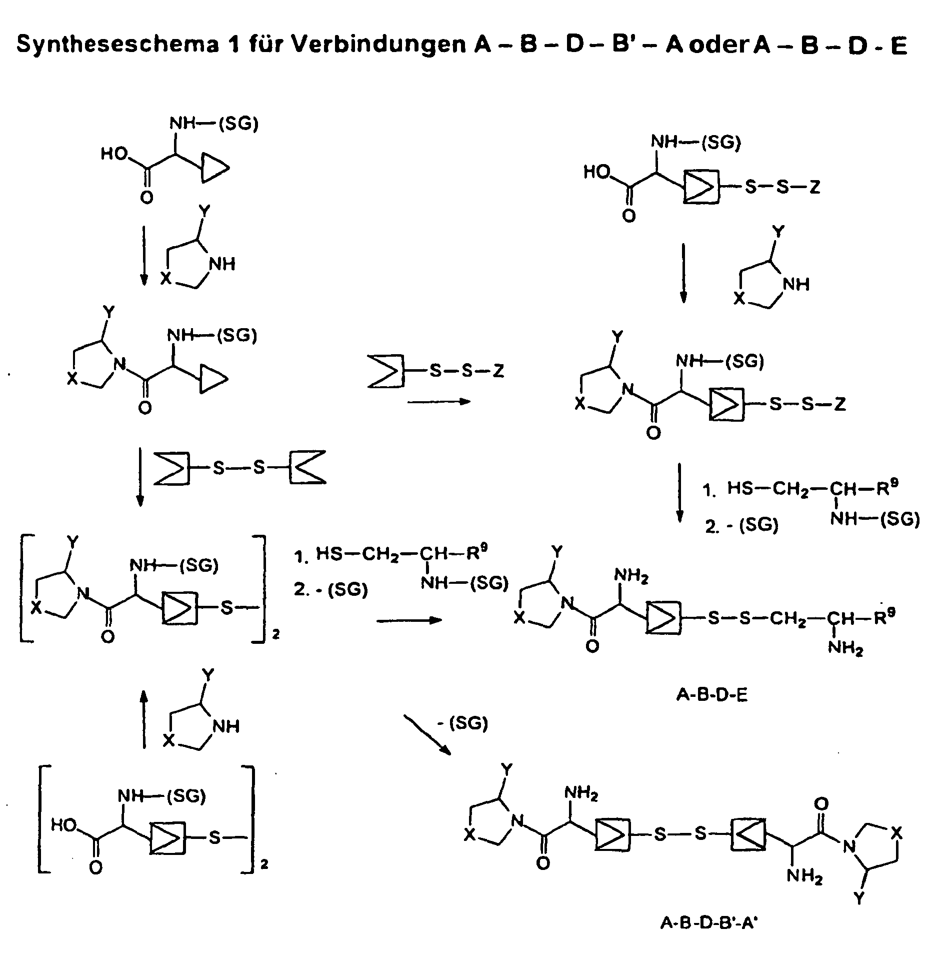

12. Verbindungen nach einem oder mehreren der Ansprüche 1 bis 10 zur Verwendung in der Medizin.

13. Verbindungen nach Anspruch 12 als Inhibitoren-Vorstufen oder Inhibitoren-Prodrugs.

14. Verbindungen nach Anspruch 12 oder Anspruch 13 als Inhibitoren der Dipeptidylpeptidase IV und Peptidasen mit analoger enzymatischer Wirkung sowie als Vorstufen und/oder Prodrugs für Inhibitoren der Dipeptidylpeptidase IV und Peptidasen mit analoger enzymatischer Wirkung sowie der Alanyl-Aminopeptidase N (APN) und Peptidasen mit analoger enzymatischer Wirkung.

15. Verbindungen nach Anspruch 12 oder Anspruch 13 zur Verwendung als Vorstufen oder Prodrugs für Inhibitoren der Dipeptidylpeptidase IV (DPIV) und Peptidasen mit analoger enzymatischer Wirkung, Alanyl-Aminopeptidase N (APN) und Peptidasen mit analoger enzymatischer Wirkung.

16. Verbindungen nach einem der Ansprüche 12 bis 15 zur Verwendung als Prodrugs für Inhibitoren der Enzyme Dipeptidylpeptidase IV (DPIV) und Alanyl-Aminopeptidase N (APN).

17. Verwendung mindestens einer Verbindung der allgemeinen Formeln (1) oder (2) nach einem der Ansprüche 1 bis 10 zur Herstellung eines Medikaments oder einer kosmetischen Zubereitung zur Prophylaxe und Therapie von Erkrankungen mit überschießender Immunantwort und entzündlicher Genese, einschließlich Arteriosklerose, neuronalen Erkrankungen, zerebralen Schädigungen, Hauterkrankungen, Tumorerkrankungen und Virus-bedingten Erkrankungen sowie Diabetes Typ II.

18. Verwendung nach Anspruch 17, worin die mindestens eine Verbindung der allgemeinen Formeln (1) oder (2) nach einem der Ansprüche 1 bis 10 mindestens einen Inhibitor der Dipeptidylpeptidase IV (DPIV) und/oder Peptidasen mit analoger enzymatischer Wirkung, sowie der Alanyl-Aminopeptidase N (APN) und/oder Peptidasen mit analoger enzymatischer Wirkung generiert.

19. Verwendung nach Anspruch 17 oder 18 unter reduzierenden physiologischen oder pathophysiologischen Bedingungen, vorzugsweise Bedingungen, unter denen -S-S-Bindungen oder -Se-Se-Bindungen in -SH-Gruppen oder -SeH-Gruppen umgewandelt werden.

20. Pharmazeutische Zubereitung, umfassend wenigstens eine Verbindung wenigstens einer der allgemeinen Formeln (1) oder (2) nach einem der Ansprüche 1 bis 10, gegebenenfalls zusammen mit einem oder mehreren pharmazeutisch annehmbaren Träger(n), Hilfsstoff(en) und/oder Adjuvant(ien).

21. Kosmetische Zubereitung, umfassend wenigstens eine Verbindung wenigstens einer der allgemeinen Formeln (1) oder (2) nach einem der Ansprüche 1 bis 10, gegebenenfalls zusammen mit einem oder mehreren kosmetisch annehmbaren Träger(n), Hilfsstoff(en) und/oder Adjuvant(ien).

## Claims

1. Compounds of the general formulae (1) or (2)
A-B-D-B'-A' (1)
or
A - B - D - E (2),
in which
- A and A' may be identical or different and are the residue 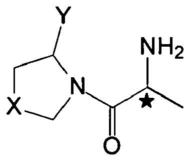 in which X is S, O, CH₂, CH₂CH₂, CH₂O or CH₂NH, and Y is H or CN, and * designates a chiral carbon atom preferably in the S-, or L-configuration;
- B and B' may be identical or different and are an O, N or S containing or non-containing, unsubstituted or substituted, unbranched or branched alkylene residue, cycloalkylene residue, aralkylene residue, heterocycloalkylene residue, heteroarylalkylene residue, arylamidoalkylene residue, heteroarylamidoalkylene residue, unsubstituted or mono- or poly-substituted arylene residue or heteroarylene residue having one or more five-, six- or seven-membered ring(s);
- D is -S-S- or -Se-Se-; and
- E is the group -CH₂-CH(NH₂)-R⁹ or -CH₂-*CH(NH₂)-R⁹, respectively in which R⁹ is an O, N or S containing or non-containing, unsubstituted or substituted, unbranched or branched alkyl residue, cycloalkyl residue, aralkyl residue, heterocycloalkyl residue, heteroarylalkyl residue, arylamidoalkyl residue, heteroarylamidoalkyl residue, unsubstituted or mono- or polysubstituted aryl residue or heteroaryl residue having one or more five-, six- or seven-membered ring(s) and * designates a chiral carbon atom preferably in the S-, or L-configuration;
- or the acid addition salts thereof with organic and/or inorganic acids.

2. Compounds of the general formulae (1) or (2) according to Claim 1, in which B and/or B' is
(a) a residue R¹, which is a straight-chained or branched alkylene residue having 1 to 6 carbon atoms preferably which is -CH₂-, -CH₂-CH₂- or (H₃C)₂-C<; or
(b) a residue -(CH₂)ₙ - R² - R³ - R⁴-, in which n is an integer from 1 to 5; R² is -NH- or -(NH)-C(=NH)-NH-, if R³ is O = C< or -SO₂- or in which R² is O = C< if R³ is -NH-; R⁴ is an O, N or S containing or non-containing unsubstituted or substituted unbranched or branched alkylene-residue, cycloalkylene-residue, aralkylene-residue, heterocycloalkylene-residue, heteroarylalkylene-residue, unsubstituted or mono-substituted or poly-substituted arylene-residue or heteroarylene-residue with one or more five-, six- or seven-membered ring(s); or
(c) is a residue - R⁷ - R⁸ -, in which R⁷ is a mono- or poly-substituted benzylene residue and R⁸ is a single bond or an O, N or S containing or non-containing unsubstitued or substituted, unbranchend or branched alkylene residue, cycloalkylene residue, aralkylene residue, heterocycloalkylene residue or heteroarylalkylene residue, which might contain as functional groups preferably one or more amino groups, carbonyl groups or carboxyl groups or is an unsubstituted or mono- or poly-substituted arylene residue or heteroarylene residue with one or more five-, six-, or seven-membered ring(s).

3. Compounds of the general formulae (1) or (2) according to Claim 1 or Claim 2, in which B is a residue -(CH₂)ₙ - R² - R³ - R⁴ in which R⁴ is -CH(COOH)-R¹-, in which R¹ has the afore-mentioned meaning if R² is O = C < and R³ is -NH-; or
- 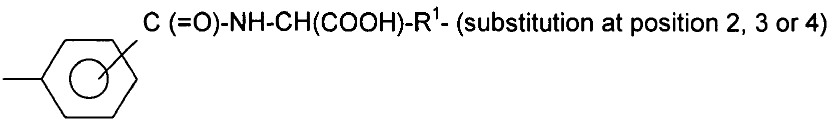 in which R¹ has the afore-mentioned meaning if R² is O = C < and R³ is -NH-; or
- -CH (NHR⁵) -R¹- if R² is -NH- or -NH-C-(=NH) -NH- and R³ is O = C< in which R⁵ is H or an acyl residue prefarably a benzyloxycarbonyl residue, a fluorene-9-ylmethoxycarbonyl residue, a tert-butyloxycarbonyl residue or a benzoyl residue; or
- 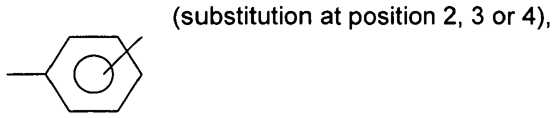 in which R⁴ is phenylene and R² is -NH- or -NH-C(=NH)-NH- if R³ is O = C< or -SO₂- or in which R² is O = C < if R³ is -NH-; or
- 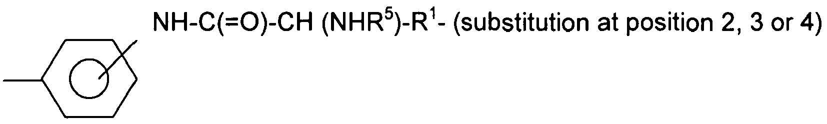 in which R⁵ is H or an acyl residue preferably a benzyloxycarbonyl residue, a fluoren-9-ylmethoxycarbonyl residue or a benzoyl residue and R² is -NH- or -NH-C(=NH) -NH- if R³ is O = C< or -SO₂- or in which R² is O = C < if R³ is -NH-; or
- 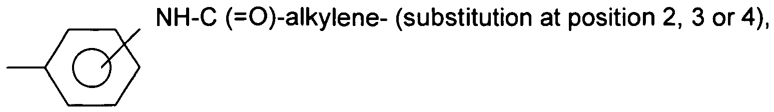 in which alkylene is an unbranched or branched alkylene residue having 1 to 6 carbon atoms and R² is -NH- or -NH-C(=NH)-NH- if R³ is O = C< or -SO₂- or in which R² is O = C < if R³ is -NH-; or
- 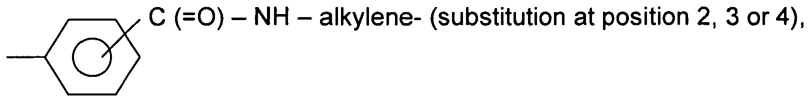 in which alkylene is an unbranched or branched alkylene residue having 1 to 6 carbon atoms and R² is -NH- or -NH-C(=NH)-NH- if R³ is O = C< or -SO₂- or in which R² is O = C < if R³ is -NH-; or
- 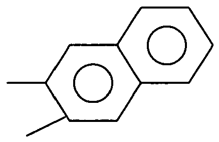 in which R² is -NH- or -NH-C(=NH) -NH- if R³ is O = C< or -SO₂- or in which R² is O = C < if R³ is -NH- ; or
- 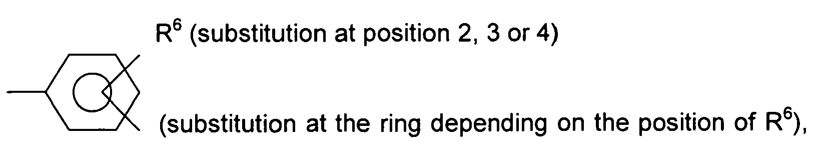 in which R⁶ is H, NO₂, CN, halogen or an acyl residue and R² is -NH- or
- NH-C(=NH) -NH- if R³ is O = C< or -SO₂- or in which R² is O = C < if R³ is
- NH-; or
- 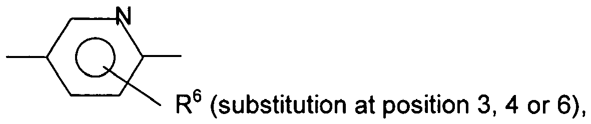 in which R⁶ is H, NO₂, CN, halogen or an acyl residue and R² is -NH- or -NH-C(=NH) -NH- if R³ is O = C< or -SO₂- or in which R² is O = C < if R³ is -NH- ; or
- 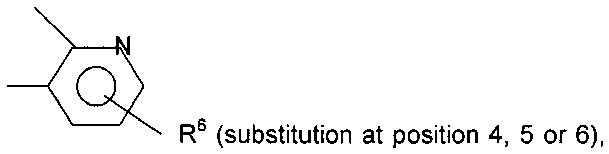 in which R⁶ is H, NO₂, CN, halogen or an acyl residue and R² is -NH- or -NH-C(=NH) -NH- if R³ is O = C< or -SO₂- or in which R² is O = C < if R³ is -NH; or
- 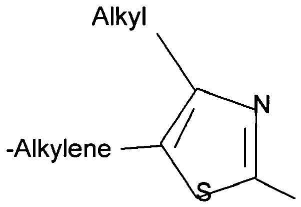 in which R² is -NH- or -NH-C(=NH) -NH- if R³ is O = C< or -SO₂- or in which R² is O = C < if R³ is -NH-.

4. Compounds of the formulae (1) or (2) according to Claim 1 or Claim 2, in which B is a residue - R⁷ - R⁸ - in which R⁷ and R⁸ in combination are a residue
- 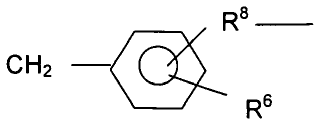 (position of R⁶ is dependent of the position R⁸) in which R⁸ and R⁶ have the afore-mentioned meanings.

5. Compounds of the formulae (1) or (2) according to any of Claim 1 or Claim 2, in which B is a residue - R⁷ - R⁸ - in which R⁷ is a mono- or poly-substituted benzylene residue and R⁸ is one of the following residues
- NH - or - C₁- to C₆-alkylene - NH - in combination with
- - C (=O) - C₁- to C₆-alkylene - or
- - C (=O) - arylene - or
- - SO₂ -alkylene - or
- - SO₂ -arylene - or
- 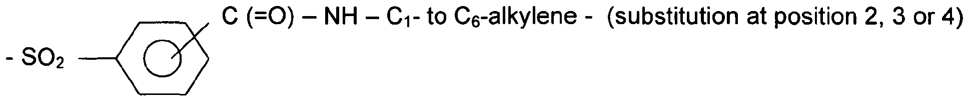 or
- - C (=O) - CH (NHR⁵) - R¹ in which R¹ and R⁵ have the afore-mentioned meanings; or
- 0 = C< in combination with
- - NH - C₁- to C₆-alkylene - or
- - NH - arylene - or
- - NH - CH (COOH) - R¹ - in which R¹ has the afore-mentioned meanings; or
- - 0 - C₁- to C₆-alkylene - or
- - 0 - arylene - or
- - 0 -alkylene - NH - C (=O) - CH (NH₂) - R¹ - in which R¹ has the afore-mentioned meanings, or
- - 0 - C₁- to C₆-alkylene - C (=O) - NH - CH (COOH) - R¹ -, in which R¹ has the afore-mentioned meanings.

6. Compounds of the general formulae (1) or (2) according to any of Claims 1 to 5, in which the acid addition salts are salts of pharmaceutically acceptable acids preferably in which the acid addition salts are hydrochlorides, trifluoracetates, tartrates, succinates, formiates and/or citrates.

7. Compounds of the general formula (1) according to any of Claims 1 to 6, namely compounds of the general formula (1a) 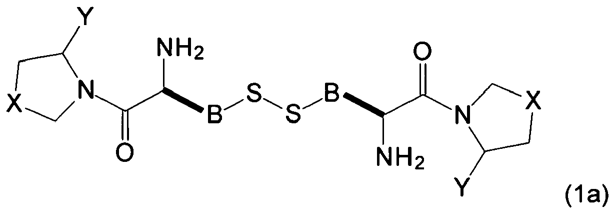 in which X, Y and B have the afore-mentioned meanings and the acid additions salts thereof, preferably the acid additions salts with pharmaceutically acceptable inorganic and/or organic acids thereof.

8. Compounds of the general formula (1a) according to Claim 7, in which X, Y and B have the following meanings
| **No.** | **B** | **X** | **Y** | **Empirical formula** |
|---|---|---|---|---|
| I | - CH₂- | -CH₂- | H | C₁₄H₂₆N₄O₂S₂ |
| II | - CH₂- | S | H | C₁₂H₂₂N₄O₂S₄ |
| III | - CH₂- | -CH₂- | CN | C₁₆H₂₄N₆O₂S₂ |
| IV | 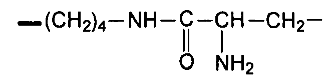 | S | H | C₂₄H₄₆N₈O₄S₄ |
| V | 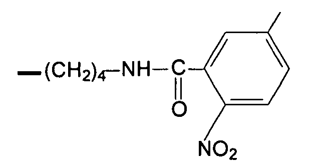 | S | H | C₃₂H₄₂N₈O₈S₄ |
| VI | 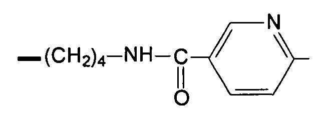 | S | H | C₃₀H₄₂N₈O₄S₄ |
and the acid addition salts thereof, preferably the acid additions salts with pharmaceutically acceptable inorganic and/or organic acids thereof.

9. Compounds of the general formula (2) according to any of Claims 1 to 6, namely compounds of the general formula (2a) 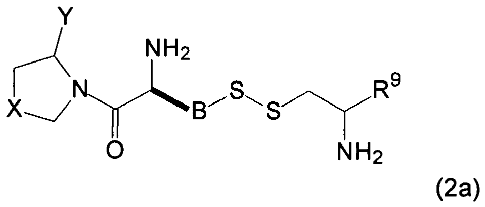 in which X, Y, R⁹ and B have the afore-mentioned meanings and the acid addition salts thereof, preferably the acid addition salts thereof with pharmaceutically acceptable inorganic and/or organic acids.

10. Compounds of the general formula (2a) according to Claim 9, in which X, Y, R⁹ and B have the following meanings:
| **No.** | **B** | **R⁹** | **X** | **Y** | **Empirical formula** |
|---|---|---|---|---|---|
| VII | - CH₂- | 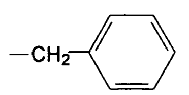 | S | H | C₁₅H₂₃N₃OS₃ |
| VIII | 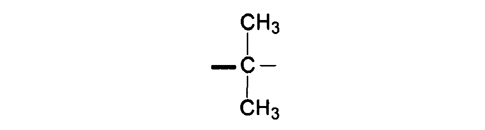 | 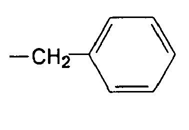 | S | H | C₁₇H₂₇N₃OS₃ |
| IX | 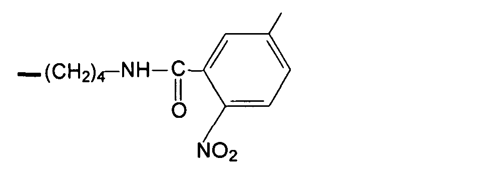 | 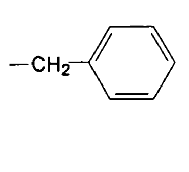 | S | H | C₂₅H₃₃N₅O₄S₃ |
| X | 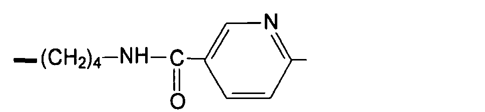 | 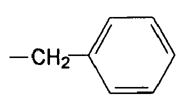 | S | H | C₂₄H₃₃N₅O₂S₃ |
| XI | 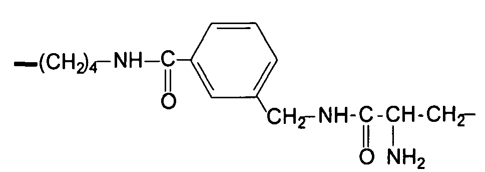 | 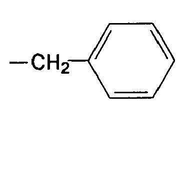 | S | H | C₂₉H₄₂N₆O₃S₃ |
| XII | 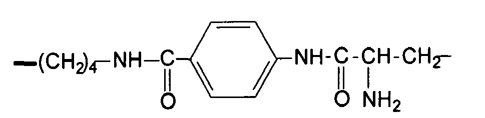 | 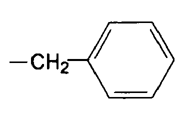 | S | H | C₂₈H₄ₒN₆O₃S₃ |
| XIII | 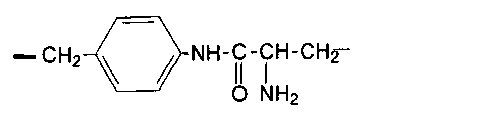 | 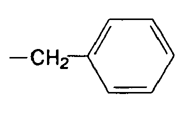 | S | H | C₂₄H₃₃N₅O₂S₃ |
and the acid addition salts thereof, preferably the acid addition salts thereof with pharmaceutically acceptable inorganic and/or organic acids.

11. Process to prepare compounds of the general formulae
A - B - D - B' - A' (1)
or
A - B - D - E (2)
in which
- A, B, D and E have the meanings mentioned in Claims 1 to 10, in which according to scheme of synthesis 1
- compounds of the general formula 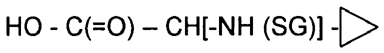 in which (SG) is a protecting group and 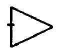 is a structure element of B are transformed with a heterocyclic compound of the general formula 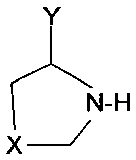 in which X is S, O, CH₂, CH₂CH₂, CH₂O or CH₂NH and Y is H or CN; the obtained condensation product of the formula 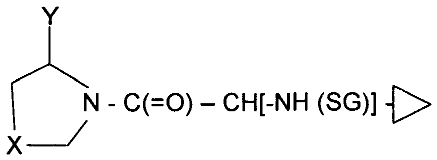 is transformed with a compound of the general formula 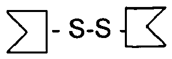 in which 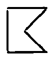 is a structure element of B; and the resulting reaction product is transformed being cleaved from the protecting group (SG) into a compound of the general formula A - B - D - B' - A' (1) in which A and A' may be identical or different and B and B' may be identical or different and in which A, A', B, B' and D may have the afore-mentioned meanings; or
- compounds of the general formula 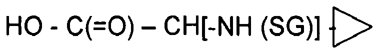 in which (SG) is a protecting group and 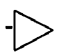 is a structure element of B are transformed with a heterocyclic compound of the general formula 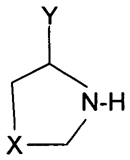 in which X is S, O, CH₂, CH₂CH₂, CH₂O or CH₂NH and Y is H or CN; the resulting condensation product of the formula 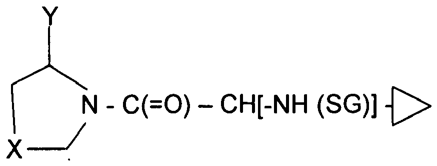 is transformed with a compound of the general formula 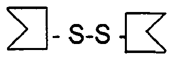 in which 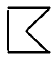 is a structure element of B; and the resulting reaction product is transformed with a compound of the general formula
HS-CH₂-CH[-NH-(SG)]-(R⁹)
and is transformed being cleaved from the protecting groups (SG) into a compound of the general formula A - B - D - E (2), in which A, B, D and E may have the afore-mentioned meanings; or
- a compound of the general formula 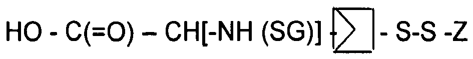 in which (SG) is a protecting group, 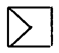 is a structure element of B and Z is a residue, which activates an -S-S-group for a thiol exchange, is transformed with a heterocyclic compound of the general formula 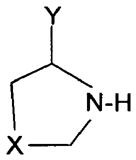 in which X is S, O, CH₂, CH₂CH₂, CH₂O or CH₂NH and Y is H or CN; the obtained condensation product of the formula 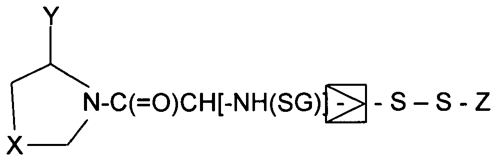 is transformed with a compound of the general formula
HS-CH₂-CH[-NH-(SG)]-(R⁹)
and is transformed being cleaved from the protecting groups (SG) into a compound of the general formula A - B - D - E (2), in which A, B, D and E may have the afore-mentioned meanings; or
- a compound of the general formula 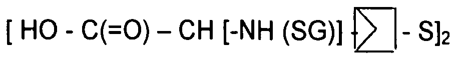 in which SG is a protecting group and 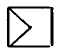 is a structure element of B, is transformed with a heterocyclic compound of the general formula 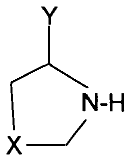 in which X is S, O, CH₂, CH₂CH₂, CH₂O or CH₂NH and Y is H or CN; and the obtained reaction product is transformed with a compound of the general formula
HS-CH₂-CH[-NH-(SG)]-R⁹
into a compound of the general formula A - B - D - E (2) in which A, B, D and E may have the afore-mentioned meanings; or
- a compound of the general formula 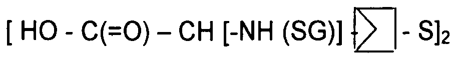 in which SG is a protecting group and 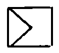 is a structure element of B, is transformed with a heterocyclic compound of the general formula 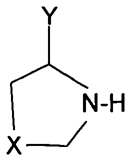 in which X is S, O, CH₂, CH₂CH₂, CH₂O or CH₂NH and Y is H or CN; and the obtained reaction product is transformed being cleaved from the protecting groups SG into a compound of the general formula A - B - D - B' - A' (1) in which A and A' may be identical or different and B and B' may be identical or different and in which A, A', B, B' and D may have the afore-mentioned meanings; or
- compounds of the general formula 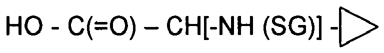
in which (SG) is a protecting group and 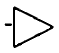 is a structure element of B, are transformed with a heterocyclic compound of the general formula 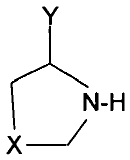 in which X is S, O, CH₂, CH₂CH₂, CH₂O or CH₂NH and Y is H or CN; the obtained condensation product of the formula 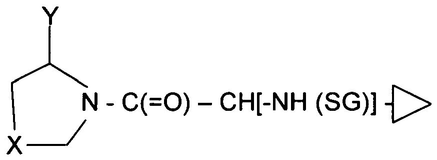 is transformed with a compound of the general formula 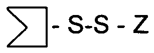 in which 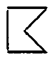 is a structure element of B and Z is a residue which activates an -S-S-group for a thiol exchange and (SG) is a protecting group; and the obtained reaction product of the formula 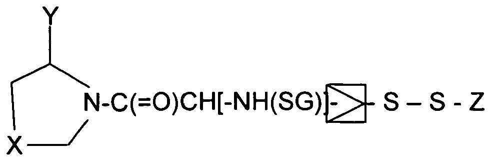 is transformed with a compound of the general formula
HS-CH₂-CH[-NH-(SG)]-(R⁹)
and is transformed being cleaved from the protecting group (SG) into a compound of the general formula A - B - D - E (2) in which A, B, D and E may have the afore-mentioned meanings.

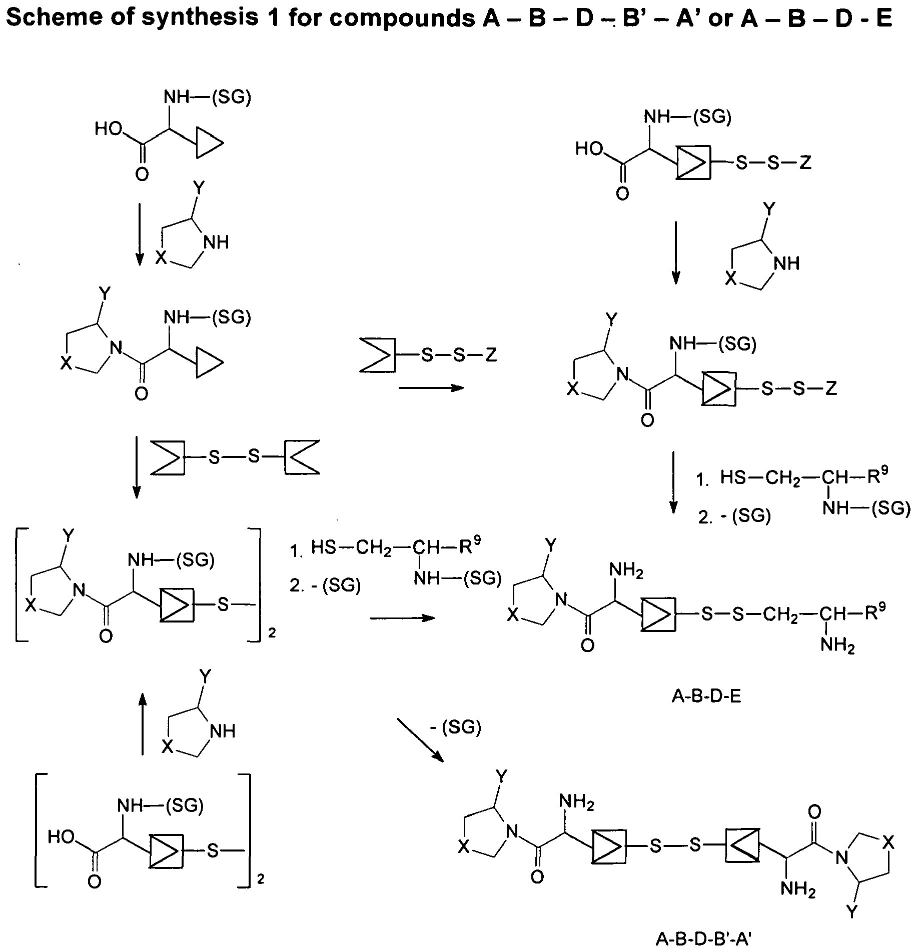

12. Compounds according to one or more than one of the Claims 1 to 10 to be used in medicine.

13. Compounds according to Claim 12 as inhibitor-precursors or inhibitor-prodrugs.

14. Compounds according to Claim 12 or Claim 13 as inhibitors of dipeptidyl peptidase IV and peptidases with analogous enzymatic effect as well as precursors and/or prodrugs for inhibitors of dipeptidyl peptidase IV and peptidases with analogous enzymatic effect as well as alanyl aminopeptidase N (APN) and peptidases with analogous enzymatic effect.

15. Compounds according to Claim 12 or Claim 13 to be used as precursors or prodrugs for inhibitors of dipeptidyl peptidase IV (DPIV), and peptidases with analogous enzymatic effect, alanyl aminopeptidase N (APN) and peptidases with analogous enzymatic effect.

16. Compounds according to any of Claim 12 to 15 to be used as prodrugs for inhibitors of the enzymes dipeptidyl peptidase IV (DPIV) and alanyl aminopeptidase N (APN).

17. Use of at least one of the compounds of the general formulae (1) or (2) according to one of the Claims 1 to 10 for the preparation of a medicament or a cosmetic preparation for the prophylaxis and therapy of diseases with exceeding immune response and inflammatory genesis including arteriosclerosis, neuronal disease, cerebral damages, skin diseases, tumour diseases and virus-caused diseases, as well as type II diabetes.

18. Use according to Claim 17, in which the at least one compound of the general formulae (1) or (2) according to any of Claims 1 to 10 generates at least one inhibitor of dipeptidyl peptidase IV (DPIV) and/or peptidases with analogous enzymatic effect as well as of alanyl aminopeptidase N (APN) and/or peptidases with analogous enzymatic effect.

19. Use according to any of Claim 17 or Claim 18 under reducing physiological or pathophysiological conditions, preferably conditions under which -S-S-bonds or -Se-Se-bonds are transformed into -SH-groups or SeH-groups

20. Pharmaceutical preparation comprising at least one of the compounds of the general formulae (1) or (2) according to any of Claims 1 to 10, optionally in combination with one or more pharmaceutically acceptable carrier(s), auxiliary substance(s) and/or adjuvant(s).

21. Cosmetic preparation comprising at least one compound of at least one of the general formulae (1) or (2) according to claims 1 to 10, optionally in combination with one or more pharmaceutically acceptable carrier(s), auxiliary substance(s) and/or adjuvant(s).

## Revendications

1. Composés répondant aux formules générales (1) ou (2)
A - B - D - B'- A' (1)
ou
A - B - D - E (2),
dans lesquelles
- A et A' peuvent être identiques ou différents et représentent le radical 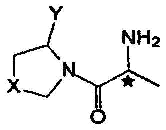
dans lequel X représente un atome de soufre, un atome d'oxygène, un groupe CH₂, un groupe CH₂CH₂, un groupe CH₂O ou un groupe CH₂NH et Y représente un atome d'hydrogène ou un groupe CN et * représente un atome de carbone chiral, de préférence dans la configuration S, respectivement L ;
- B et B' peuvent être identiques ou différents et représentent un radical alkylène, un radical cycloalkylène, un radical aralkylène, un radical hétérocycloalkylène, un radical hétéroarylalkylène, un radical arylamidoalkylène, un radical hétéroarylamidoalkylène non ramifié ou ramifié, non substitué ou substitué, contenant ou ne contenant pas un atome d'oxygène, un atome d'azote ou un atome de soufre, un radical arylène ou un radical hétéroarylène non ramifié ou ramifié, non substitué ou substitué une fois ou plusieurs fois, comprenant un ou plusieurs noyaux à cinq membres, à six membres ou à sept membres ;
- D représente un groupe - S - S - ou un groupe - Se - Se - ; et
- E représente le groupe - CH₂ - CH (NH₂) - R⁹, respectivement le groupe - CH₂ - *CH(NH₂) - R⁹, dans lequel R⁹ représente un radical alkyle, un radical cycloalkyle, un radical aralkyle, un radical hétérocycloalkyle, un radical hétéroarylalkyle, un radical arylamidoalkyle, un radical hétéroarylamidoalkyle non ramifié ou ramifié, non substitué ou substitué, contenant ou ne contenant pas un atome d'oxygène, un atome d'azote ou un atome de soufre, un radical aryle ou un radical hétéroaryle non substitué ou substitué une fois ou plusieurs fois, comprenant un ou plusieurs noyaux à cinq membres, à six membres ou à sept membres et * représente un atome de carbone chiral, de préférence dans la configuration S, respectivement L ;
- ou leurs sels d'addition d'acides avec des acides organiques et/ou inorganiques.

2. Composés répondant aux formules générales (1) ou (2), selon la revendication 1, dans lesquelles B et/ou B' représentent
(a) un radical R¹ qui représente un radical alkylène à chaîne droite ou ramifiée contenant de 1 à 6 atomes de carbone, de préférence qui représente un groupe -CH₂-, un groupe -CH₂-CH₂- ou un groupe (H₃C)₂-C< ; ou
(b) un radical -(CH₂)ₙ - R² - R³ - R⁴ -, dans lequel n représente un nombre entier de 1 à 5 ; R² représente un groupe -NH- ou un groupe -(NH) - C (=NH) - NH -, lorsque R³ représente un groupe O = C< ou un groupe - SO₂-, ou dans lequel R² représente un groupe O = C<, lorsque R³ représente un groupe - NH - ; R⁴ représente un radical alkylène, un radical cycloalkylène, un radical aralkylène, un radical hétérocycloalkylène, un radical hétéroarylalkylène, non ramifié ou ramifié, non substitué ou substitué, contenant ou ne contenant pas un atome d'oxygène, un atome d'azote ou un atome de soufre, un radical arylène ou un radical hétéroarylène non ramifié ou ramifié, non substitué ou substitué une fois ou plusieurs fois, comprenant un ou plusieurs noyaux à cinq membres, à six membres ou à sept membres ; ou
(c) un radical - R⁷ - R⁸ -, dans lequel R⁷ représente un radical benzylène substitué une fois ou plusieurs fois, et R⁸ représente une liaison simple ou un radical alkylène, un radical cycloalkylène, un radical aralkylène, un radical hétérocycloalkylène ou un radical hétéroarylalkylène, non ramifié ou ramifié, non substitué ou substitué, contenant ou ne contenant pas un atome d'oxygène, un atome d'azote ou un atome de soufre, qui peut présenter de préférence un ou plusieurs groupes amino, un ou plusieurs groupes carbonyle ou un ou plusieurs groupes carboxyle à titre de groupes fonctionnels, ou représente un radical arylène ou un radical hétéroarylène non substitué ou substitué une fois ou plusieurs fois, comprenant un ou plusieurs noyaux à cinq membres, à six membres ou à sept membres.

3. Composés répondant aux formules générales (1) et (2), selon la revendication 1 ou 2, dans lesquelles B représente un radical -(CH₂)ₙ - R² - R³ - R⁴ -, dans lequel R⁴ représente
- un groupe - CH(COOH)-R¹-, dans lequel R¹ a la signification indiquée ci-dessus, lorsque R² représente un groupe O = C < et R³ représente un groupe -NH- ; ou un groupe
- 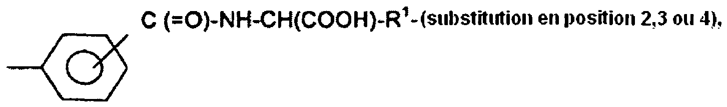 dans lequel R¹ a la signification indiquée ci-dessus, lorsque R² représente un groupe O = C < et R³ représente un groupe -NH- ; ou
- un groupe -CH (NHR⁵) -R¹-, lorsque R² représente un groupe -NH- ou un groupe -NH-C-(=NH)-NH- et R³ représente un groupe O = C<, dans lequel R⁵ représente un atome d'hydrogène ou un groupe acyle, de préférence un radical benzyloxycarbonyle, un radical fluorén-9-ylméthoxycarbonyle, un radical tert-butyloxycarbonyle ou un radical benzoyle ; ou un groupe
- 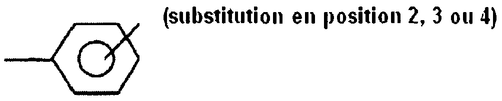 dans lequel R⁴ représente un groupe phénylène et R² représente un groupe -NH- ou un groupe -NH-C(=NH)-NH-, lorsque R³ représente un groupe O = C< ou un groupe -SO₂-, ou dans lequel R² représente un groupe O = C <, lorsque R³ représente un groupe -NH- ; ou un groupe
- 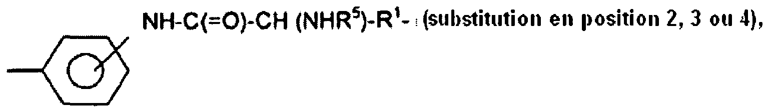 dans lequel R⁵ représente un atome d'hydrogène ou un radical acyle, de préférence un radical benzyloxycarbonyle, un radical fluorén-9-ylméthoxycarbonyle ou un radical benzoyle, et R² représente un groupe -NH- ou un groupe -NH-C(=NH)-NH-, lorsque R³ représente un groupe O = C< ou un groupe -SO₂-, ou dans lequel R² représente un groupe O = C <, lorsque R³ représente un groupe -NH- ; ou un groupe 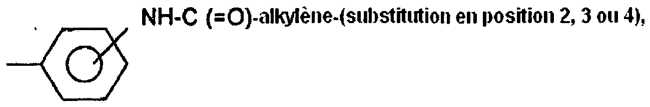 dans lequel le groupe alkylène représente un radical alkylène non ramifié ou ramifié contenant de 1 à 6 atomes de carbone et R² représente un groupe -NH- ou un groupe -NH-C(=NH)-NH-, lorsque R³ représente un groupe O = C< ou un groupe -SO₂-, ou dans lequel R² représente un groupe O = C <, lorsque R³ représente un groupe -NH- ; ou un groupe
- 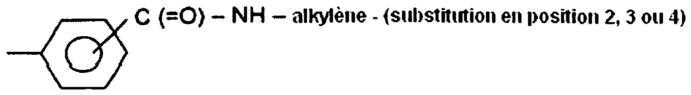 dans lequel le groupe alkylène représente un radical alkylène non ramifié ou ramifié contenant de 1 à 6 atomes de carbone et R² représente un groupe -NH- ou un groupe -NH-C(=NH)-NH-, lorsque R³ représente un groupe O = C< ou un groupe -SO₂-, ou dans lequel R² représente un groupe O = C <, lorsque R³ représente un groupe -NH- ; ou un groupe
- 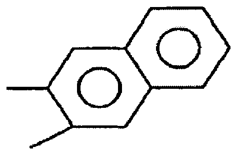 dans lequel R² représente un groupe -NH- ou un groupe -NH-C(=NH)-NH-, lorsque R³ représente un groupe O = C< ou un groupe -SO₂-, ou dans lequel R² représente un groupe O = C <, lorsque R³ représente un groupe -NH- ; ou un groupe
- 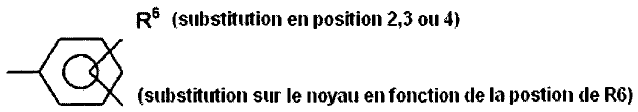 R⁶ représentant un atome d'hydrogène, un groupe NO₂, un groupe CN, un atome d'halogène ou un radical acyle et R² représente un groupe - NH- ou un groupe -NH-C(=NH)-NH-, lorsque R³ représente un groupe O = C< ou un groupe -SO₂-, ou dans lequel R² représente un groupe O = C <, lorsque R³ représente un groupe -NH- ; ou un groupe
- 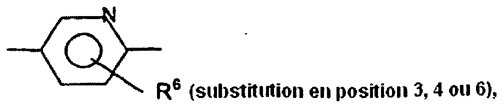 R⁶ représentant un atome d'hydrogène, un groupe NO₂, un groupe CN, un atome d'halogène ou un radical acyle et R² représente un groupe - NH- ou un groupe -NH-C(=NH)-NH-, lorsque R³ représente un groupe O = C< ou un groupe -SO₂-, ou dans lequel R² représente un groupe O = C<, lorsque R³ représente un groupe -NH- ; ou un groupe 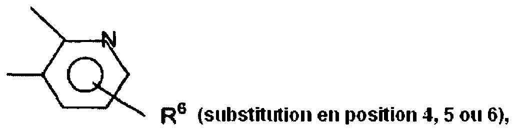 R⁶ représentant un atome d'hydrogène, un groupe NO₂, un groupe CN, un atome d'halogène ou un radical acyle et R² représente un groupe - NH- ou un groupe -NH-C(=NH)-NH-, lorsque R³ représente un groupe O = C< ou un groupe -SO₂-, ou dans lequel R² représente un groupe O = C <, lorsque R³ représente un groupe -NH- ; ou un groupe
- 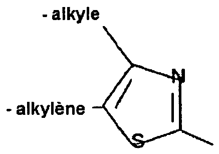 dans lequel R² représente un groupe -NH- ou un groupe -NH-C(=NH)-NH-, lorsque R³ représente un groupe O = C< ou un groupe -SO₂-, ou dans lequel R² représente un groupe O = C <, lorsque R³ représente un groupe -NH-.

4. Composés répondant aux formules générales (1) ou (2), selon la revendication 1 ou 2, dans lesquelles B représente un radical - R⁷ - R⁸ -, dans lequel R⁷ et R⁸ représentent ensemble un radical 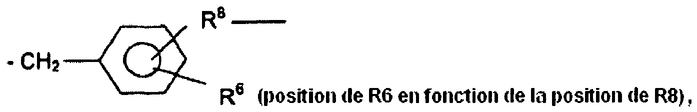 dans lequel R⁸ et R⁶ ont les significations indiquées ci-dessus.

5. Composés répondant aux formules générales (1) ou (2), selon la revendication 1 ou 2, dans lesquelles B représente un radical - R⁷ - R⁸ -, dans lequel R⁷ représente un radical benzylène substitué une fois ou plusieurs fois et R⁸ représente les radicaux suivants :
- un groupe NH - ou un groupe alkylène en C₁-C₆ - NH - en combinaison avec
- un groupe - C (=O) - alkylène en C₁-C₆ - ; ou
- un groupe C (=O) - arylène - ; ou
- un groupe SO₂ - alkylène - ; ou
- un groupe - SO₂ - arylène - ; ou un groupe 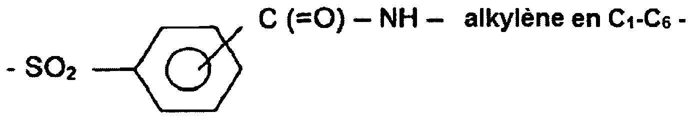 (substitution en position 2, 3 ou 4) ; ou
- un groupe C (=O) - CH (NHR⁵) - R¹, dans lequel R¹ et R⁵ ont les significations indiquées ci-dessus ; ou bien
- un groupe O = C< en combinaison avec
- un groupe NH - alkylène en C₁-C₆-; ou
- un groupe - NH - arylène - ; ou
- un groupe - NH - CH (COOH) - R¹-, dans lequel R¹ présente les significations mentionnées ci-dessus ; ou bien
- un groupe 0 - alkylène en C₁-C₆-; ou
- un groupe 0 - arylène - ; ou
- un groupe - 0 - alkylène - NH - C (=O) - CH (NH₂) - R¹ -, dans lequel R¹ présente les significations mentionnées ci-dessus ; ou
- un groupe - 0 - alkylène en C₁-C₆ - C (=O) - NH - CH (COOH) - R¹ -, dans lequel R¹ présente les significations mentionnées ci-dessus.

6. Composés répondant aux formules générales (1) et (2), selon l'une quelconque des revendications 1 à 5, dans lesquels les sels d'addition d'acides représentent des sels d'acides pharmaceutiquement acceptables ; de préférence dans lesquels les sels d'addition d'acides représentent des chlorhydrates, des trifluoroacétates, des tartrates, succinates, des formiates et/ou des citrates.

7. Composés répondant à la formule générale (1), selon l'une quelconque des revendications 1 à 6, plus précisément des composés répondant à la formule générale (1a) 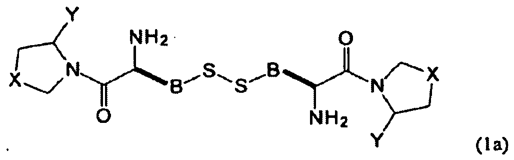 dans laquelle X, Y et B ont les significations indiquées ci-dessus, ainsi que leurs sels d'addition d'acides, de préférence leurs sels d'addition d'acides avec des acides inorganiques et/ou organiques pharmaceutiquement acceptables.

8. Composés répondant à la formule générale (1a) selon la revendication 7, dans laquelle X, Y et B ont les significations indiquées ci-après
| **N°** | **B** | **X** | **Y** | **Formule brut** |
|---|---|---|---|---|
| I | - CH₂- | -CH₂- | H | C₁₄H₂₆N₄O₂S₂ |
| II | - CH₂- | S | H | C₁₂H₂₂N₄O₂S₄ |
| III | - CH₂- | -CH₂- | CN | C₁₆H₂₄N₈O₂S₂ |
| IV | 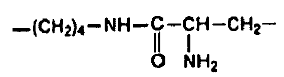 | S | H | C₂₄H₄₈N₂O₄S₄ |
| V | 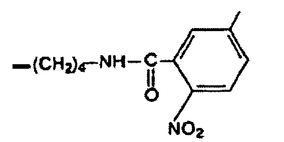 | S | H | C₃₂H₄₂N₈O₈S₄ |
| VI | 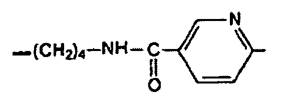 | S | H | C₃₀H₄₂N₈O₄S₄ |
ainsi que leurs sels d'addition d'acides, de préférence leurs sels d'addition d'acides avec des acides inorganiques et/ou organiques pharmaceutiquement acceptables.

9. Composés répondant à la formule générale (2), selon l'une quelconque des revendications 1 à 6, plus précisément des composés répondant à la formule générale (2a) 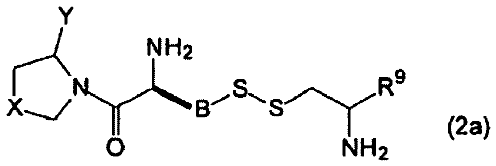 dans laquelle X, Y, R⁹ et B ont les significations indiquées ci-dessus, ainsi que leurs sels d'addition d'acides, de préférence leurs sels d'addition d'acides avec des acides inorganiques et/ou organiques pharmaceutiquement acceptables.

10. Composés répondant à la formule générale (2a) selon la revendication 9, dans laquelle X, Y, R⁹ et B ont les significations indiquées ci-après
| **N°** | **B** | **R⁹** | **X** | **Y** | **Formule brut** |
|---|---|---|---|---|---|
| VII | - CH₂- | 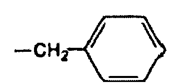 | S | H | C₁₅H₂₃N₃OS₃ |
| VIII | 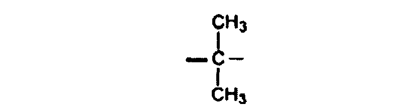 | 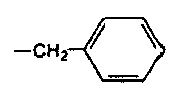 | S | H | C₁₇H₂₇N₃OS₃ |
| IX | 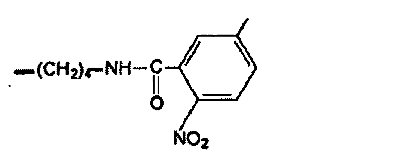 | 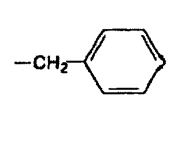 | S | H | C₂₅H₃₃N₅O₄S₃ |
| X | 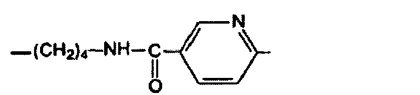 | 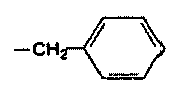 | S | H | C₂₄H₃₃N₅O₂S₃ |
| XI | 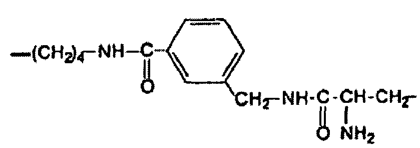 | 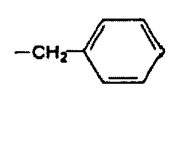 | S | H | C₂₃H₄₂N₆O₃S₃ |
| XII | 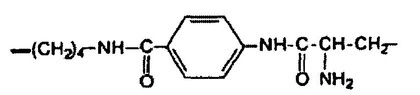 | 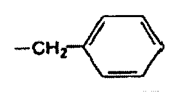 | S | H | C₂₈H₄₀N₅O₂S₃ |
| XIII | 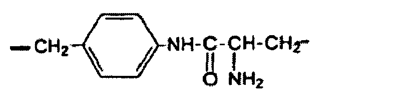 | 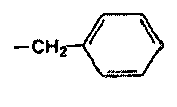 | S | H | C₂₄H₃₃N₅O₂S₃ |
ainsi que leurs sels d'addition d'acides, de préférence leurs sels d'addition d'acides avec des acides inorganiques et/ou organiques pharmaceutiquement acceptables.

11. Procédé pour la préparation de composés répondant aux formules générales
A - B - D - B' - A' (1)
ou
A - B - D - E (2),
dans lesquelles
- A, B, D et E ont les significations indiquées dans les revendications 1 à 10, dans lequel, de manière correspondante au schéma de synthèse 1,
- on fait réagir des composés répondant à la formule générale 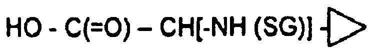 dans laquelle (SG) représente un groupe de protection et 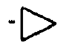 représente un élément de structure de B, avec un composé hétérocyclique répondant à la formule générale 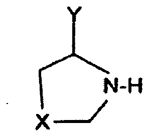 dans laquelle X représente un atome de soufre, un atome d'oxygène, un groupe CH₂, un groupe CH₂CH₂, un groupe CH₂O ou un groupe CH₂NH et Y représente un atome d'hydrogène ou un groupe CN ; on fait réagir le produit de condensation obtenu répondant à la formule 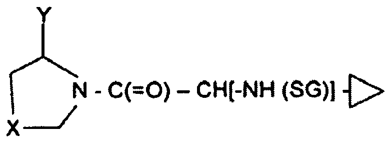 avec un composé répondant à la formule générale 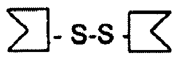 dans laquelle 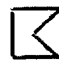 représente un élément de structure de B ; et on transforme le produit réactionnel obtenu par élimination des groupes de protection (SG) en un composé répondant à la formule générale A - B - D - B' - A' (1), dans laquelle A et A' peuvent être identiques ou différents et dans laquelle B et B' peuvent être identiques ou différents et dans laquelle A, A', B, B' et D peuvent avoir les significations indiquées ci-dessus ; ou bien
- on fait réagir des composés répondant à la formule générale 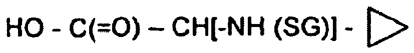 dans laquelle (SG) représente un groupe de protection et 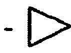 représente un élément de structure de B avec un composé hétérocyclique répondant à la formule générale 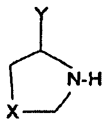 dans laquelle X représente un atome de soufre, un atome d'oxygène, un groupe CH₂, un groupe CH₂CH₂, un groupe CH₂O ou un groupe CH₂NH et Y représente un atome d'hydrogène ou un groupe CN ; on fait réagir le produit de condensation obtenu répondant à la formule 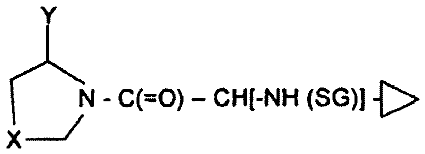 avec un composé répondant à la formule générale 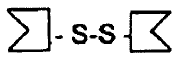 dans laquelle 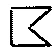 représente un élément de structure de B ; et on faire réagir le produit réactionnel obtenu avec un composé répondant à la formule générale
HS-CH₂-CH[-NH-(SG)]-(R⁹)
et après élimination des groupes de protection (SG) pour obtenir un composé répondant à la formule générale A - B - D - E (2), dans laquelle A, B, D et E peuvent avoir les significations indiquées ci-dessus ; ou bien
- on fait réagir un composé répondant à la formule générale 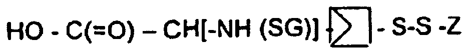 dans laquelle (SG) représente un groupe de protection, 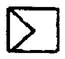 représente un élément de structure de B et Z représente un radical qui active un groupe -S-S-pour un échange de thiols, avec un composé hétérocyclique répondant à la formule générale 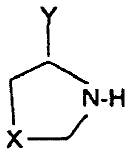 dans laquelle X représente un atome de soufre, un atome d'oxygène, un groupe CH₂, un groupe CH₂CH₂, un groupe CH₂O ou un groupe CH₂NH et Y représente un atome d'hydrogène ou un groupe CN ; on fait réagir le produit de condensation répondant à la formule 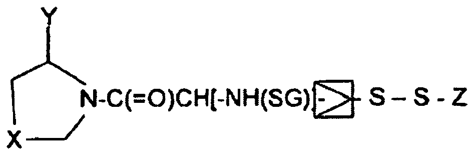 avec un composé répondant à la formule générale
HS-CH₂-CH[-NH-(SG)]-(R⁹)
et après élimination des groupes de protection (SG) pour obtenir un composé répondant à la formule générale A - B - D - E (2), dans laquelle A, B, D et E peuvent avoir les significations indiquées ci-dessus ; ou bien
- on fait réagir un composé répondant à la formule générale 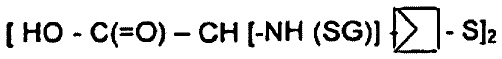 dans laquelle SG représente un groupe de protection et 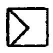 représente un élément de structure de B, avec un composé hétérocyclique répondant à la formule générale 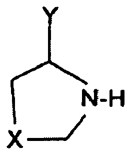 dans laquelle X représente un atome de soufre, un atome d'oxygène, un groupe CH₂, un groupe CH₂CH₂, un groupe CH₂O ou un groupe CH₂NH et Y représente un atome d'hydrogène ou un groupe CN ; et on fait réagir le produit réactionnel obtenu avec un composé répondant à la formule générale
HS-CH₂-CH[-NH-(SG)]-R⁹
pour obtenir un composé répondant à la formule générale A - B - D - E (2), dans laquelle A, B, D et E peuvent avoir les significations indiquées ci-dessus ; ou bien
- on fait réagir un composé répondant à la formule générale 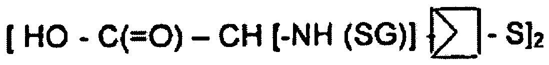 dans laquelle SG représente un groupe de protection et 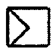 représente un élément de structure de B, avec un composé hétérocyclique répondant à la formule générale 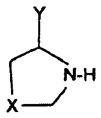 dans laquelle X représente un atome de soufre, un atome d'oxygène, un groupe CH₂, un groupe CH₂CH₂, un groupe CH₂O ou un groupe CH₂NH et Y représente un atome d'hydrogène ou un groupe CN ; et on transforme le produit réactionnel obtenu avec élimination des groupes de protection (SG) en un composé répondant à la formule générale A - B - D - B' - A' (1), dans laquelle A et A' peuvent être identiques ou différents et B et B' peuvent être identiques ou différents et dans laquelle A, A', B, B' et D peuvent avoir les significations indiquées ci-dessus ; ou bien
- on fait réagir des composés répondant à la formule générale 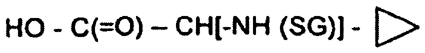 dans laquelle (SG) représente un groupe de protection et 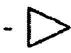 représente un élément de structure de B avec un composé hétérocyclique répondant à la formule générale 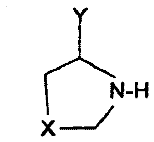 dans laquelle X représente un atome de soufre, un atome d'oxygène, un groupe CH₂, un groupe CH₂CH₂, un groupe CH₂O ou un groupe CH₂NH et Y représente un atome d'hydrogène ou un groupe CN ; on fait réagir le produit de condensation obtenu répondant à la formule 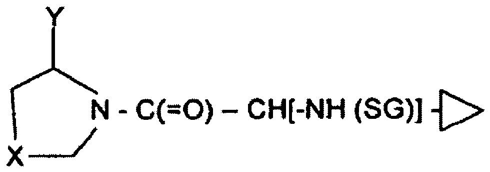 avec un composé répondant à la formule générale 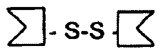 dans laquelle 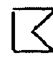 représente un élément de structure de B et Z représente un radical qui active un groupe -S-S- pour un échange de thiols, et (SG) représente un groupe de protection ; et on fait réagir le produit réactionnel obtenu répondant à la formule 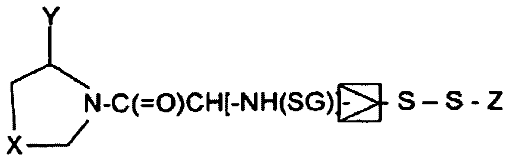 avec un composé répondant à la formule générale
HS - CH₂ - CH [-NH - (SG)] - (R⁹)
et après élimination des groupes de protection (SG) pour obtenir un composé répondant à la formule générale A - B - D - E (2), dans laquelle A, B, D et E peuvent avoir les significations indiquées ci-dessus.

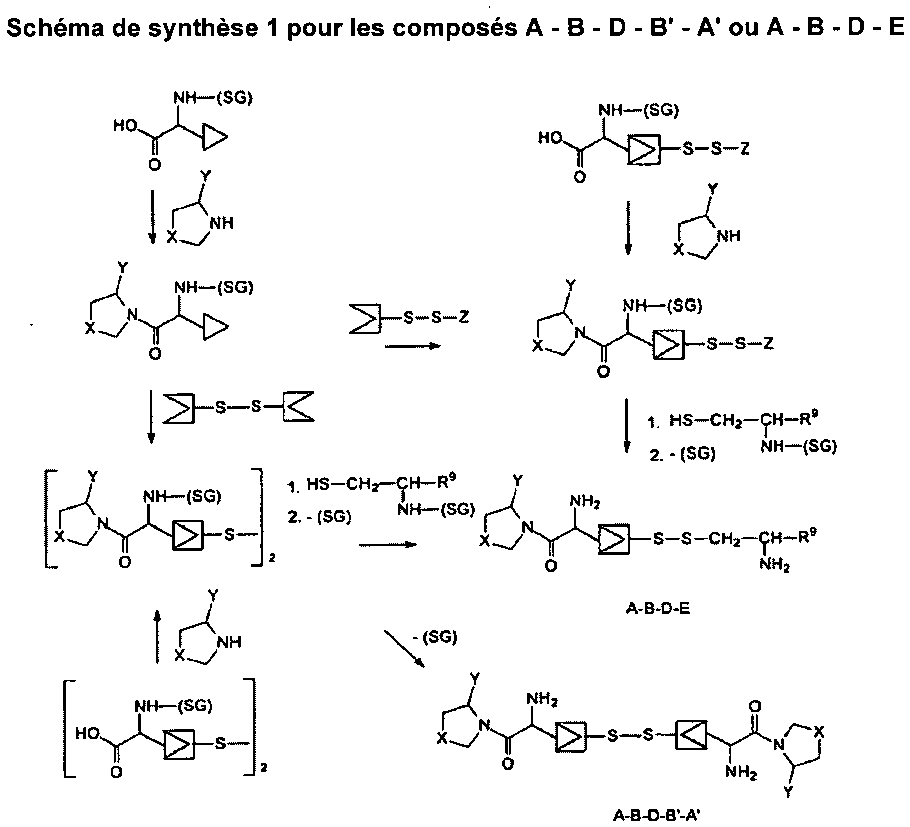

12. Composés selon une ou plusieurs des revendications 1 à 10, à utiliser en médecine.

13. Composés selon la revendication 12, à titre de précurseurs d'inhibiteurs ou à titre de promédicaments d'inhibiteurs.

14. Composés selon la revendication 12 ou 13, à titre d'inhibiteurs de la dipeptidylpeptidase IV et de peptidases possédant une activité enzymatique analogue, ainsi qu'à titre de précurseurs et/ou de promédicaments pour des inhibiteurs de la dipeptidylpeptidase IV et de peptidases possédant une activité enzymatique analogue, de même que de l'alanyl-aminopeptidase N (APN) et de peptidases possédant une activité enzymatique analogue.

15. Composés selon la revendication 12 ou 13, à des fins d'utilisation comme précurseurs ou comme promédicaments pour des inhibiteurs de la dipeptidylpeptidase IV et de peptidases possédant une activité enzymatique analogue, de l'alanyl-aminopeptidase N (APN) et de peptidases possédant une activité enzymatique analogue.

16. Composés selon l'une quelconque des revendications 12 à 15, à des fins d'utilisation comme promédicaments pour des inhibiteurs des enzymes dipeptidylpeptidase IV (DPIV) et alanyl-aminopeptidase N (APN).

17. Utilisation d'au moins un composé répondant aux formules générales (1) ou (2), selon l'une quelconque des revendications 1 à 10, pour la fabrication d'un médicament ou d'une préparation cosmétique pour la prophylaxie et la thérapie de maladies qui s'accompagnent d'une réponse immunitaire surabondante et qui sont d'origine inflammatoire, y compris l'artériosclérose, des maladies neuronales, des lésions cérébrales, des maladies cutanées, des cancers et des maladies virales, ainsi que le diabète de type II.

18. Utilisation selon la revendication 17, dans laquelle ledit au moins un composé répondant aux formules générales (1) ou (2) selon l'une quelconque des revendications 1 à 10, génère au moins un inhibiteur de la dipeptidylpeptidase IV (DPIV) et/ou de peptidases possédant une activité enzymatique analogue, ainsi que de l'alanyl-aminopeptidase N (APN) et/ou de peptidases possédant une activité enzymatique analogue.

19. Utilisation selon la revendication 17 ou 18, dans des conditions de réduction de type physiologique ou de type pathophysiologique, de préférence des conditions dans lesquelles on transforme des liaisons -S-S- ou des liaisons -Se-Se- en groupes -SH- ou en groupes -SeH-.

20. Préparation pharmaceutique, comprenant au moins un composé répondant à au moins une des formules générales (1) ou (2) selon l'une quelconque des revendications 1 à 10, de manière facultative conjointement avec un ou plusieurs supports, substances auxiliaires et/ou adjuvants pharmaceutiquement acceptables.

21. Préparation cosmétique, comprenant au moins un composé répondant à au moins une des formules générales (1) ou (2) selon l'une quelconque des revendications 1 à 10, de manière facultative conjointement avec un ou plusieurs supports, substances auxiliaires et/ou adjuvants acceptables du point de vue cosmétique.
